# EUROPÄISCHE PATENTSCHRIFT

(11) **EP 3 218 368 B1**
(45) Veröffentlichungstag und Bekanntmachung des Hinweises auf die Patenterteilung: **25.11.2020**
(21) Anmeldenummer: 15780766.0
(22) Anmeldetag: 13.10.2015
(51) Int. Cl.: C07D 405/14, C07D 401/04, C07D 401/10, C07D 405/12, C07D 409/14, C07D 413/10, C07D 417/10, C07D 487/06, C07D 491/107, C07D 211/10, C09K 11/06, H01L 51/50

(54) **MATERIALIEN FÜR ORGANISCHE ELEKTROLUMINESZENZVORRICHTUNGEN**
MATERIALS FOR ORGANIC ELECTROLUMINESCENT DEVICES
MATÉRIAUX POUR DISPOSITIFS ÉLECTROLUMINESCENTS ORGANIQUES

(30) Priorität: 11.11.2014 EP 14003784
(43) Veröffentlichungstag der Anmeldung: 20.09.2017
(73) Patentinhaber: Merck Patent GmbH, 64293 Darmstadt (DE)
(72) Erfinder: PARHAM, Amir, Hossain, 60486 Frankfurt am Main (DE); STOESSEL, Philipp, 60389 Frankfurt am Main (DE); EBERLE, Thomas, 76829 Landau (DE); JATSCH, Anja, 60489 Frankfurt (DE); KROEBER, Jonas, Valentin, 60311 Frankfurt am Main (DE); PFLUMM, Christof, 64291 Darmstadt (DE)
(86) Internationale Anmeldenummer: PCT/EP2015/002016
(87) Internationale Veröffentlichungsnummer: WO 2016/074755

(56) Entgegenhaltungen:
- EP-A2- 2 327 679
- WO-A1-2015/046955
- JP-A- 2006 151 844

## Beschreibung

Die vorliegende Erfindung beschreibt Phenanthridin-Derivate, insbesondere zur Verwendung als Triplettmatrixmaterialien oder Lochtransportmaterialien in organischen Elektrolumineszenzvorrichtungen. Die Erfindung betrifft ferner ein Verfahren zur Herstellung der erfindungsgemäßen Verbindungen sowie elektronische Vorrichtungen enthaltend diese Verbindungen.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden häufig metallorganische Komplexe eingesetzt, die Phosphoreszenz statt Fluoreszenz zeigen. Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Triplettemission (Phosphoreszenz) zeigen, immer noch Verbesserungsbedarf, beispielsweise im Hinblick auf Effizienz, Betriebsspannung und Lebensdauer.

Die Eigenschaften von phosphoreszierenden OLEDs werden nicht nur von den eingesetzten Triplettemittern bestimmt. Hier sind insbesondere auch die anderen verwendeten Materialien wie zum Beispiel Matrixmaterialien von besonderer Bedeutung. Verbesserungen dieser Materialien können somit auch zu deutlichen Verbesserungen der OLED-Eigenschaften führen.

Gemäß dem Stand der Technik werden unter anderem Indolocarbazolderivate (z. B. gemäß WO 2007/063754 oder WO 2008/056746) oder Indenocarbazolderivate (z. B. gemäß WO 2010/136109 oder WO 2011/000455), insbesondere solche, die mit elektronenarmen Heteroaromaten wie Triazin substituiert sind, als Matrixmaterialien für phosphoreszierende Emitter verwendet. Weiterhin werden beispielsweise Bisdibenzofuranderivate (z. B. gemäß EP 2301926) als Matrixmaterialien für phosphoreszierende Emitter verwendet. Weiterhin werden Phenanthridinderivate (z. B. gemäß JP 2006 151844 oder EP 2 327 679) in organischen Elektrolumineszenzvorrichtungen verwendet. Allerdings besteht bei Verwendung dieser Matrixmaterialien noch Verbesserungsbedarf, insbesondere in Bezug auf die Effizienz, die Lebensdauer und die Betriebsspannung der Vorrichtung.

Aufgabe der vorliegenden Erfindung ist die Bereitstellung von Verbindungen, welche sich für den Einsatz in einer fluoreszierenden oder phosphoreszierenden OLED, insbesondere einer phosphoreszierenden OLED, eignen, beispielsweise als Matrixmaterial oder als Ladungstransportmaterial, insbesondere Lochtransport- bzw. Elektronenblockiermaterial. Insbesondere ist es die Aufgabe der vorliegenden Erfindung, Matrixmaterialien bereitzustellen, welche sich auch für grün und blau phosphoreszierende OLEDs eignen, sowie neue Ladungstransportmaterialien bereitzustellen.

Es wurde überraschend gefunden, dass Elektrolumineszenzvorrichtungen, die Verbindungen gemäß der folgenden Formel (3) enthalten, Verbesserungen gegenüber dem Stand der Technik aufweisen, insbesondere beim Einsatz als Matrixmaterialien für phosphoreszierende Dotanden oder Lochtransportmaterialien. 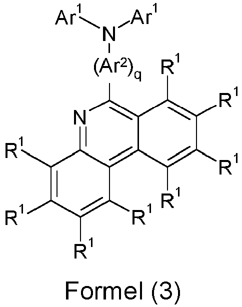 wobei für die verwendeten Symbole und Indizes gilt:
- Ar¹: sind bei jedem Auftreten gleich oder verschieden ausgewählt aus den Gruppen mit den folgenden Formeln (Ar-1) bis (Ar-13) 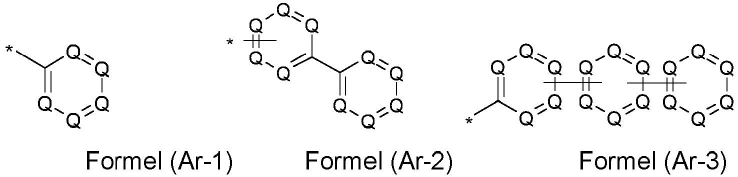 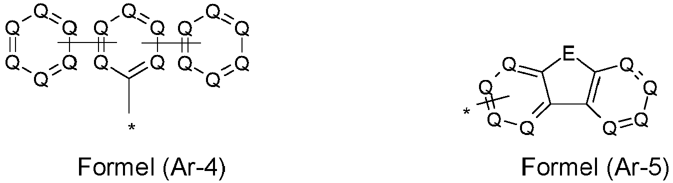 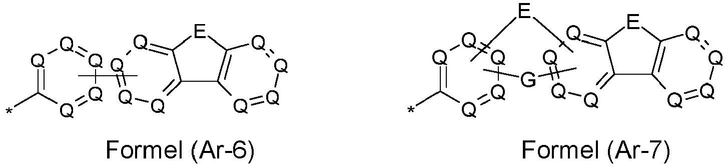 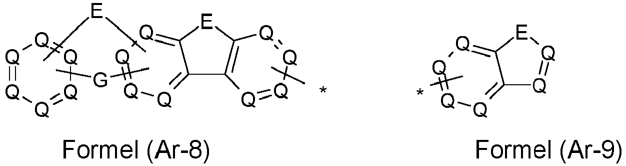 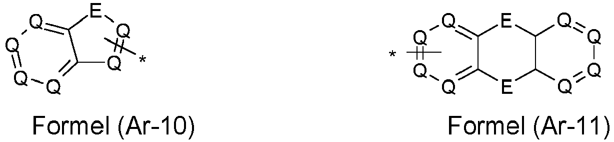 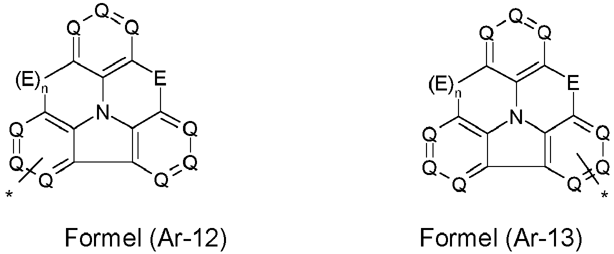 wobei:
Q ist bei jedem Auftreten gleich oder verschieden CR² oder N, wobei maximal 3 Symbole Q pro Cyclus für N stehen;
E ist bei jedem Auftreten gleich oder verschieden (CR²)₂, NR², O, S oder C=O;
G ist bei jedem Auftreten eine Einfachbindung, (CR²)₂, NR², O, S oder C=O;
n ist 0 oder 1, wobei n gleich 0 bedeutet, dass an dieser Position keine Gruppe E gebunden ist und an den entsprechenden Kohlenstoffatomen stattdessen Reste R² gebunden sind; und
* die Bindung zur Ar²-N-Gruppe darstellt;
- Ar²: ist bei jedem Auftreten gleich oder verschieden ausgewählt aus der Gruppe bestehend aus ortho-, meta- oder para-Biphenyl, Fluoren, Dibenzofuran, Dibenzothiophen oder Carbazol, wobei im Falle von heteroaromatischen Ringsystemen die divalente Bindung zum Grundgerüst nicht über das N-Atom erfolgt, wobei diese Gruppen durch einen oder mehrere Reste R² substituiert sein können;
- R¹: ist bei jedem Auftreten gleich oder verschieden H, D, F, Si(R²)₃, CN, eine geradkettige Alkyl- oder Alkoxygruppe mit 1 bis 10 C-Atomen oder eine verzweigte oder cyclische Alkyl- oder Alkoxygruppe mit 3 bis 10 C- Atomen, die jeweils mit einem oder mehreren Resten R² substituiert sein kann, wobei eine oder mehrere nicht-benachbarte CH₂-Gruppen durch O ersetzt sein können und wobei ein oder mehrere H-Atome durch D oder F ersetzt sein können, ein aromatisches oder heteroaromatisches Ringsystem mit 6 bis 60 aromatischen Ringatomen, das jeweils mit einem oder mehreren Resten R² substituiert sein kann;
- R²: ist bei jedem Auftreten gleich oder verschieden H, D, F, Si(R³)₃, CN, eine geradkettige Alkyl- oder Alkoxygruppe mit 1 bis 10 C-Atomen oder eine verzweigte oder cyclische Alkyl- oder Alkoxygruppe mit 3 bis 10 C- Atomen, die jeweils mit einem oder mehreren Resten R³ substituiert sein kann, wobei eine oder mehrere nicht-benachbarte CH₂-Gruppen durch O ersetzt sein können und wobei ein oder mehrere H-Atome durch D oder F ersetzt sein können, ein aromatisches oder heteroaromatisches Ringsystem mit 6 bis 60 aromatischen Ringatomen, das jeweils mit einem oder mehreren Resten R³ substituiert sein;
- R³: ist bei jedem Auftreten gleich oder verschieden H, D, F, CN, Si(R⁴)₃, eine geradkettige Alkyl- oder Alkoxygruppe mit 1 bis 40 C-Atomen oder eine verzweigte oder cyclische Alkyl- oder Alkoxygruppe mit 3 bis 40 C-Atomen, die jeweils mit einem oder mehreren Resten R⁴ substituiert sein kann, oder ein aromatisches oder heteroaromatisches Ringsystem mit 5 bis 60 aromatischen Ringatomen, das jeweils durch einen oder mehrere Reste R⁴ substituiert sein kann;
- R⁴: ist bei jedem Auftreten gleich oder verschieden H, D, F oder ein aliphatischer Kohlenwasserstoffrest mit 1 bis 20 C-Atomen oder eine Aryl- oder Heteroarylgruppe mit 5 bis 40 Ringatomen, die durch einen oder mehrere Reste R⁵ substituiert sein kann;
- R⁵: ist bei jedem Auftreten gleich oder verschieden H, D, F oder ein aliphatischer Kohlenwasserstoffrest mit 1 bis 20 C-Atomen;
- q: ist 1;
wobei
- Ar²: im Falle eines heteroaromatischen Ringsystems über eine C-C-Bindung mit dem Phenanthridin-Grundgerüst verbunden ist; und
- R¹: im Falle eines heteroaromatischen Ringsystems über eine C-C-Bindung mit dem Phenanthridin-Grundgerüst verbunden ist.

Eine Arylgruppe im Sinne dieser Erfindung enthält 6 bis 60 C-Atome; eine Heteroarylgruppe im Sinne dieser Erfindung enthält 2 bis 60 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Thiophen, etc., oder eine kondensierte (anellierte) Aryl- oder Heteroarylgruppe, beispielsweise Naphthalin, Anthracen, Phenanthren, Dibenzofuran, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 6 bis 80 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind. Weiterhin werden miteinander durch Einfachbindung verknüpfte Aromaten, wie zum Beispiel Biphenyl, als aromatisches Ringsystem im Sinne dieser Anmeldung bezeichnet.

Eine elektronenarme Heteroarylgruppe im Sinne der vorliegenden Erfindung ist definiert als 5-Ring-Heteroarylgruppe mit mindestens zwei Heteroatomen, beispielsweise Imidazol, Oxazol, Oxadiazol, etc., oder als 6-Ring-Heteroarylgruppe mit mindestens einem Heteroatom, beispielsweise Pyridin, Pyrimidin, Pyrazin, Triazin, etc.. Dabei können an diese Gruppen auch noch weitere 6-Ring-Aryl- oder 6-Ring-Heteroarylgruppen ankondensiert sein, wie beispielsweise in Benzimidazol oder Chinolin.

Im Rahmen der vorliegenden Erfindung werden unter einem aliphatischen Kohlenwasserstoffrest bzw. einer Alkylgruppe bzw. einer Alkenyl- oder Alkinylgruppe, die typischerweise 1 bis 40 oder auch 1 bis 20 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkenyl-, Alkinyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 80 aromatischen Ringatomen, welches noch jeweils mit den oben genannten Resten substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis-oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombinationen dieser Systeme. Dabei können diese Gruppen jeweils durch die oben genannten Reste substituiert sein.

Unter einer Aryloxygruppe gemäß der Definition der vorliegenden Erfindung wird eine Arylgruppe, wie oben definiert, verstanden, welche über ein Sauerstoffatom gebunden ist. Eine analoge Definition gilt für Heteroaryloxygruppen.

Eine elektronenarme Heteroarylgruppe im Sinne der vorliegenden Erfindung ist definiert als 5-Ring-Heteroarylgruppe mit mindestens zwei Heteroatomen, beispielsweise Imidazol, Oxazol, Oxadiazol, etc., oder als 6-Ring-Heteroarylgruppe mit mindestens einem Heteroatom, beispielsweise Pyridin, Pyrimidin, Pyrazin, Triazin, etc. Dabei können an diese Gruppen auch noch weitere 6-Ring-Aryl- oder 6-Ring-Heteroarylgruppen ankondensiert sein, wie beispielsweise in Benzimidazol oder Chinolin.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung verknüpft sind. Dies wird durch das folgende Schema verdeutlicht: Ringbildung 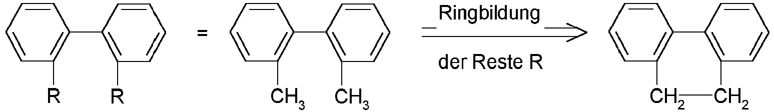 Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

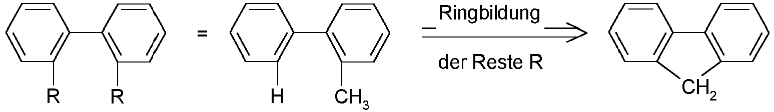

In einer bevorzugten Ausführungsform der Erfindung umfasst die Verbindung keine Arylgruppe oder Heteroarylgruppe mit mehr als 22 aromatischen Ringatomen, bevorzugt mit mehr als 19 aromatischen Ringatomen. Dies führt dazu, dass die Verbindungen bessere Leistungsdaten in phosphoreszierenden OLEDs zeigen.

In einer weiteren bevorzugten Ausführungsform stehen in der Formel (Ar-1) 0, 2 oder 3 Symbole Q für N.

Bevorzugte Ausführungsformen der Formel (Ar-8) zeigen die folgenden Formeln (Ar-8-1) bis (Ar-8-7): 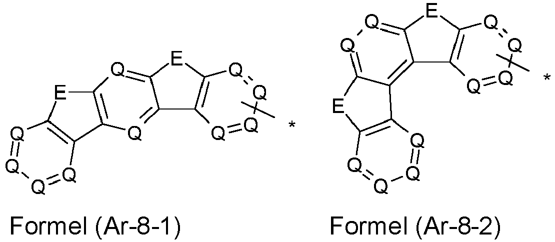 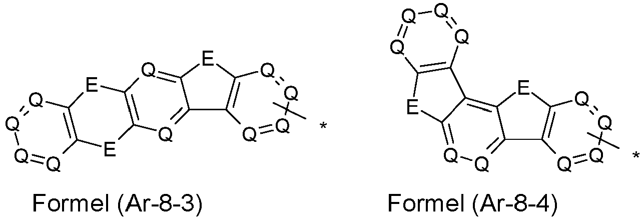 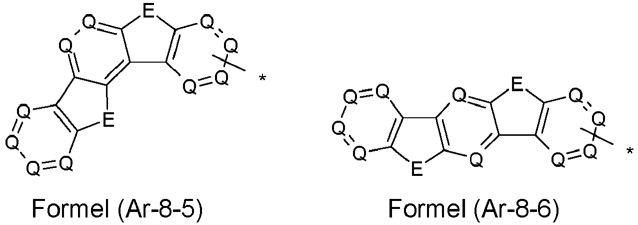 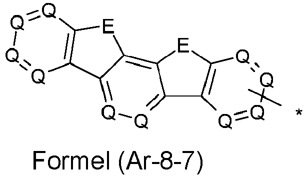 wobei die Symbole den Symbolen der Formel (Ar-8) entsprechen. Besonders bevorzugt ist Q immer CR².

In einer weiteren bevorzugten Ausführungsform ist die Gruppe Ar¹ bei jedem Auftreten gleich oder verschieden ausgewählt aus den Gruppen mit den Strukturen der Formeln (Ar-1) bis (Ar-13), wobei die allgemeinen Formeln durch die besonders bevorzugten Ausführungsformen jeweils gemäß der folgenden Formeln (Ar-1-1) bis (Ar-13-1) ersetzt werden (z. B. wird Formel (Ar-1) durch eine der Formeln (Ar-1-1) bis (Ar-1-9) ersetzt): 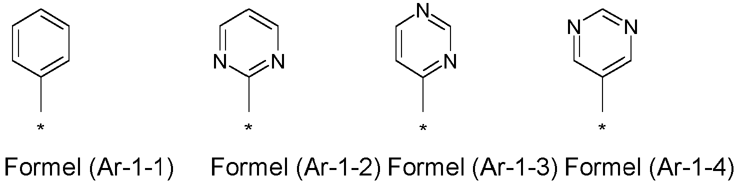 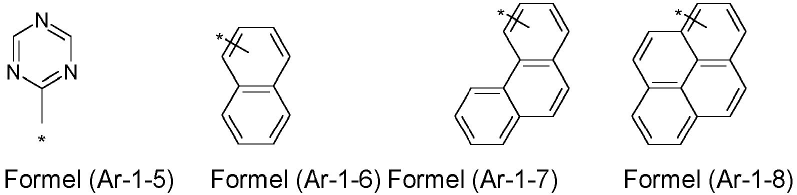 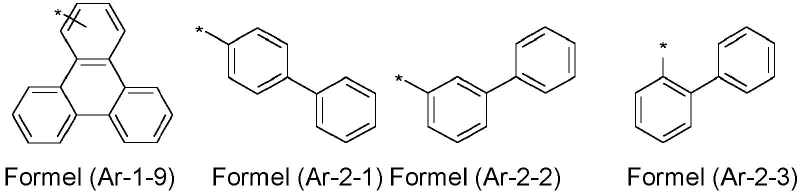 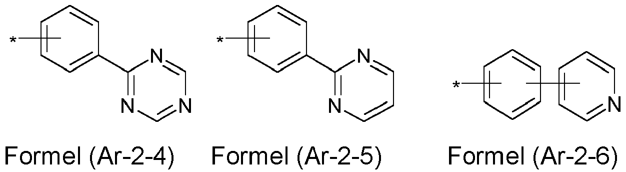 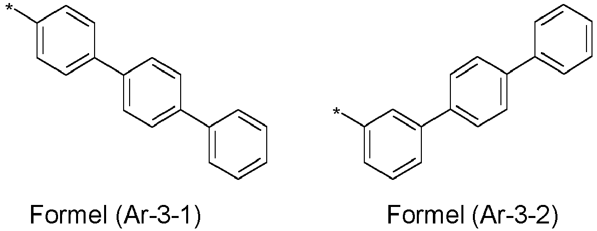 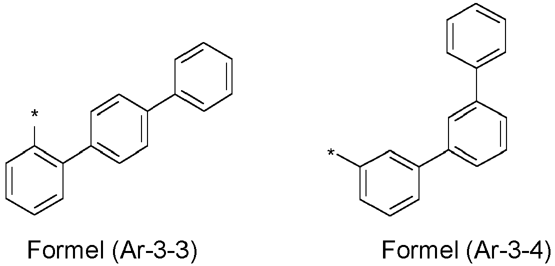 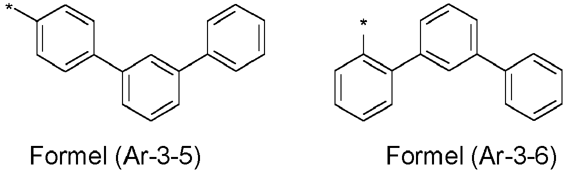 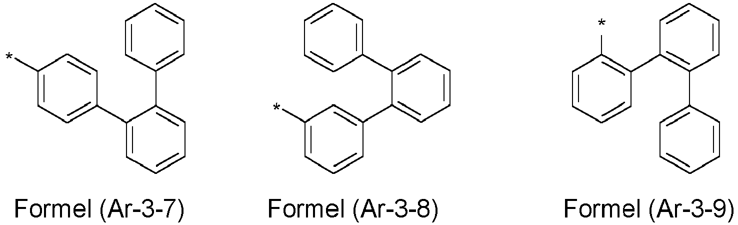 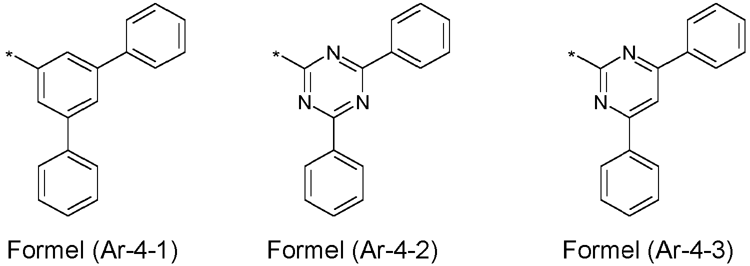 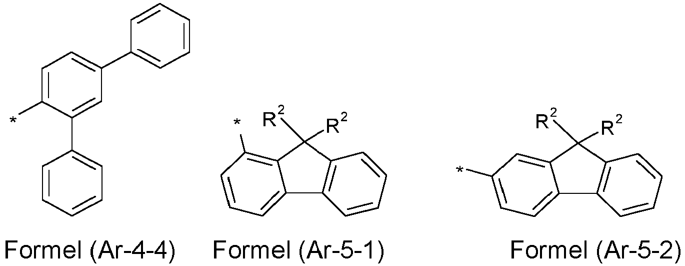 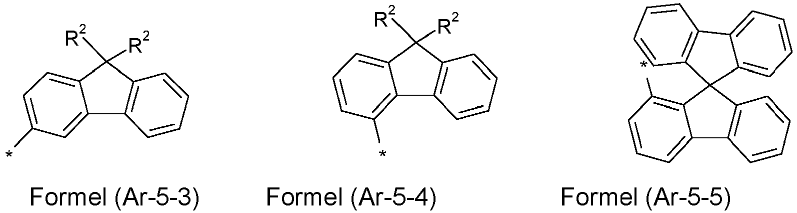 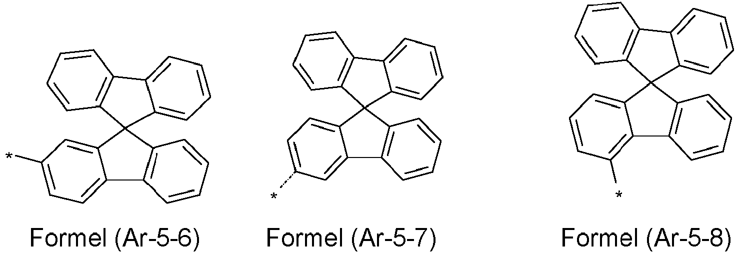 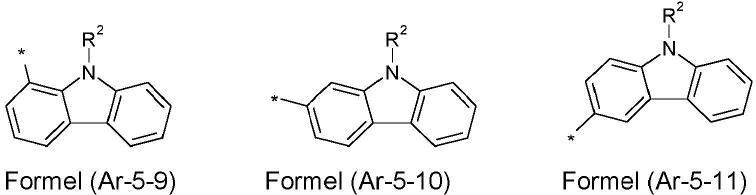 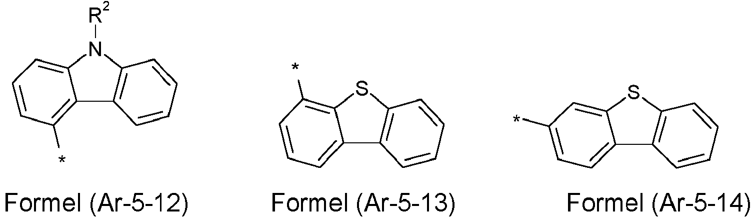 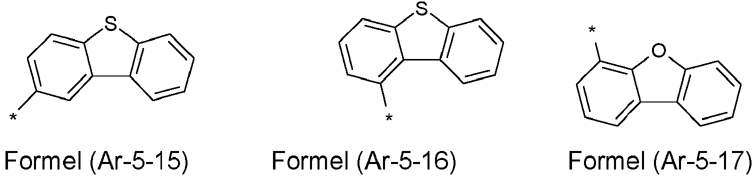 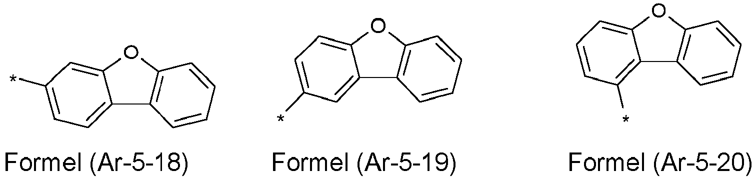 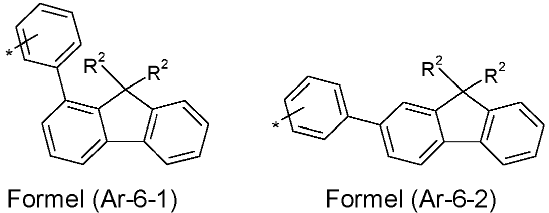 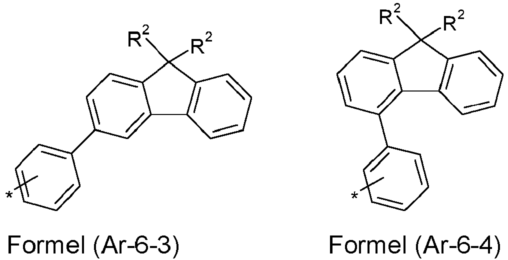 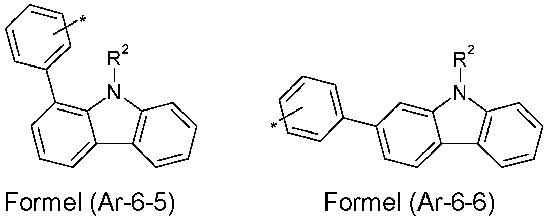 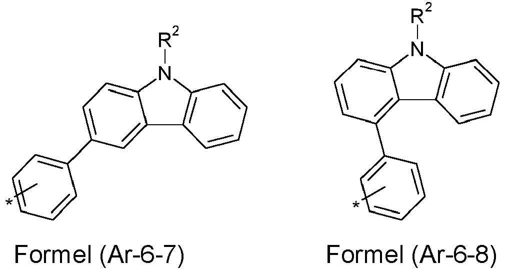 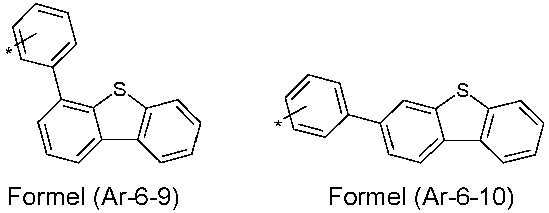 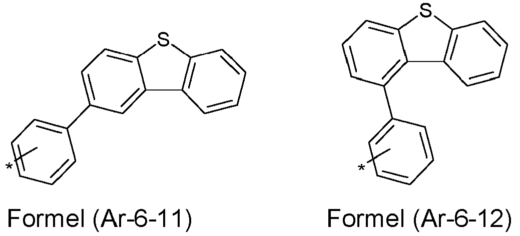 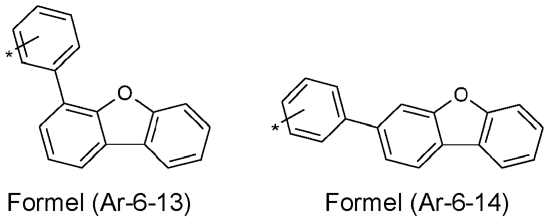 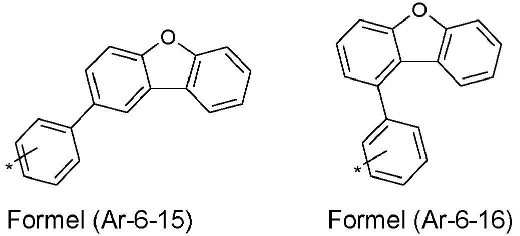 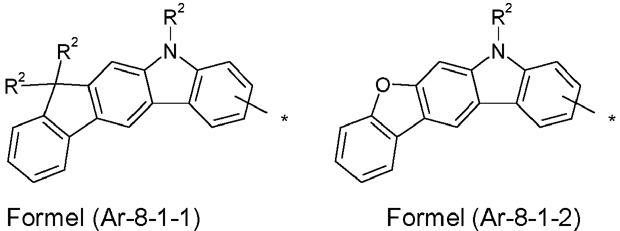 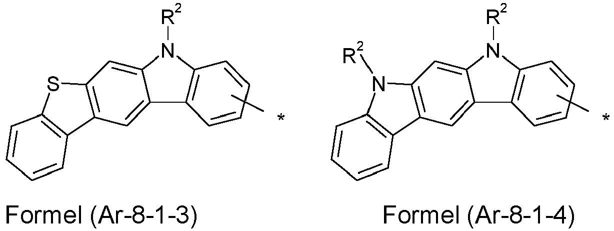 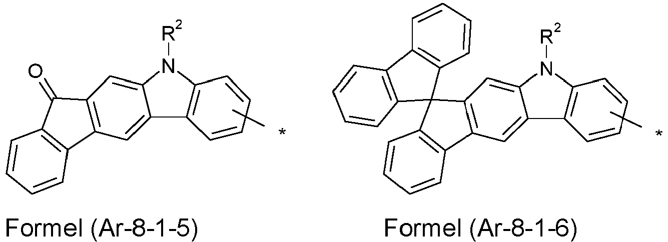 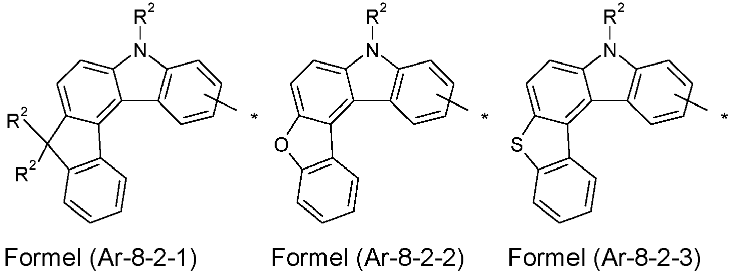 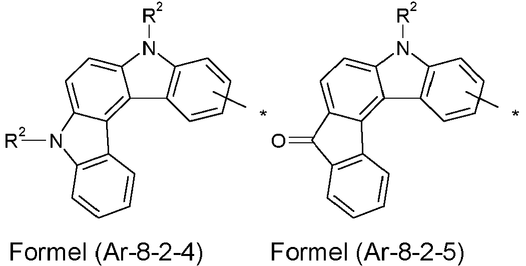 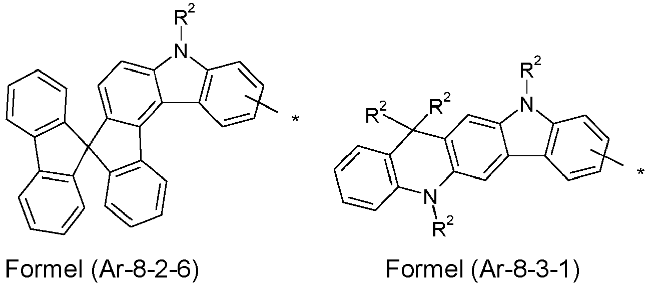 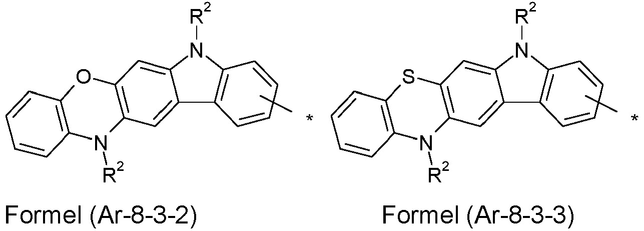 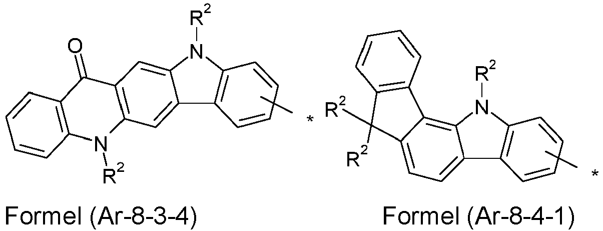 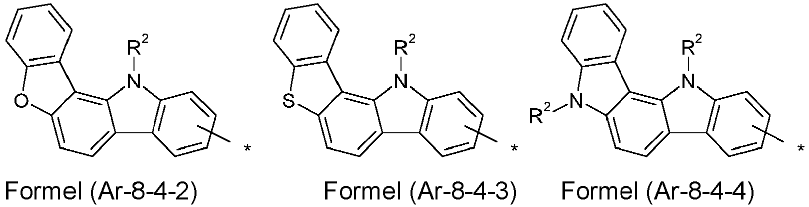 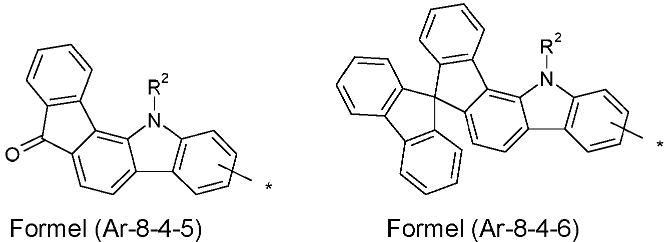 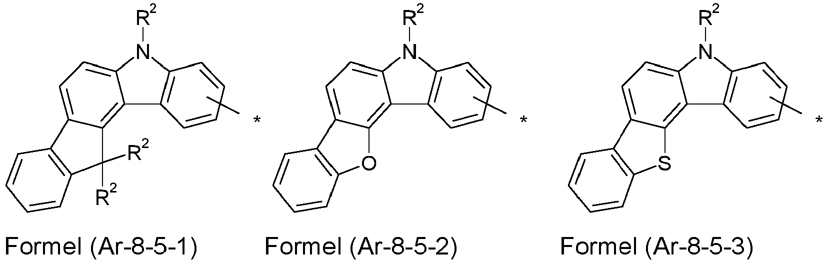 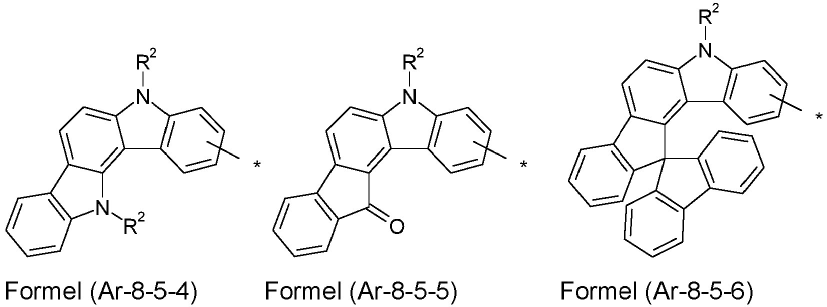 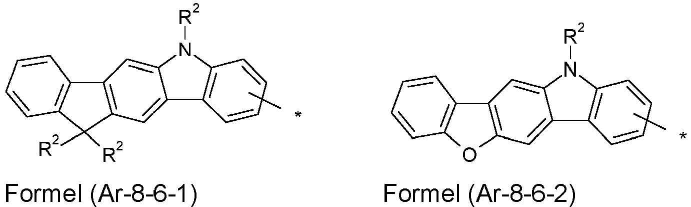 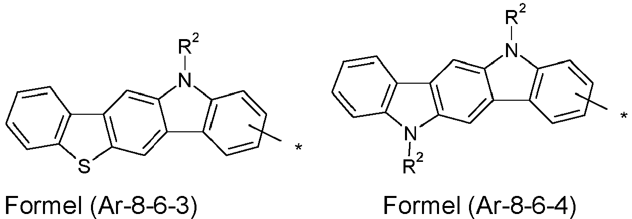 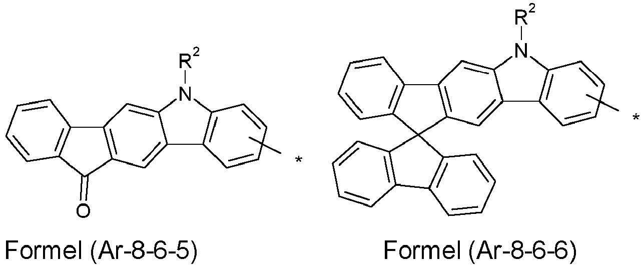 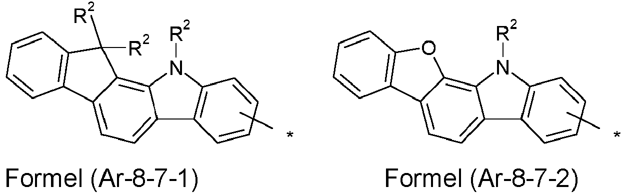 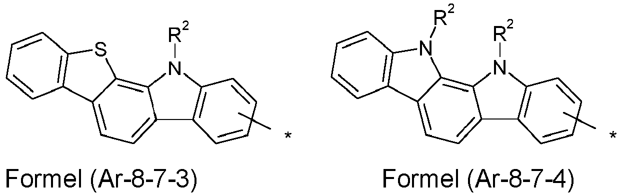 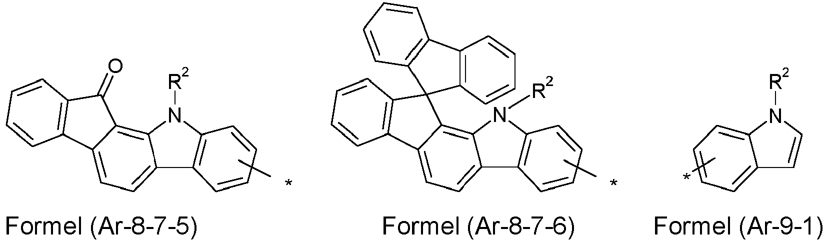 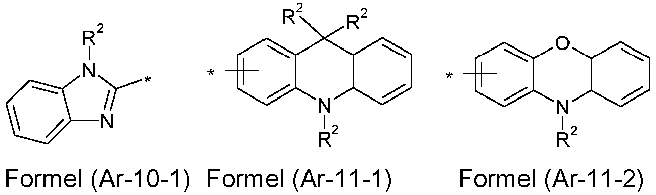 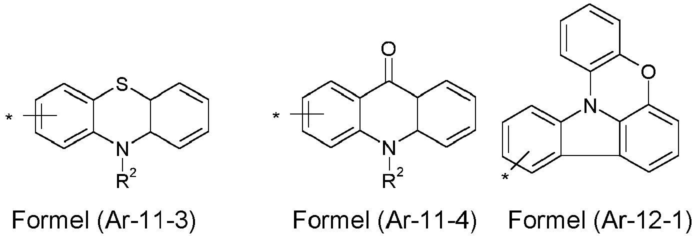 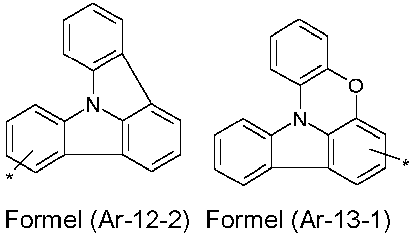 wobei die Symbole den Symbolen in Formel (3) entsprechen. Die Formeln können an den freien Positionen mit R² substituiert sein.

In einer weiteren Ausführungsform der Erfindung sind die Gruppen gemäß Formel (Ar-8), bzw. deren bevorzugte Ausführungsformen ausgewählt aus den Gruppen gemäß einer der Formeln (Ar-8-1-1a) bis (Ar-8-7-6a) 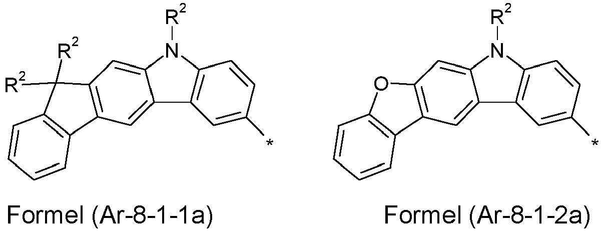 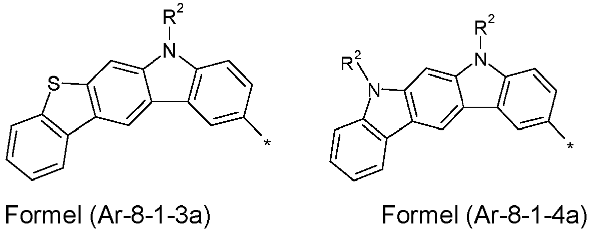 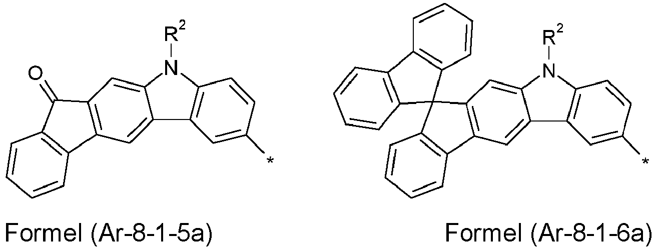 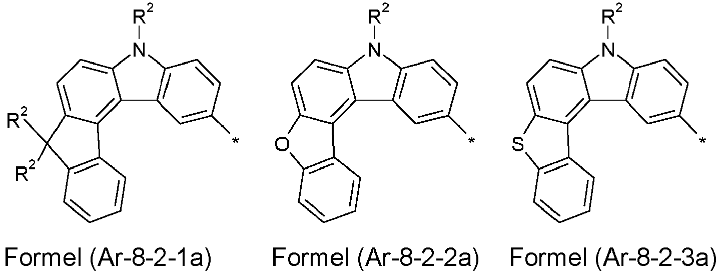 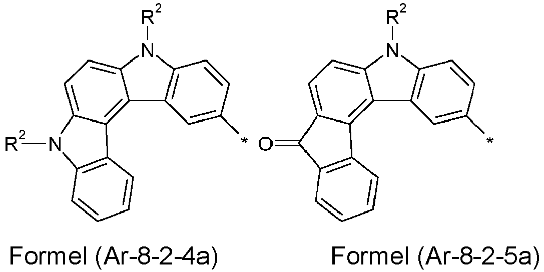 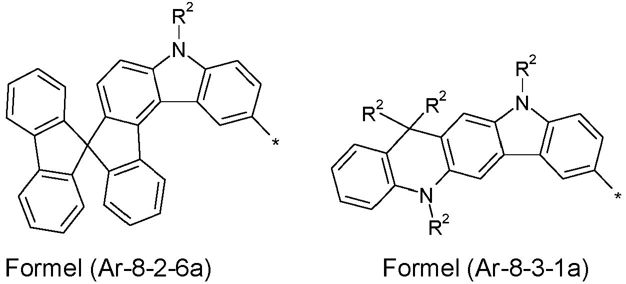 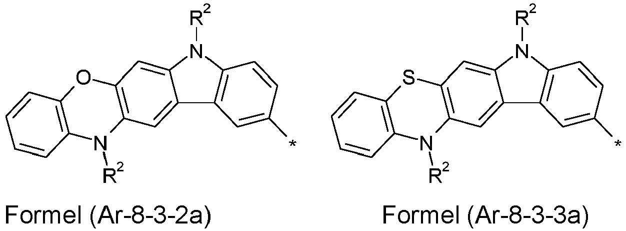 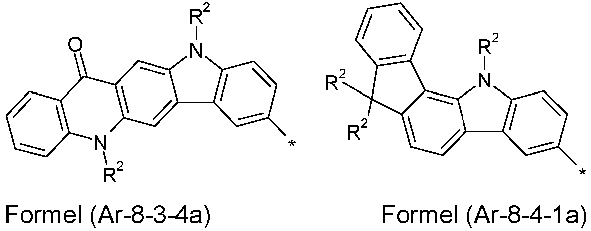 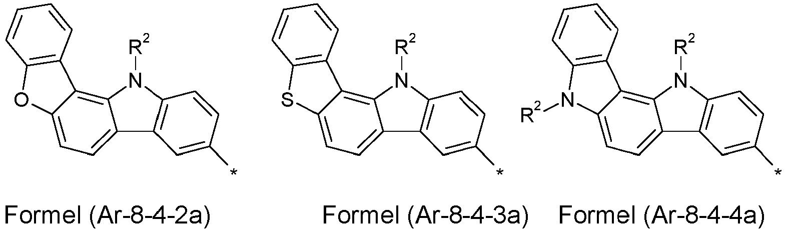 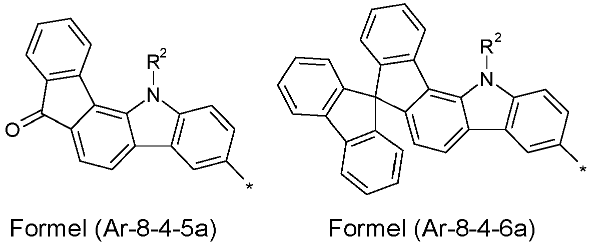 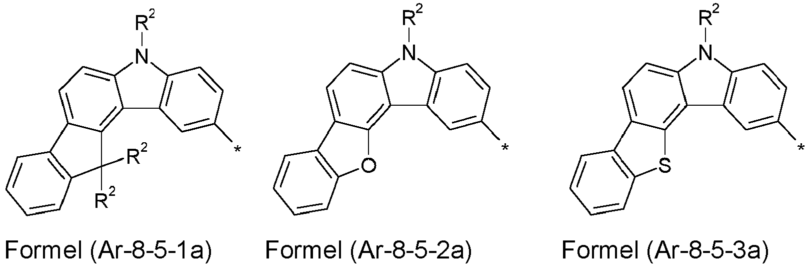 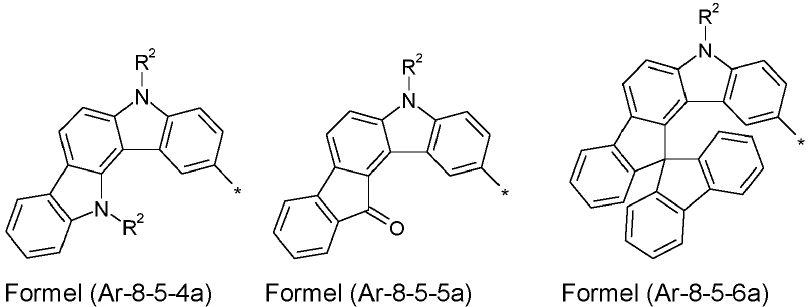 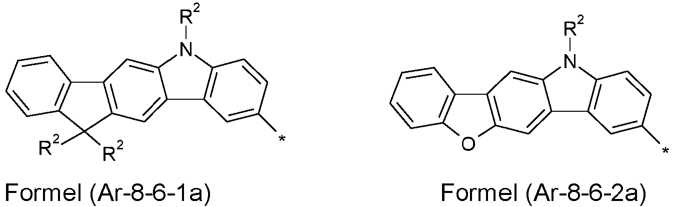 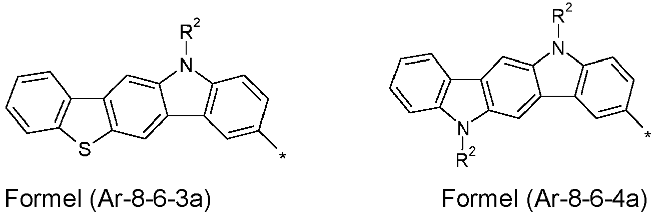 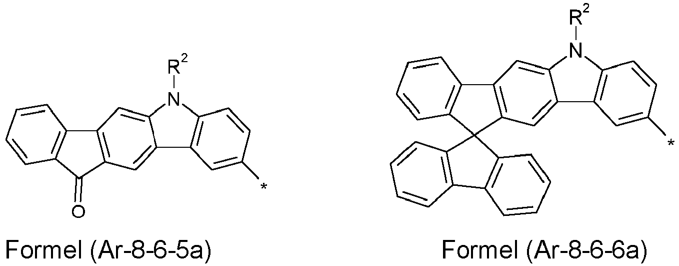 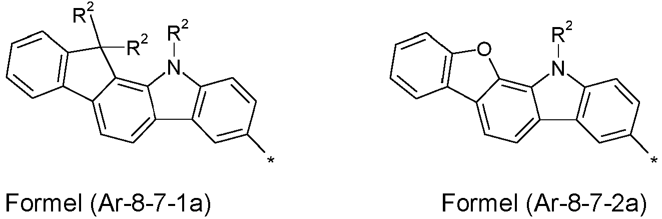 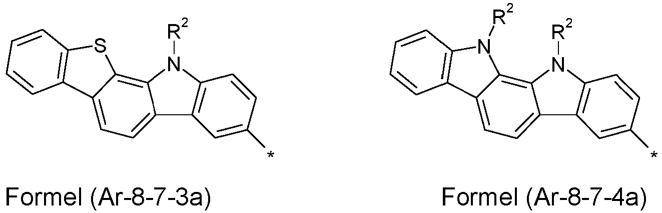 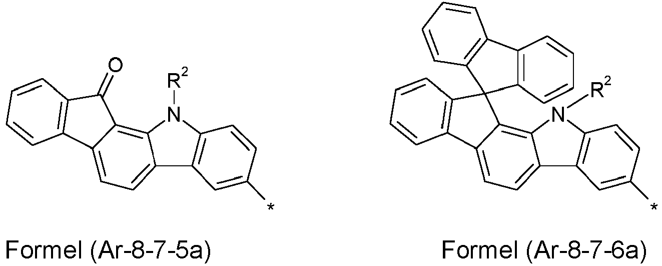 wobei die Symbole den Symbolen in Formel (Ar-8) entsprechen. Die Formeln können an den freien Positionen mit R² substituiert sein.

In einer weiteren Ausführungsform der Erfindung umfasst Ar² kein aromatisches oder heteroaromatisches Ringsystem mit mehr als 19 aromatischen Ringatomen.

In einer weiteren bevorzugten Ausführungsform der Erfindung stehen maximal vier R¹ für ein aromatisches oder heteroaromatisches Ringsystem mit 6 bis 60 aromatischen Ringatomen, das jeweils mit einem oder mehreren Resten R² substituiert sein kann.

Es sind insbesondere keine weiteren aromatischen Ringe an das Phenanthridin-Grundgerüst anelliert.

In einer weiteren bevorzugten Ausführungsform der Erfindung umfasst R¹ im Falle eines aromatischen oder heteroaromatischen Ringsystems Strukturen der Formeln (Ar-1) bis (Ar-13) oder ihrer bevorzugten Ausführungsformen, wobei die Symbole und Indizes der Formeln (Ar-1) bis (Ar-13) gelten und zusätzlich gilt, dass * die Bindung zum Phenanthridin-Grundgerüst darstellt.

In einer besonders bevorzugten Ausführungsform der Erfindung ist R² gleich oder verschieden bei jedem Auftreten ausgewählt aus der Gruppe bestehend aus H, D, F, einer geradkettigen Alkylgruppe mit 1 bis 10 C-Atomen oder einer verzweigten oder cyclischen Alkylgruppe mit 3 bis 10 C-Atomen, die jeweils mit einem oder mehreren Resten R³ substituiert sein kann, einem aromatischen oder heteroaromatischen Ringsystem mit 6 bis 60 C-Atomen, das jeweils mit einem oder mehreren Resten R³ substituiert sein kann.

In einer weiteren bevorzugten Ausführungsform ist R², welcher an eine Kohlenstoffbrücke in einem aromatischen oder heteroaromatischen Ringsystem bindet, wie beispielsweise in den Formeln (Ar-5-1), (Ar-5-2), (Ar-5-3), (Ar-5-4), (Ar-6-1), (Ar-6-2), (Ar-6-3), (Ar-6-4), (Ar-8-3-1), (Ar-11-1), (Ar1-5-1), (Ar2-9) oder (Ar2-9-2), gleich oder verschieden bei jedem Auftreten ausgewählt aus der Gruppe bestehend aus einer geradkettigen Alkylgruppe mit 1 bis 10 C-Atomen, einer verzweigten oder cyclischen Alkylgruppe mit 3 bis 10 C-Atomen oder einem aromatischen Ringsystem mit 6 bis 30 C-Atomen, das wie oben definiert ist und das mit einem oder mehreren Resten R³ substituiert sein kann.

In einer weiteren bevorzugten Ausführungsform ist R², welcher an ein Stickstoffatom bindet, ausgewählt aus der Gruppe bestehend aus einer geradkettigen Alkylgruppe mit 1 bis 10 C-Atomen, einer verzweigten oder cyclischen Alkylgruppe mit 3 bis 10 C-Atomen oder einem aromatischen Ringsystem mit 6 bis 30 C-Atomen, insbesondere ein aromatisches Ringsystem mit 6 bis 24 C-Atomen, das wie oben definiert ist, und das mit einem oder mehreren Resten R³ substituiert sein kann.

Die oben genannten Ausführungsformen können beliebig miteinander kombiniert werden. Insbesondere ist es bevorzugt, die oben aufgeführten bevorzugten Ausführungsformen miteinander zu kombinieren.

Beispiele für bevorzugte Verbindungen gemäß den oben aufgeführten Ausführungsformen bzw. Verbindungen, wie sie bevorzugt in organischen elektronischen Vorrichtungen eingesetzt werden können, sind die folgenden Verbindungen (* Referenzmaterialien):

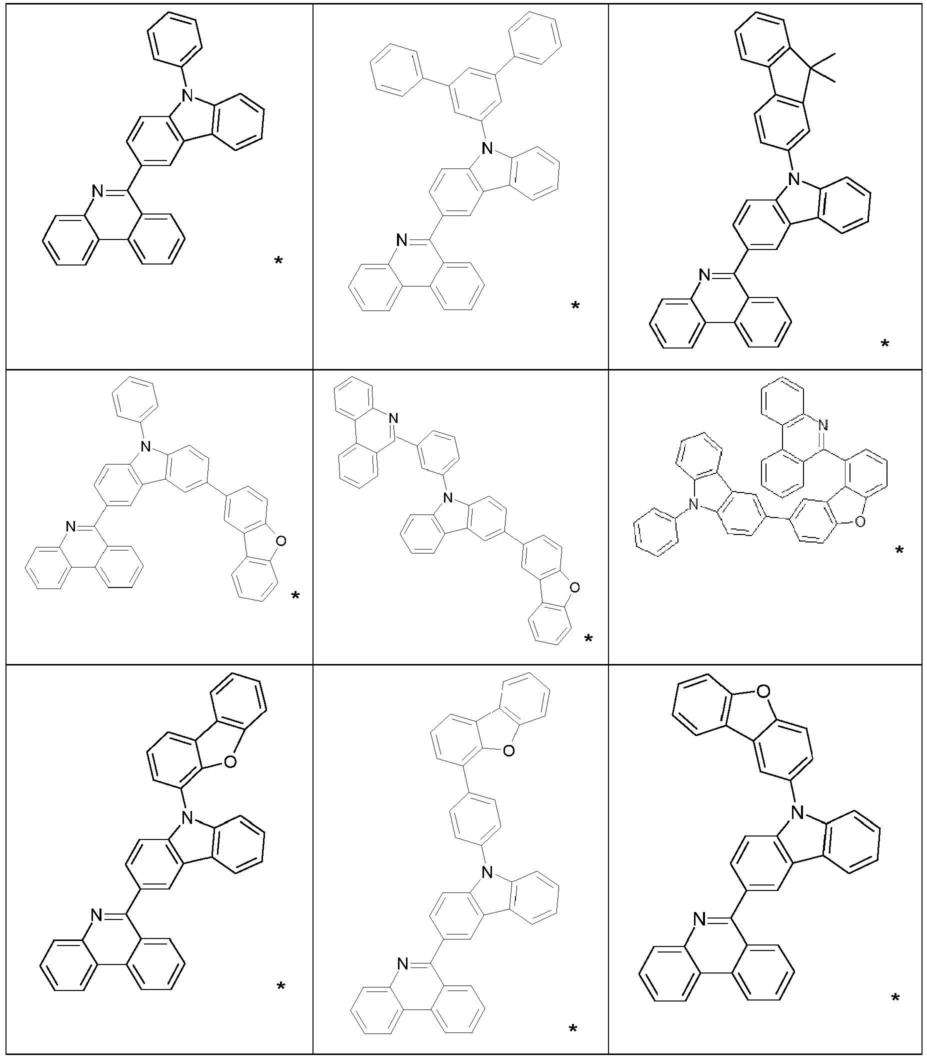

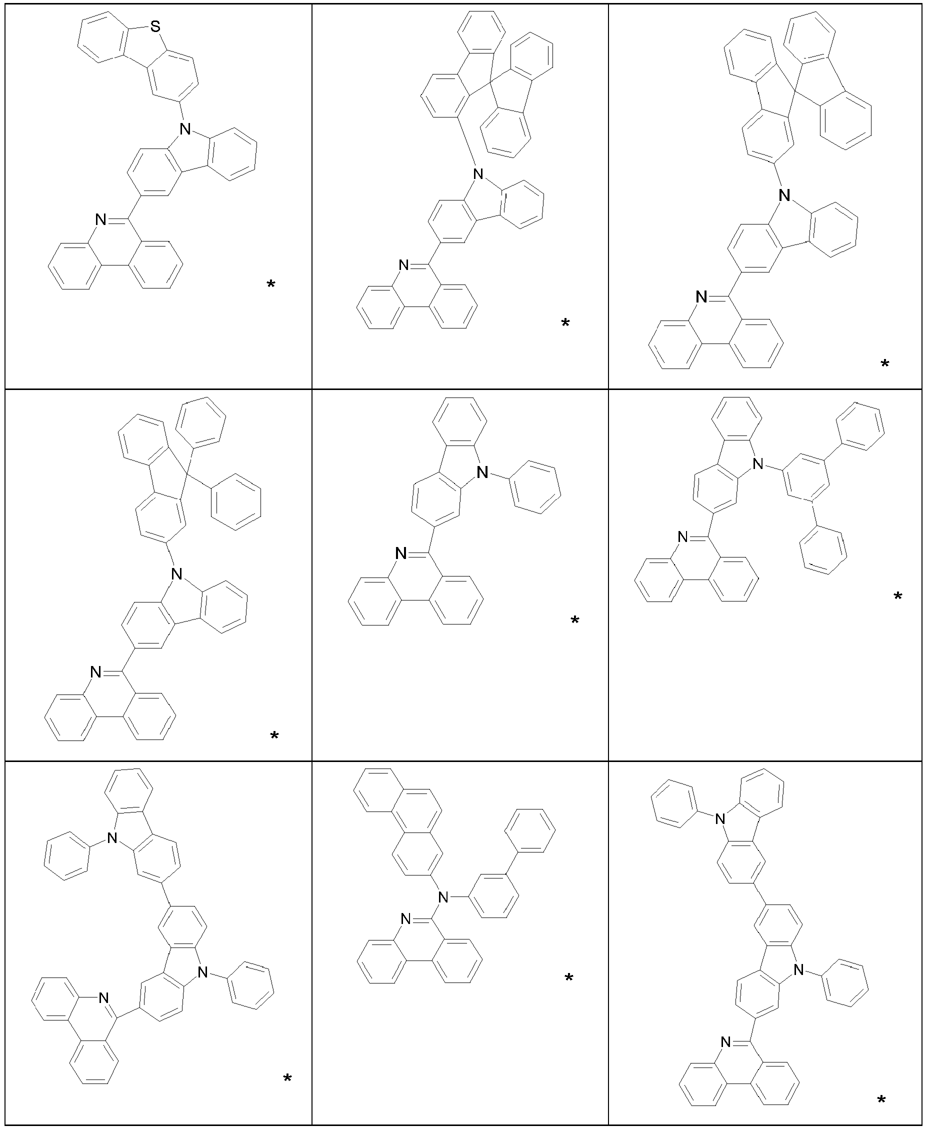

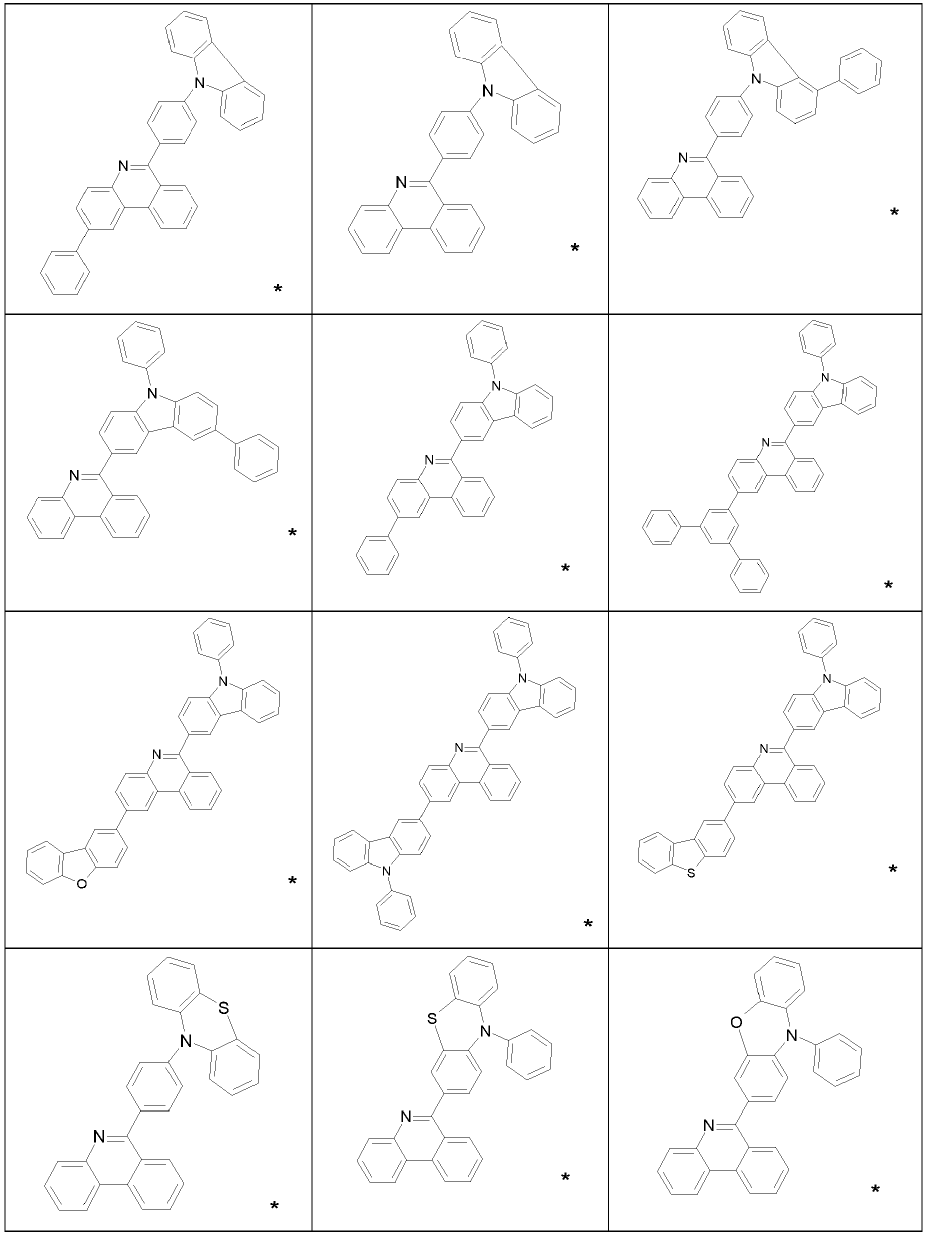

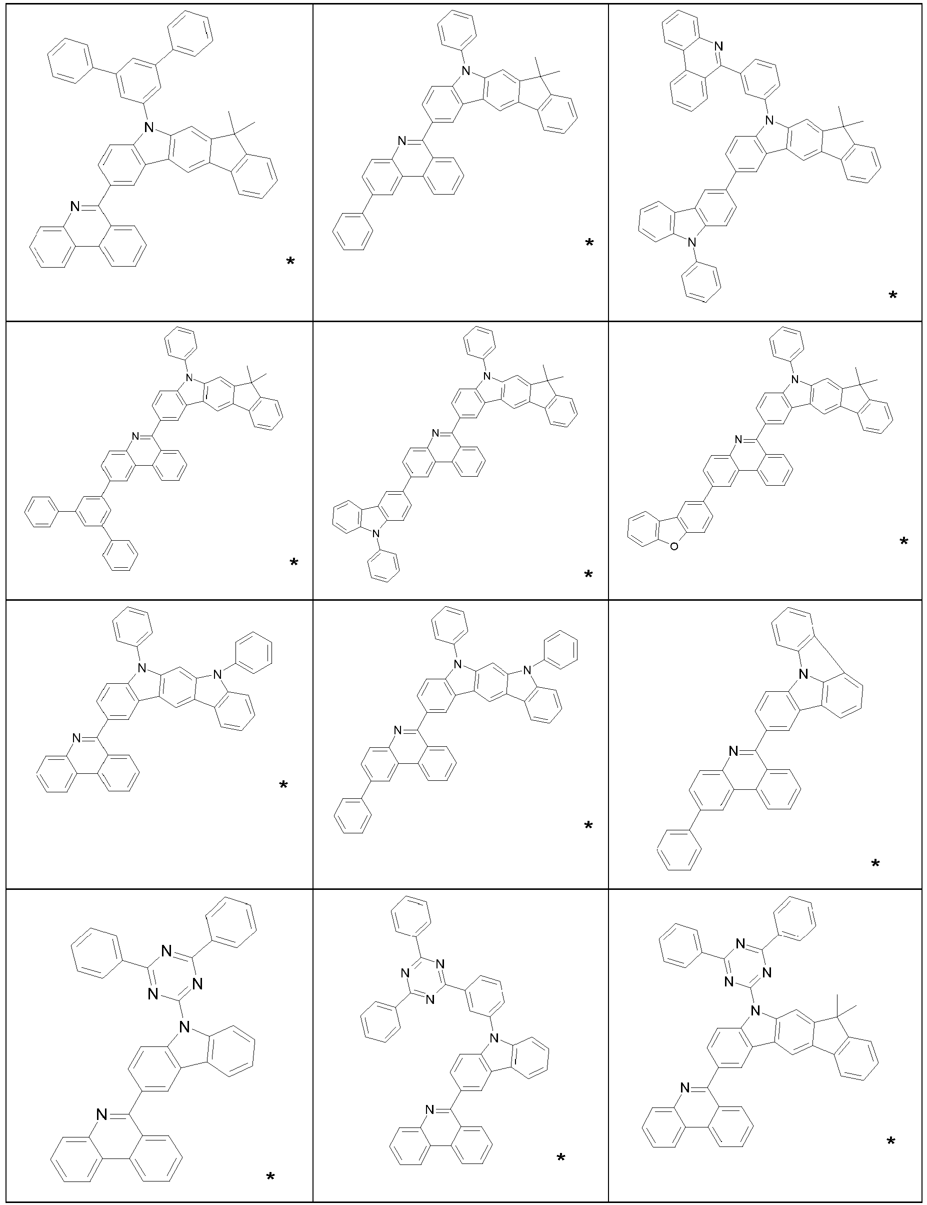

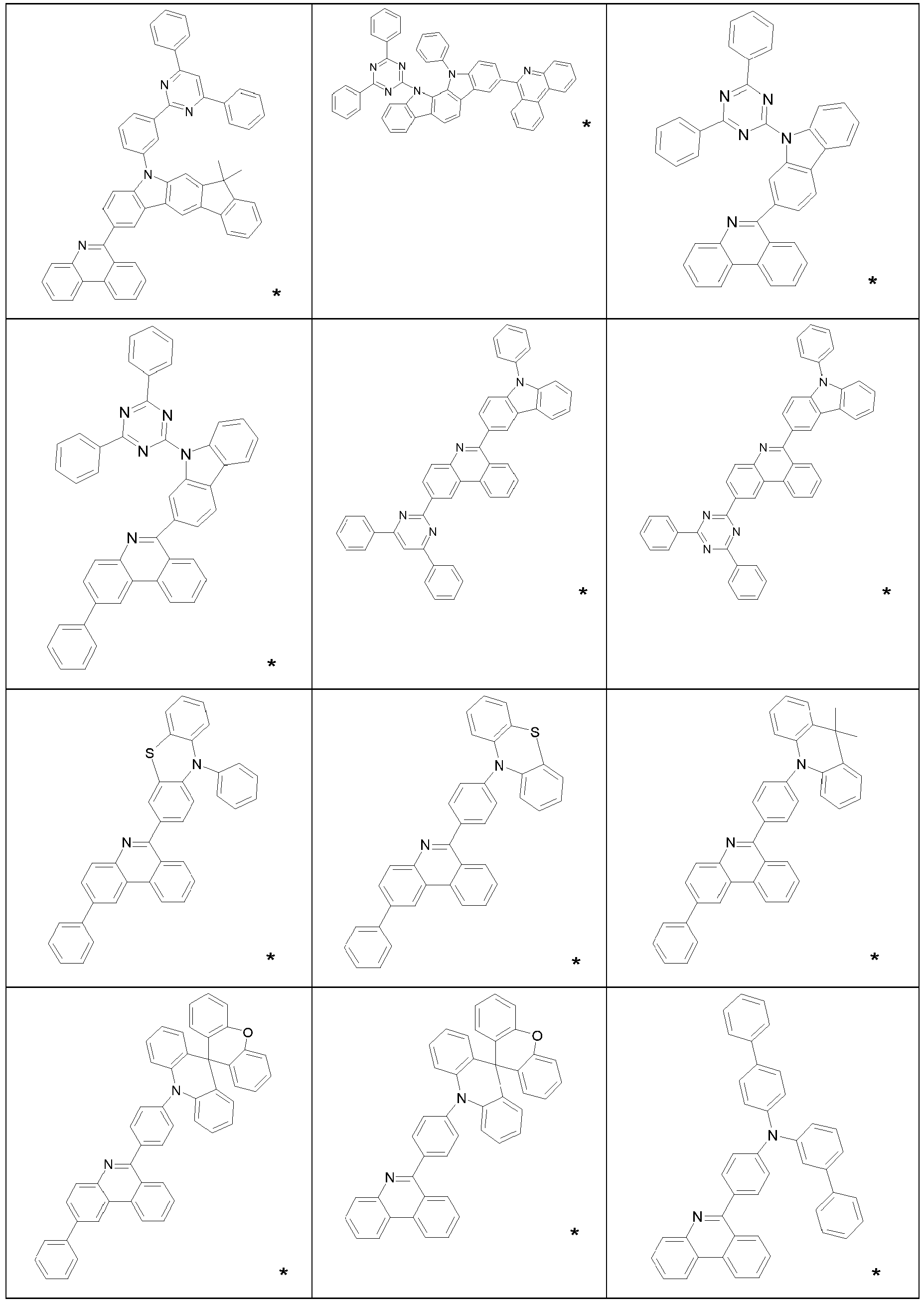

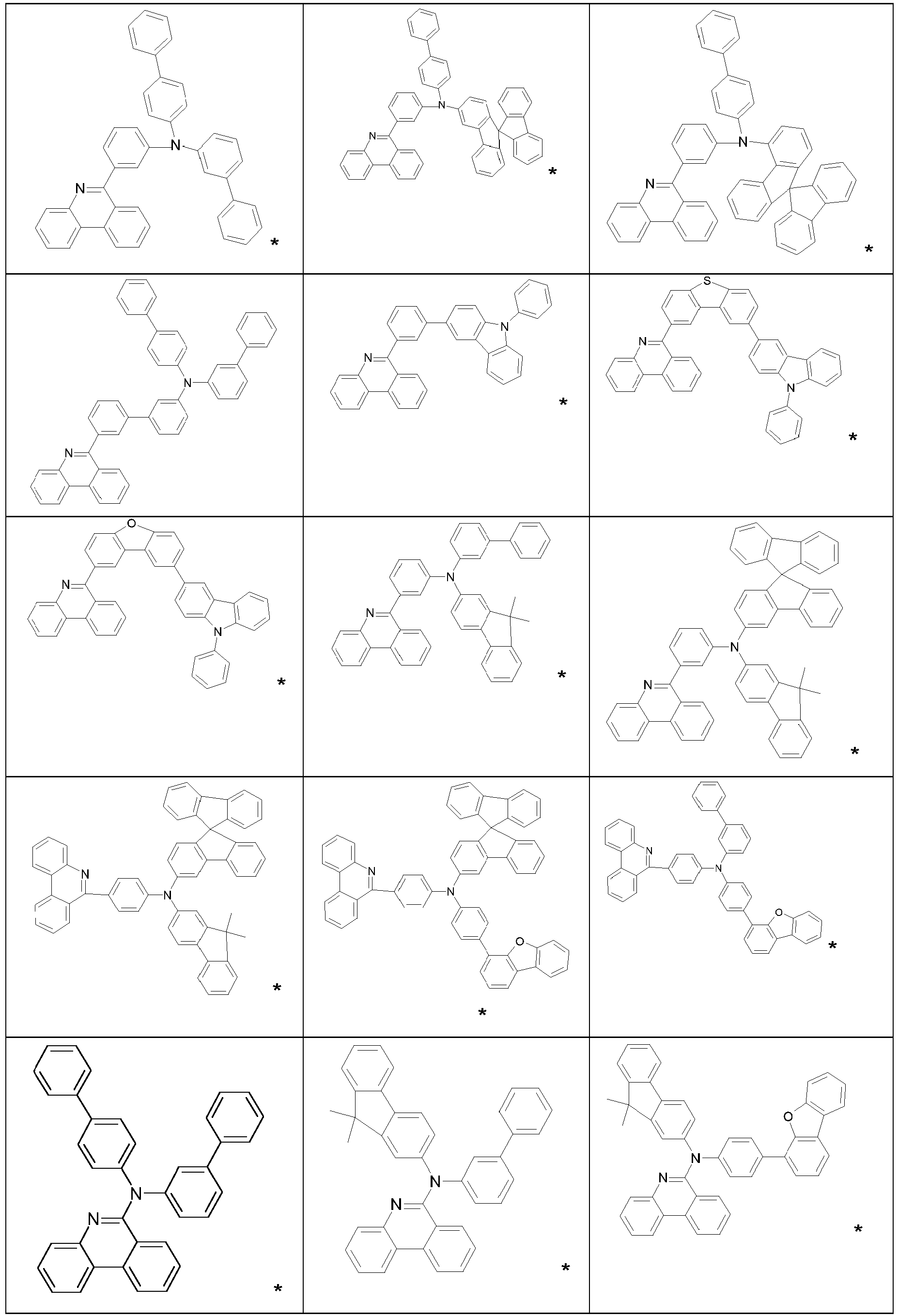

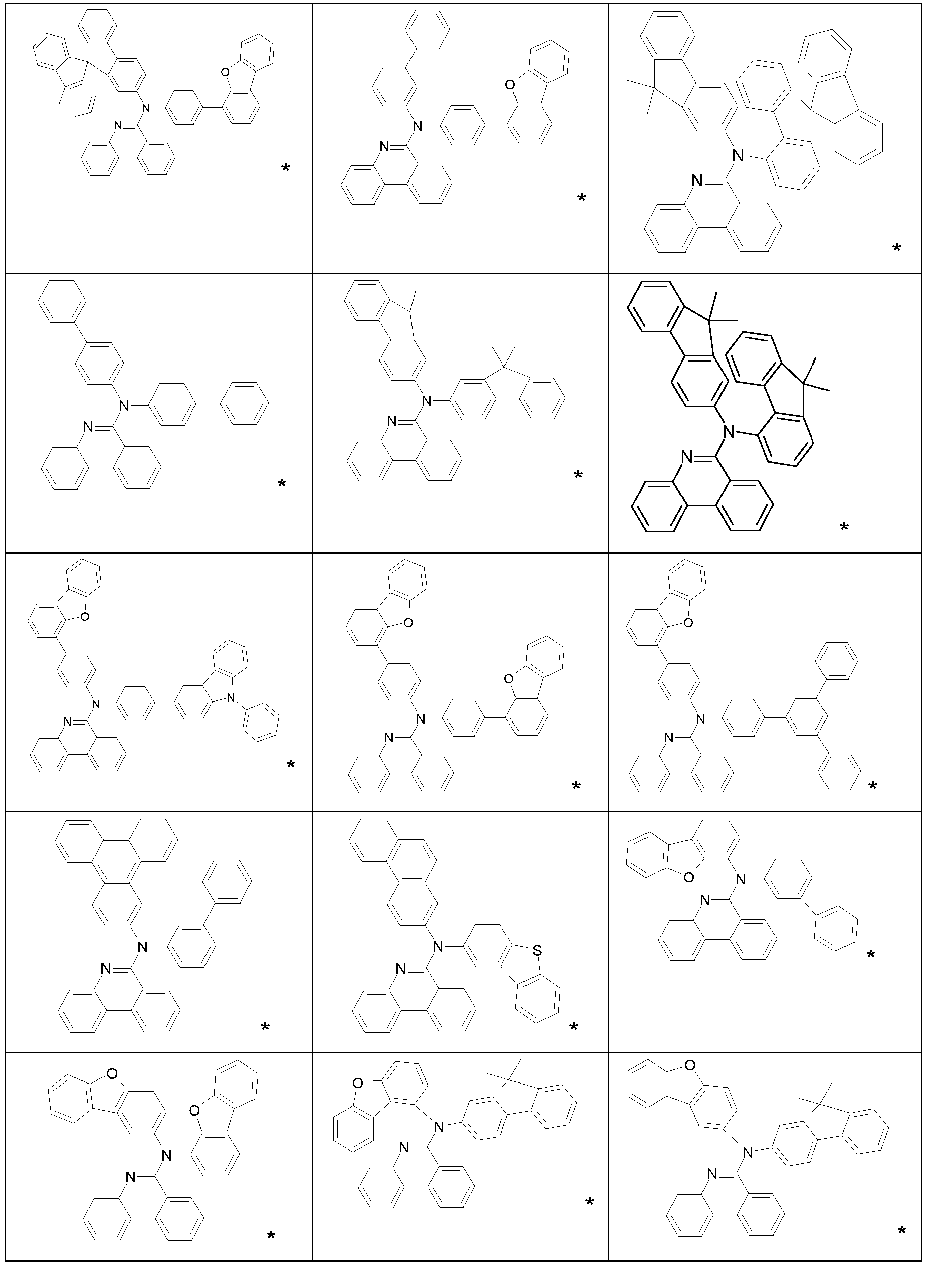

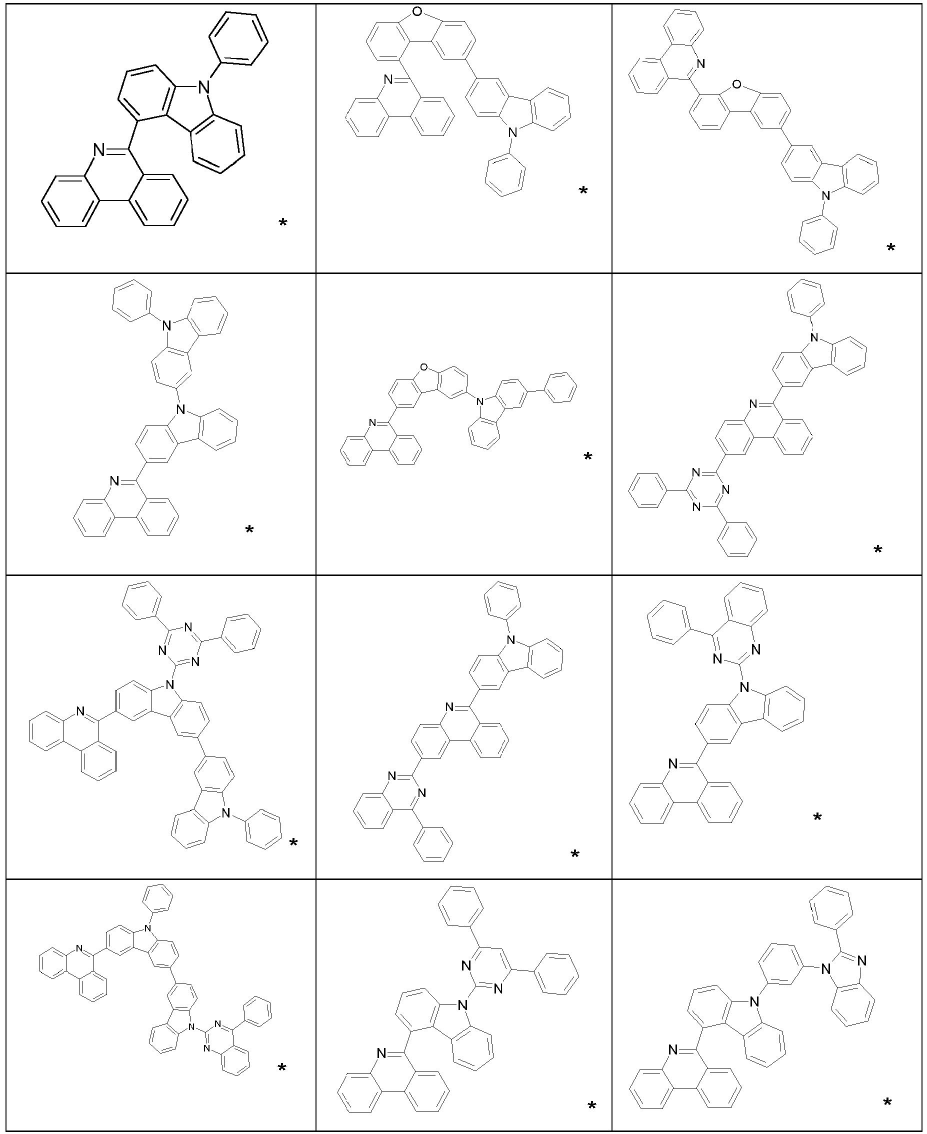

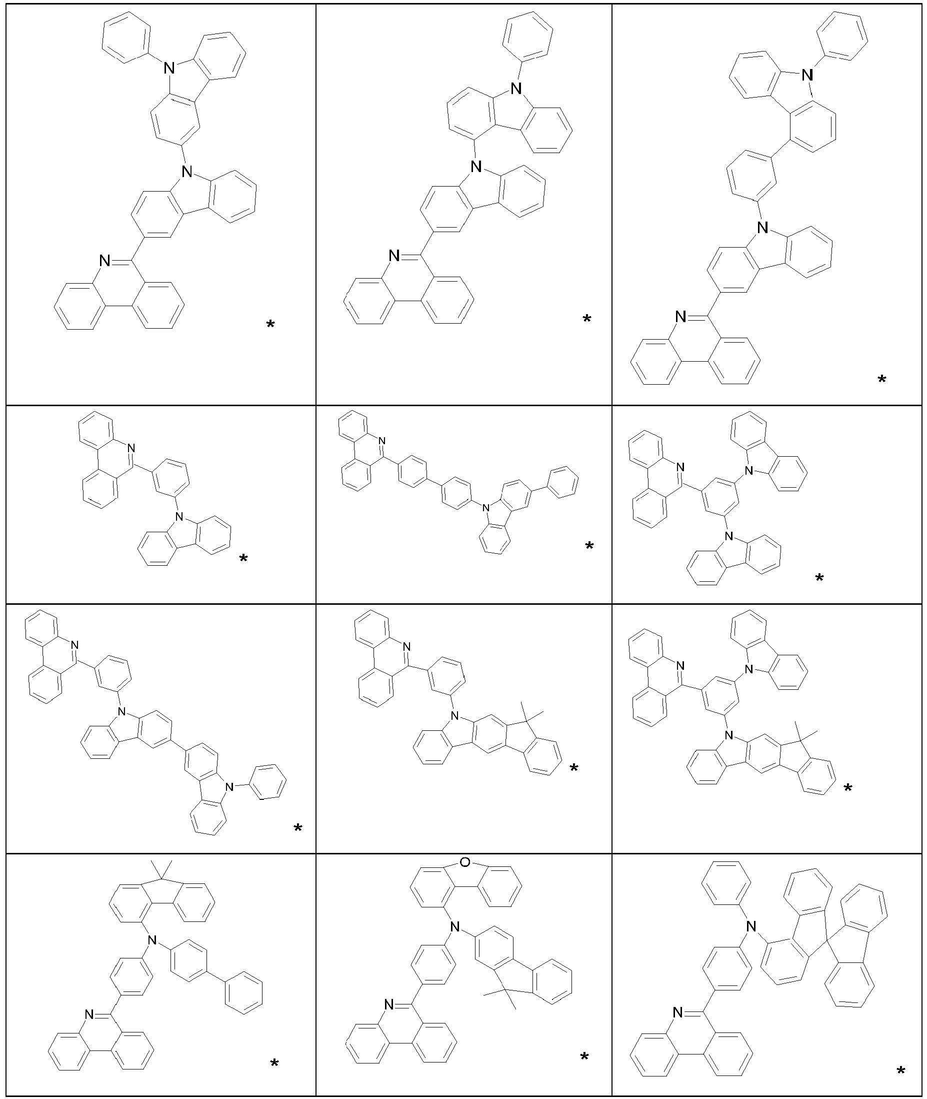

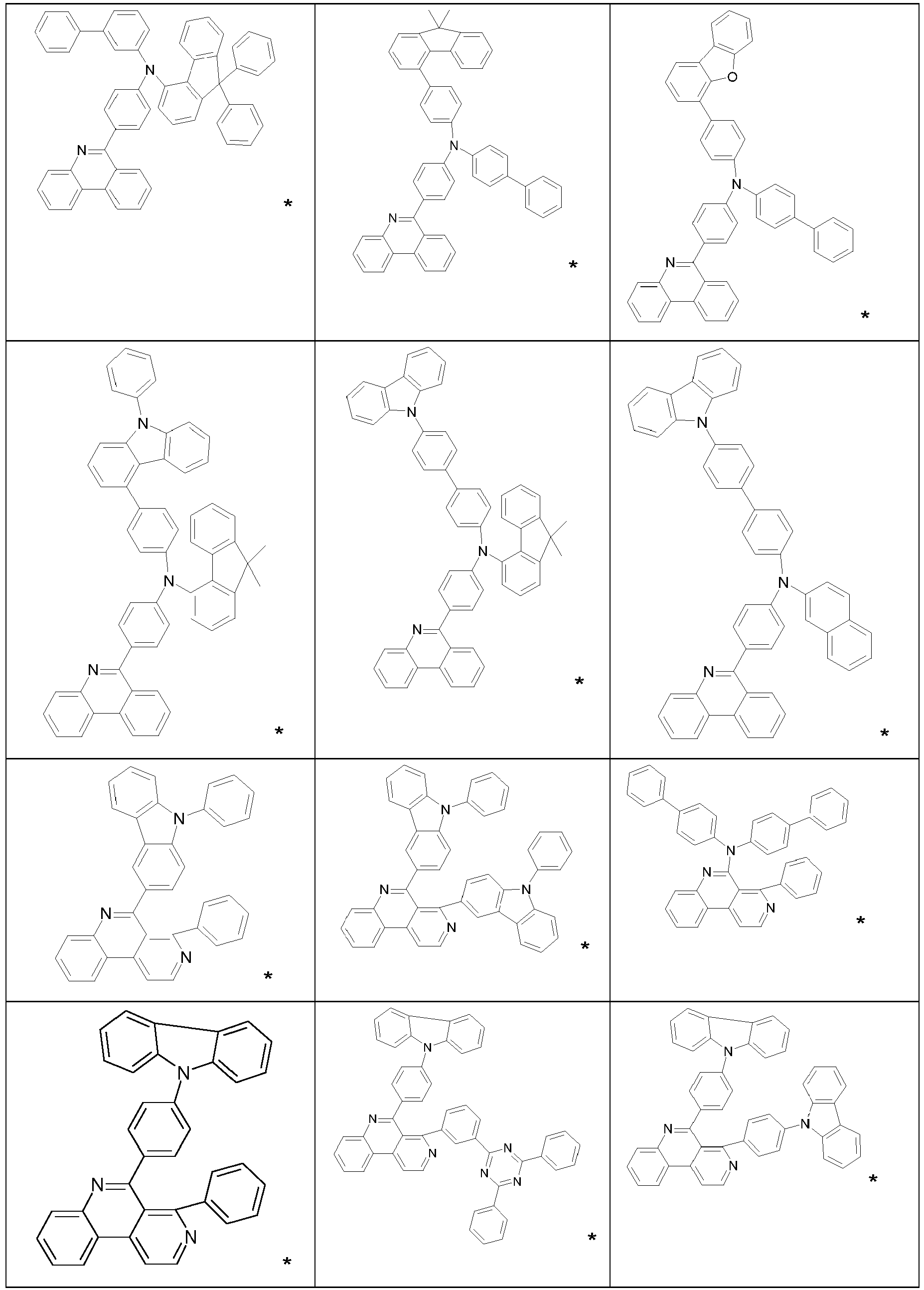

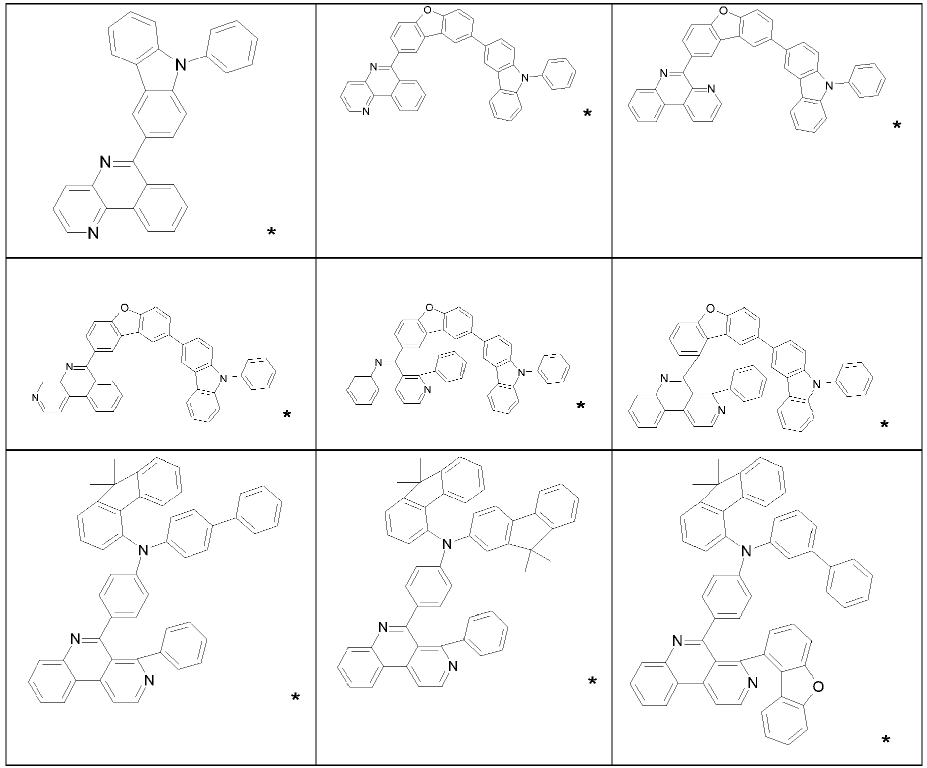

Die erfindungsgemäßen Verbindungen können nach dem Fachmann bekannten Syntheseschritten, wie z. B. Bromierung, Suzuki-Kupplung, Ullmann-Kupplung, Hartwig-Buchwald-Kupplung, etc., dargestellt werden.

Die Herstellung des Phenanthridin-Grundgerüsts erfolgt beispielsweise ausgehend von Fluoren-9-on mit nachfolgender Bildung eines Amids durch eine Beckmann-Umlagerung. Die Ketogruppe des erhaltenen Amids kann in eine Abgangsgruppe, beispielsweise ein Halogenid, überführt werden. Durch eine oder mehrere Kupplungsreaktionen kann dann eine Verbindung der Formel (3) erhalten werden.

Ein weiterer Gegenstand der vorliegenden Erfindung ist daher ein Verfahren zur Herstellung einer Verbindung gemäß Formel (3) wobei die Verbindung der Formel (3) durch eine oder mehrere Kupplungsreaktionen, Umlagerungen und/oder Zyklisierungen aufgebaut wird.

Die oben gezeigten Syntheseverfahren haben exemplarischen Charakter und können vom Fachmann auf dem Gebiet der organischen Synthese in geeigneter Weise abgewandelt werden, wenn dies für die Synthese bestimmter Ausführungsformen von erfindungsgemäßen Verbindungen vorteilhaft ist.

Die oben beschriebenen erfindungsgemäßen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. CC-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterere Gegenstände der Erfindung sind daher Oligomere, Polymere oder Dendrimere enthaltend eine oder mehrere Verbindungen gemäß Formel (3), wobei die Bindung(en) zum Polymer, Oligomer oder Dendrimer an beliebigen freien Positionen in Formel (3) lokalisiert sein können. Je nach Verknüpfung der erfindungsgemäßen Verbindung ist die Verbindung Bestandteil einer Seitenkette des Oligomers oder Polymers oder Bestandteil der Hauptkette.

Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist.

Die erfindungsgemäßen Polymere, Oligomere oder Dendrimere können konjugiert, teilkonjugiert oder nicht-konjugiert sein. Die erfindungsgemäßen Oligomere oder Polymere können linear, verzweigt oder dendritisch sein.

In den linear verknüpften Strukturen können die Einheiten gemäß Formel (3) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein.

In verzweigten und dendritischen Strukturen können beispielsweise 3, 5 oder mehrere Einheiten gemäß Formel (3) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (3) in Oligomeren, Dendrimeren und Polymeren gelten dieselben Bevorzugungen wie oben für die erfindungsgemäßen Verbindungen beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 2000/22026), Spirobifluorenen (z. B. gemäß EP 707020, EP 894107 oder WO 2006/061181), Paraphenylenen (z. B. gemäß WO 1992/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere, Oligomere und Dendrimere weisen vorteilhafte Eigenschaften, insbesondere hohe Lebensdauern, hohe Effizienzen und gute Farbkoordinaten auf.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (3) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation
(B) YAMAMOTO-Polymerisation
(C) STILLE-Polymerisation und
(D) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 2003/048225, WO 2004/037887 und WO 2004/037887, im Detail beschrieben.

Gegenstand der vorliegenden Erfindung ist somit auch ein Verfahren zur Herstellung der erfindungsgemäßen Polymere, Oligomere und Dendrimere, das dadurch gekennzeichnet ist, dass sie durch Polymerisation gemäß SUZUKI, Polymerisation gemäß YAMAMOTO, Polymerisation gemäß STILLE oder Polymerisation gemäß HARTWIG-BUCHWALD hergestellt werden. Die erfindungsgemäßen Dendrimere können durch dem Fachmann bekannte Verfahren oder in Analogie dazu hergestellt werden. Geeignete Verfahren sind in der Literatur beschrieben, wie z. B. in Frechet, Jean M. J.; Hawker, Craig J., "Hyperbranched polyphenylene and hyperbranched polyesters: new soluble, three-dimensional, reactive polymers", Reactive & Functional Polymers (1995), 26(1-3), 127-36; Janssen, H. M.; Meijer, E. W., "The synthesis and characterization of dendritic molecules", Materials Science and Technology (1999), 20 (Synthesis of Polymers), 403-458; Tomalia, Donald A., "Dendrimer molecules", Scientific American (1995), 272(5), 62-6; WO 2002/067343 A1 und WO 2005/026144 A1 .

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethyl-ether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion enthaltend mindestens eine Verbindung gemäß Formel (3) oder mindestens ein Polymer, Oligomer oder Dendrimer enthaltend mindestens eine Einheit gemäß Formel (3) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Ein weiterer Gegenstand der vorliegenden Erfindung sind Mischungen enthaltend mindestens eine erfindungsgemäße Verbindung und mindestens eine weitere Verbindung. Die weitere Verbindung kann beispielsweise ein fluoreszierender oder phosphoreszierender Dotand sein, wenn die erfindungsgemäße Verbindung als Matrixmaterial verwendet wird, insbesondere ein phosphoreszierender Dotand. Geeignete Dotanden sind unten im Zusammenhang mit den organischen Elektrolumineszenzvorrichtungen aufgeführt und sind auch für die erfindungsgemäßen Mischungen bevorzugt.

Die erfindungsgemäßen Verbindungen und Mischungen eignen sich für die Verwendung in einer elektronischen Vorrichtung. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Ein weiterer Gegenstand der vorliegenden Erfindung ist daher die Verwendung der erfindungsgemäßen Verbindungen oder Mischungen in einer elektronischen Vorrichtung, insbesondere in einer organischen Elektrolumineszenzvorrichtung.

Nochmals ein weiterer Gegenstand der vorliegenden Erfindung ist eine elektronische Vorrichtung enthaltend mindestens eine der oben ausgeführten erfindungsgemäßen Verbindungen oder Mischungen. Dabei gelten die oben für die Verbindung ausgeführten Bevorzugungen auch für die elektronischen Vorrichtungen.

Die elektronische Vorrichtung ist bevorzugt ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen farbstoff-sensibilisierten Solarzellen, organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices" (D. M. Koller et al., Nature Photonics 2008, 1-4), bevorzugt aus organischen Elektrolumineszenzvorrichtungen (OLEDs, PLEDs), insbesondere phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Zwischenschichten (Interlayer) eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, sodass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen (für den prinzipiellen Aufbau siehe z. B. WO 2005/011013). Es kann sich dabei um fluoreszierende oder um phosphoreszierende Emissionsschichten handeln oder um Hybrid-Systeme, bei denen fluoreszierende und phosphoreszierende Emissionsschichten miteinander kombiniert werden.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (3) oder gemäß den bevorzugten Ausführungsformen als Matrixmaterial für fluoreszierende oder phosphoreszierende Emitter, insbesondere für phosphoreszierende Emitter, und/oder in einer Elektronentransportschicht und/oder in einer elektronenblockierenden bzw. exzitonenblockierenden Schicht und/oder in einer Lochtransportschicht, je nach genauer Substitution, besonders bevorzugt als Matrixmaterial für fluoreszierende oder phosphoreszierende Emitter, insbesondere für phosphoreszierende Emitter, und/oder in einer Elektronentransportschicht und/oder in einer Lochtransportschicht. Dabei gelten die oben aufgeführten bevorzugten Ausführungsformen auch für die Verwendung der Materialien in organischen elektronischen Vorrichtungen.

In einer bevorzugten Ausführungsform der Erfindung wird die Verbindung gemäß Formel (3) bzw. gemäß den bevorzugten Ausführungsformen als Matrixmaterial für eine fluoreszierende oder phosphoreszierende Verbindung, insbesondere für eine phosphoreszierende Verbindung, in einer emittierenden Schicht eingesetzt. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält.

Eine weitere bevorzugte Ausführungsform der vorliegenden Erfindung ist der Einsatz der Verbindung gemäß Formel (3) bzw. gemäß den bevorzugten Ausführungsformen als Matrixmaterial für einen phosphoreszierenden Emitter in Kombination mit einem weiteren Matrixmaterial. Besonders geeignete Matrixmaterialien, welche in Kombination mit den Verbindungen gemäß Formel (3) bzw. gemäß den bevorzugten Ausführungsformen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109 und WO 2011/000455, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte Carbazol-Derivate, z. B. gemäß US 2009/0136779, WO 2010/050778, WO 2011/042107, WO 2011/088877 oder WO 2012/143080, Triphenylenderivaten, z. B. gemäß WO 2012/048781, oder Lactame, z. B. gemäß WO 2011/116865 oder WO 2011/137951. Ebenso kann ein weiterer phosphoreszierender Emitter, welcher kürzerwellig als der eigentliche Emitter emittiert, als Co-Host in der Mischung vorhanden sein.

Die Mischung aus der Verbindung gemäß Formel (3) bzw. gemäß den bevorzugten Ausführungsformen und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der Verbindung gemäß Formel (3) bzw. gemäß den bevorzugten Ausführungsformen bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

Vom Begriff phosphoreszierende Dotanden (Emitter) sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Dotanden eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Dotanden Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Beispiele für phosphoreszierende Dotanden können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen. Auch kann der Fachmann ohne erfinderisches Zutun weitere phosphoreszierende Komplexe in Kombination mit den erfindungsgemäßen Verbindungen in OLEDs einsetzen.

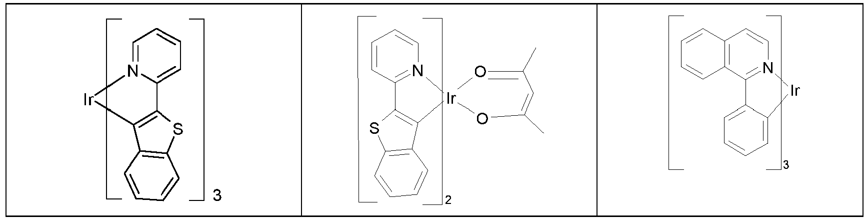

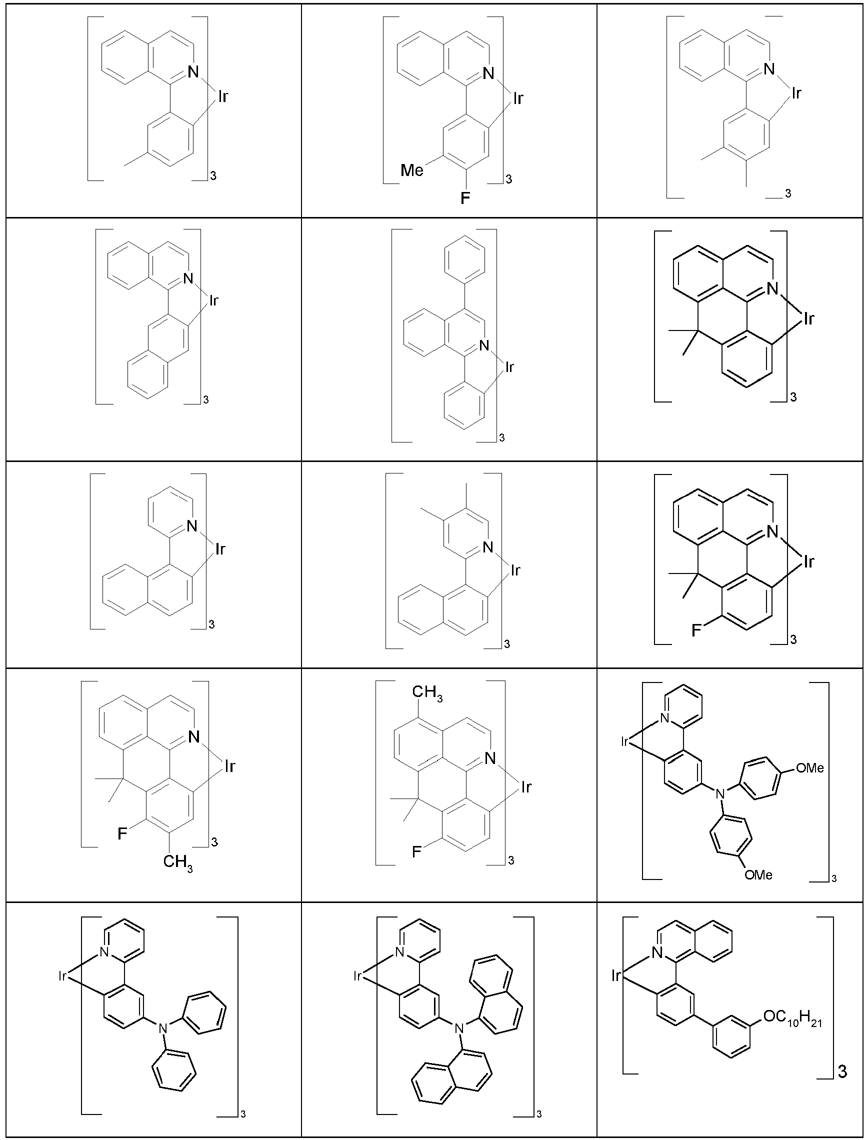

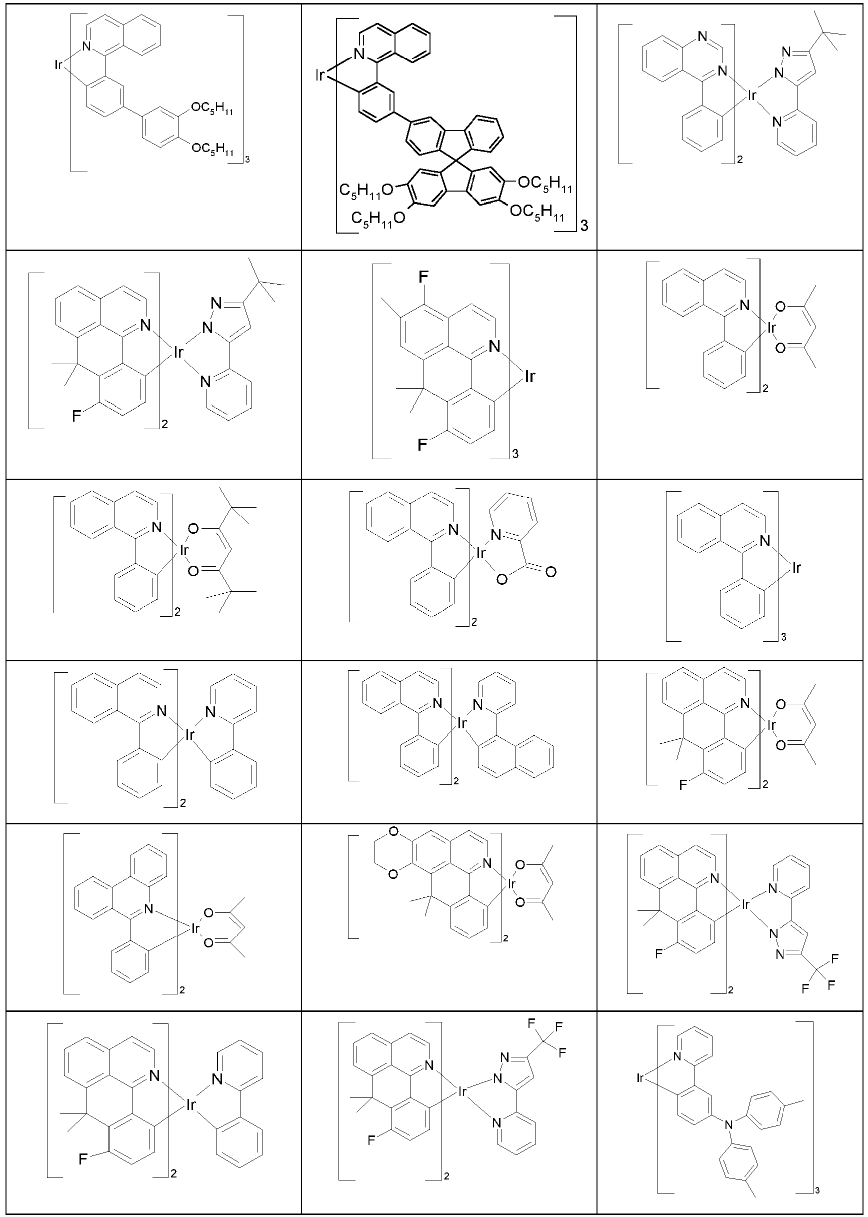

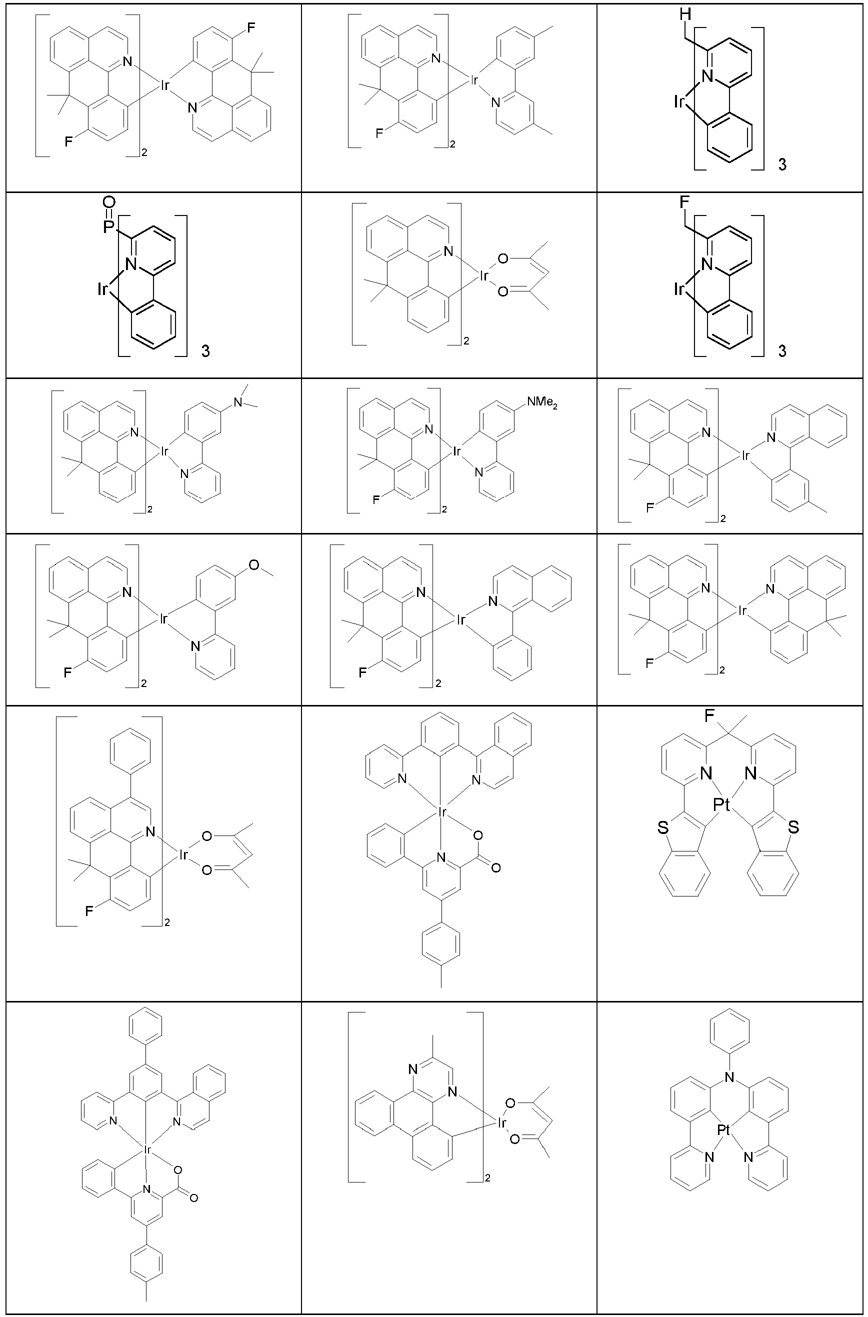

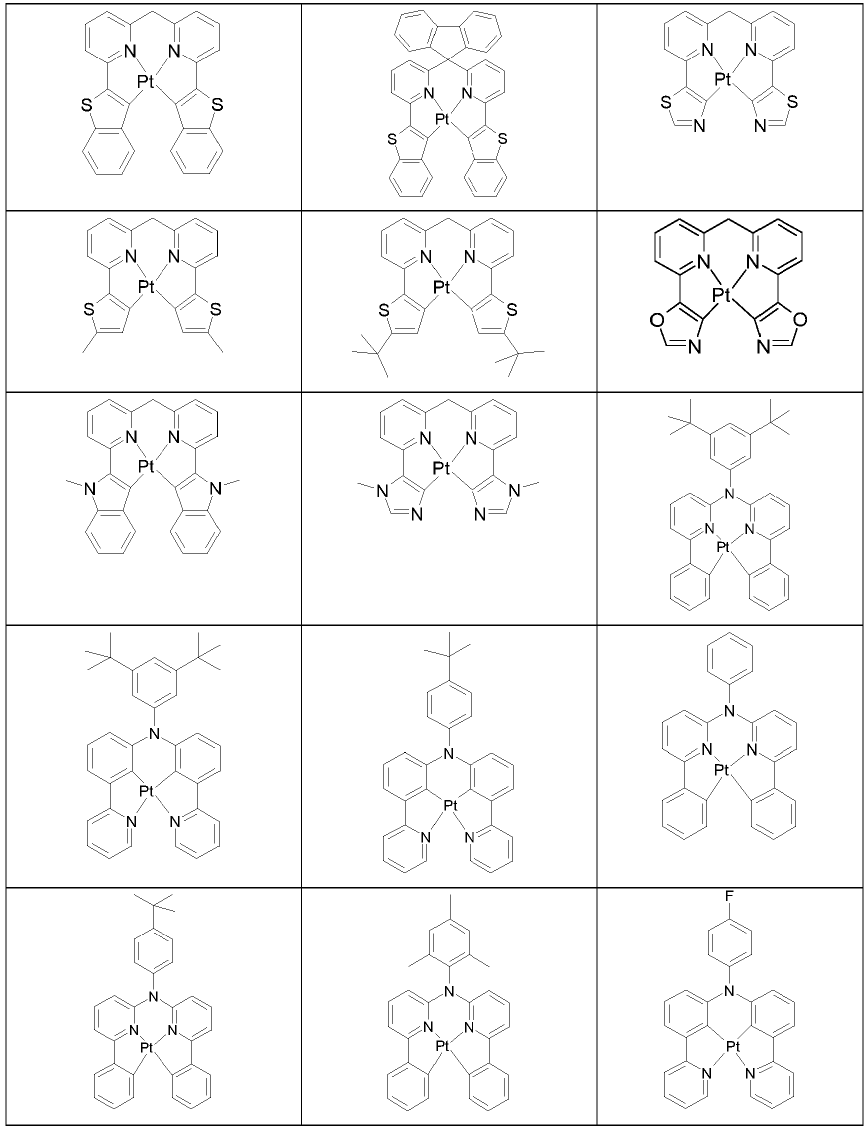

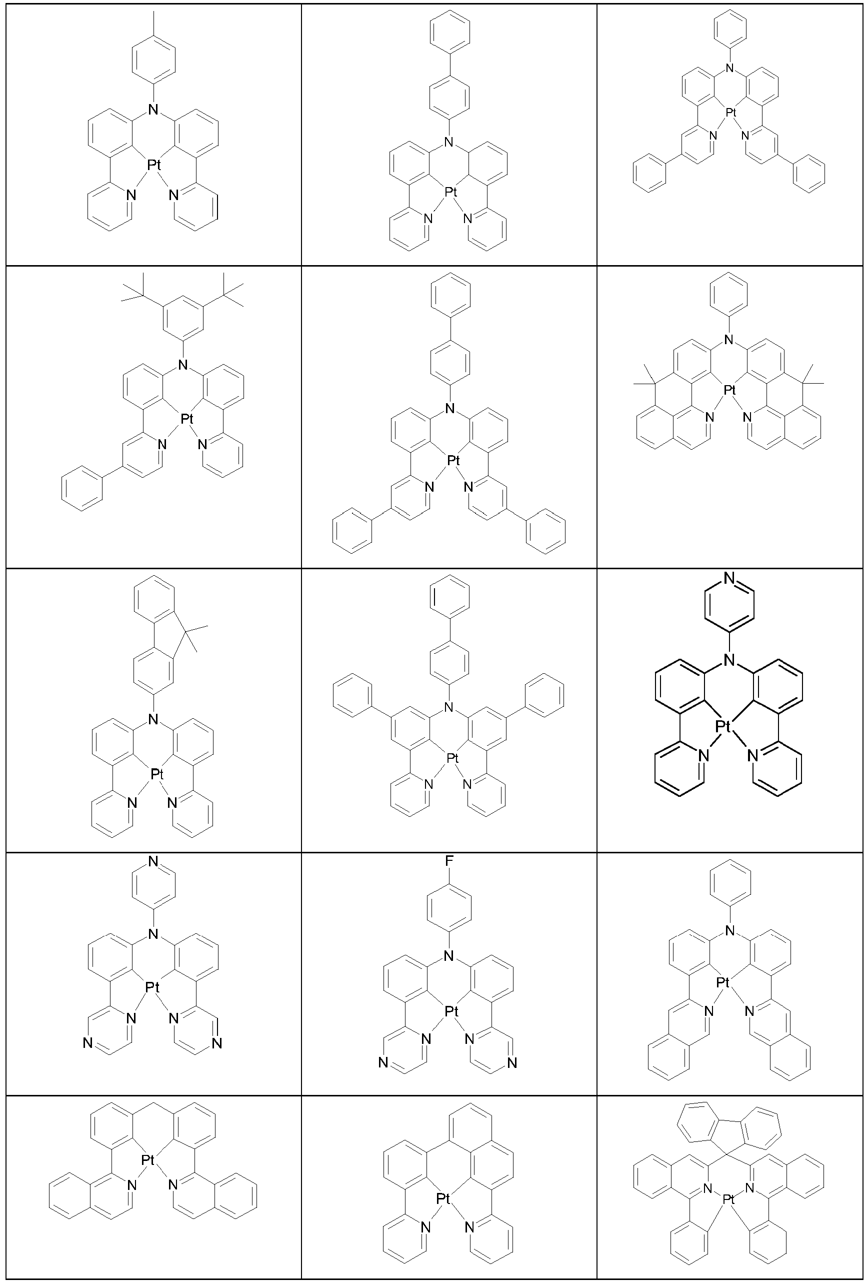

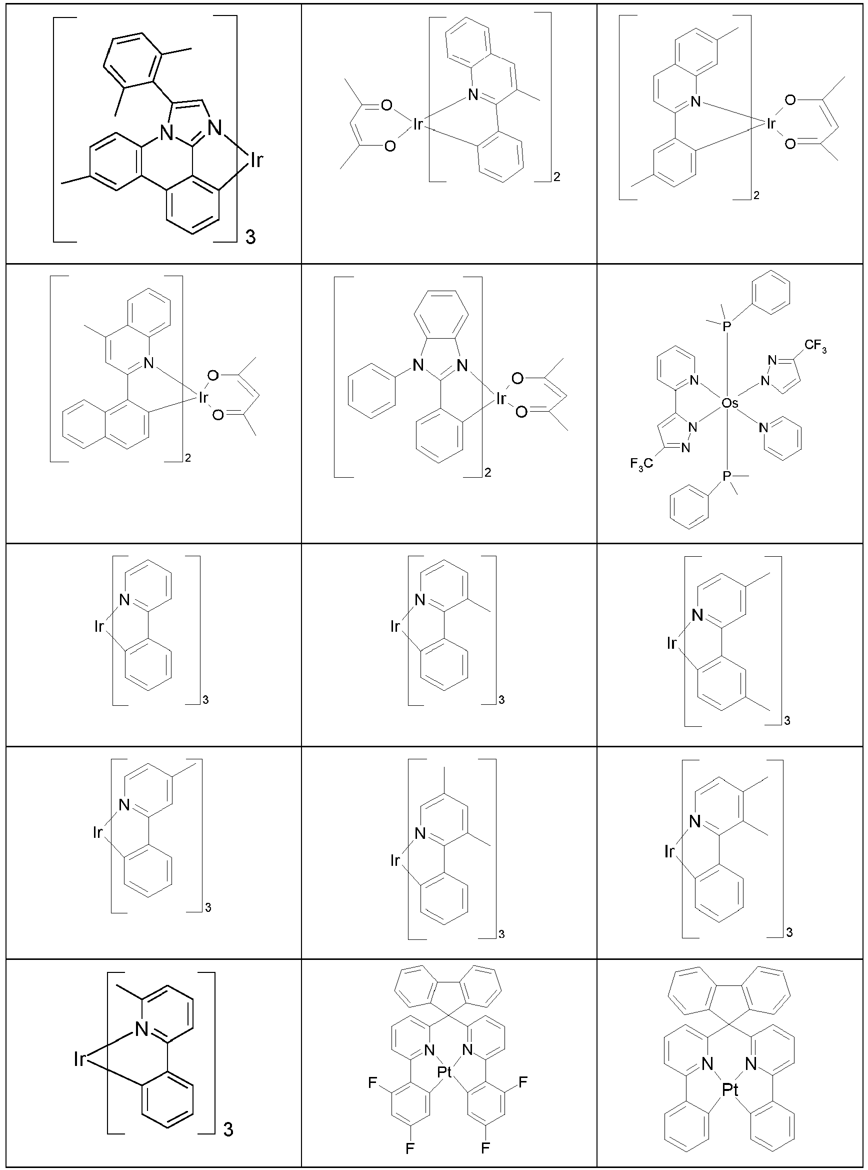

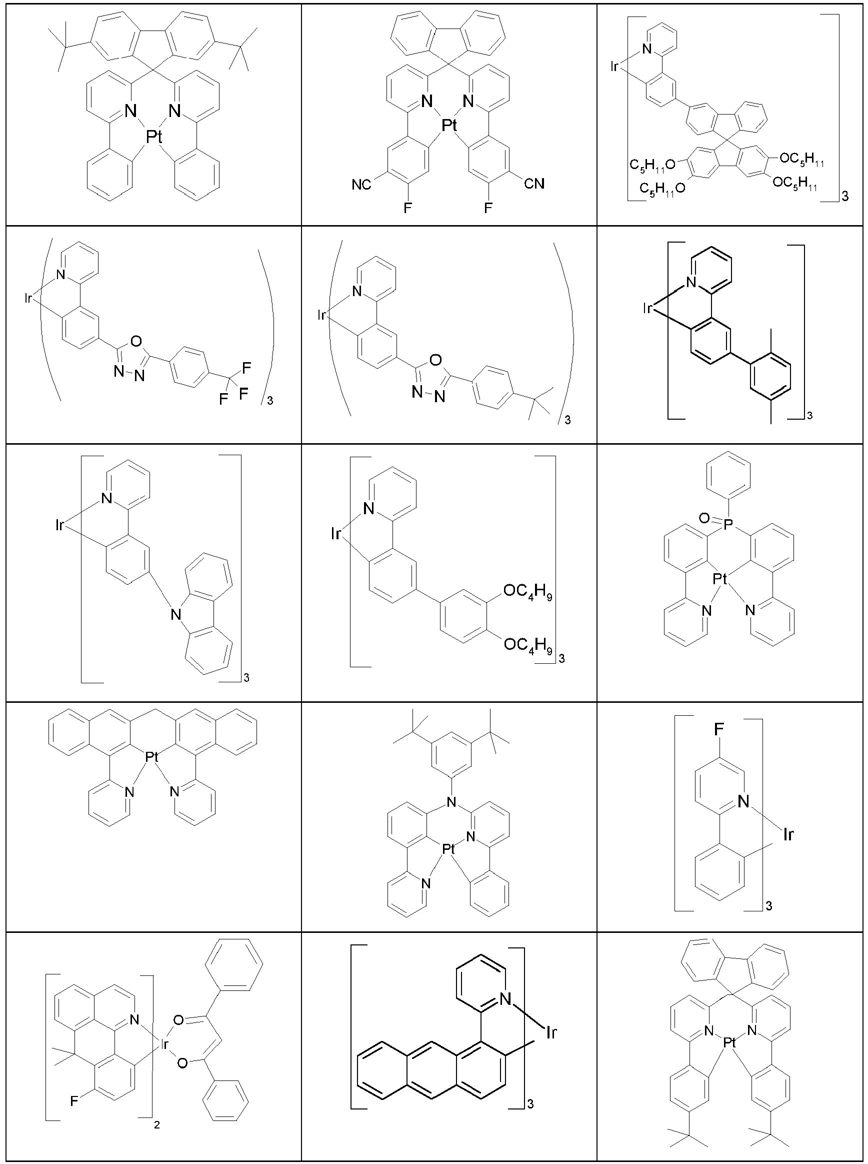

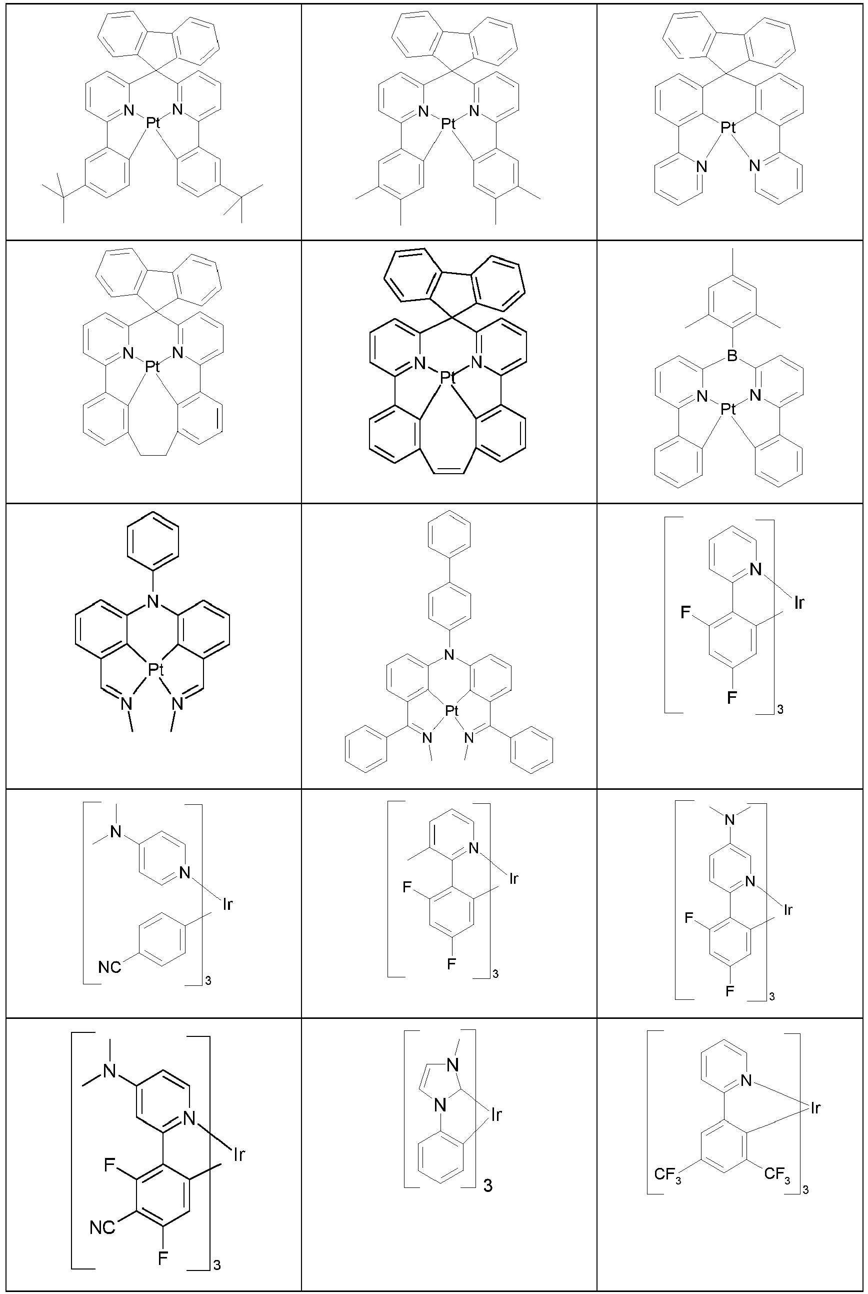

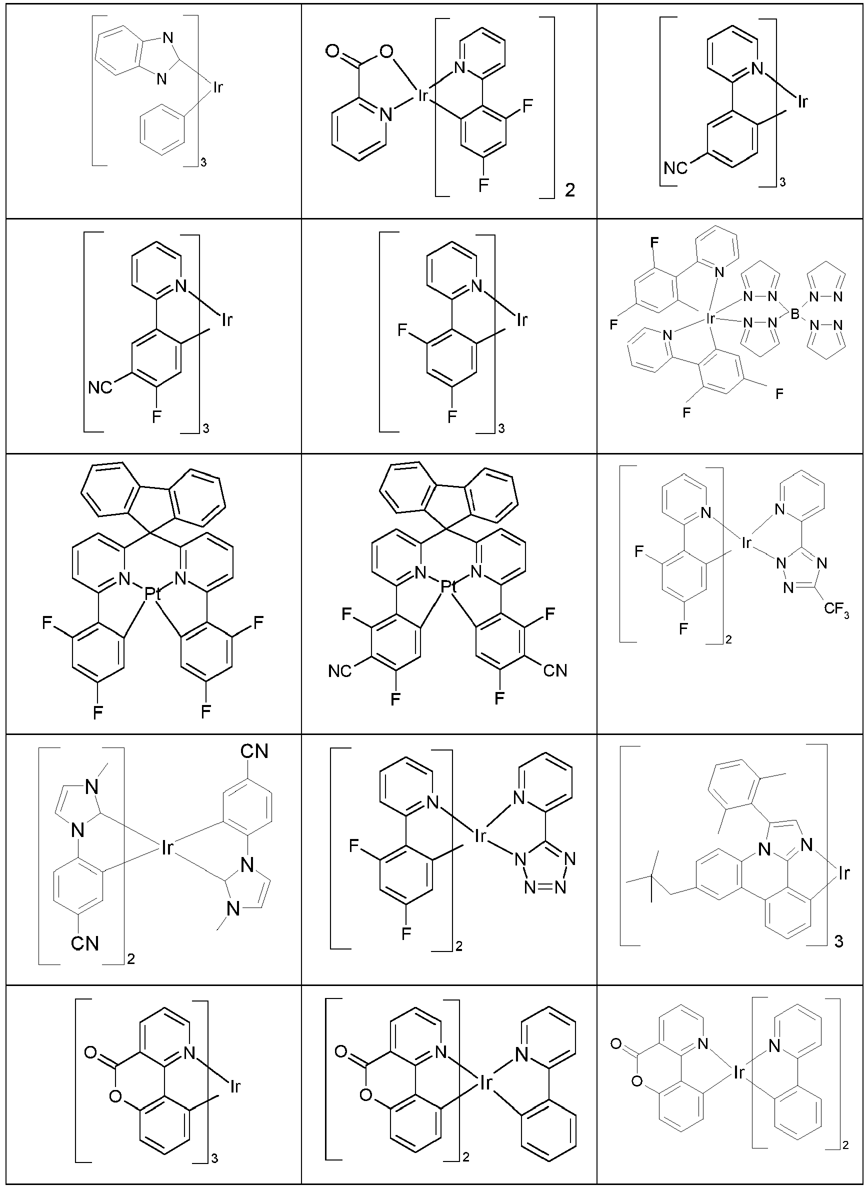

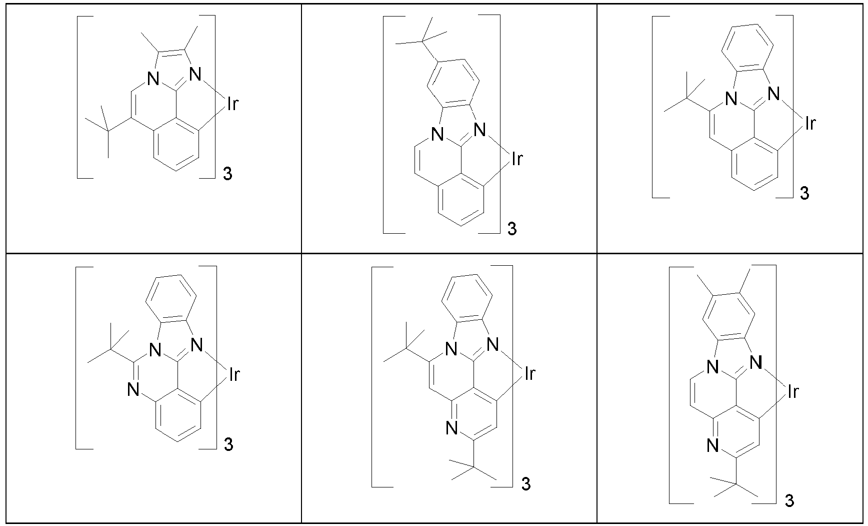

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (3) bzw. gemäß den bevorzugten Ausführungsformen verwenden.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer oder höher ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Ink-Jet Druck (Tintenstrahldruck), LITI (Light Induced Thermal Imaging, Thermotransferdruck), Siebdruck, Flexodruck, Offsetdruck oder Nozzle-Printing hergestellt werden. Hierfür sind lösliche Verbindungen nötig, welche beispielsweise durch geeignete Substitution erhalten werden. Diese Verfahren eignen sich insbesondere auch für Oligomere, Dendrimere und Polymere.

Weiterhin sind Hybridverfahren möglich, bei denen beispielsweise eine oder mehrere Schichten aus Lösung aufgebracht werden und eine oder mehrere weitere Schichten aufgedampft werden. So ist es beispielsweise möglich, die emittierende Schicht aus Lösung aufzubringen und die Elektronentransportschicht aufzudampfen.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen weisen bei Verwendung in organischen Elektrolumineszenzvorrichtungen einen oder mehrere der folgenden überraschenden Vorteile gegenüber dem Stand der Technik auf:
1. Die Leistungseffizienz entsprechender Vorrichtungen wird höher im Vergleich zu Systemen gemäß dem Stand der Technik.
2. Die Stabilität entsprechender Vorrichtungen wird höher im Vergleich zu Systemen gemäß dem Stand der Technik, was sich vor allem in einer deutlich höheren Lebensdauer zeigt.
3. Die erfindungsgemäßen organische Elektroluminszenzvorrichtungen weisen eine verringerte Betriebsspannung auf.
4. Wenn die erfindungsgemäßen Verbindungen als Matrixmaterial für phosphoreszierende Emitter verwendet werden, ist es möglich, mit nur einer geringen Emitterkonzentration im Bereich von weniger als 10 Vol.% bereits sehr gute Ergebnisse zu erzielen.
5. Die erfindungsgemäßen Verbindungen weisen eine sehr gute thermische Stabilität auf.

Die Erfindung wird nun durch die nachfolgenden Beispiele näher erläutert, ohne sie dadurch einschränken zu wollen.

### Ausführungsbeispiele

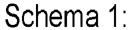

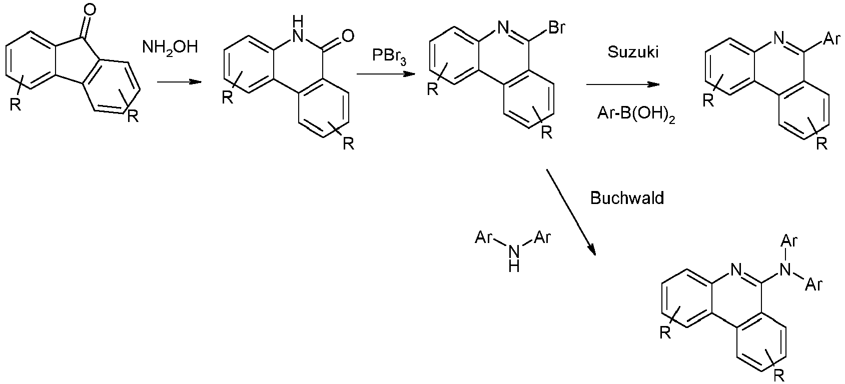

Schema 1 zeigt einen möglichen Syntheseweg. Dabei steht R für einen beliebigen Rest und Ar für ein heteroaromatisches Ringsystem der Formel (Ar²)_{q}N(Ar¹)₂ oder Ar¹ im Falle der Buchwald-Kupplung. Statt Br kann auch eine andere zur Kupplung geeignete Gruppe vorhanden sein, wie ein Chlor oder Iod oder eine Sulfonsäuregruppe. Ausgehend von Fluorenon kann über eine Beckmann-Umlagerung 5H-Phenanthridin-6-on erhalten werden. Daraus kann mit PBr₃ 6-Bromphenanthridin erhalten werden. Diese Verbindung kann über metallorganische Kupplungen wie Suzuki- oder Buchwald-Kupplungen zu den erfindungsgemäßen Verbindungen umgesetzt werden.

Die nachfolgenden Synthesen werden, sofern nicht anders angegeben, unter einer Schutzgasatmosphäre durchgeführt. Die Edukte können von den Firmen ALDRICH bzw. ABCR (Palladium(II)acetat, Tri-o-tolylphosphin, Anorganika, Lösemittel) bezogen werden.

### Beispiel 1: 3-Bromo-9-[1,1';3',1"]terphenyl-5'-yl-9H-carbazol

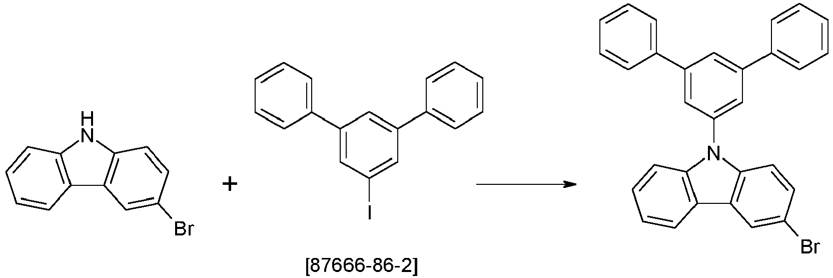

10 g (41 mmol) 3-Bromo-9H-Carbazol (CAS 86-74-8) und 16 g (45 mmol, 1,1 eq) 5'-Iodo-[1,1';3',1"]terphenyl werden zusammen mit 9,2 g (160 mmol, 4 eq) Kaliumhydroxid, 300 mg (1,6 mmol, 0,04 eq) 1,10-Phenanthrolin und 160 mg (1,6 mmol, 0,04 eq) Kupfer(I)-iodid in 250 ml p-Xylol gelöst und unter Rückfluss erhitzt. Nach beendeter Reaktion wird das Gemisch dreimal mit Wasser extrahiert und die organische Phase über Natriumsulfat getrocknet, das Lösungsmittel im Vakuum entfernt und der erhaltene Feststoff mittels Säulenchromatogrpahie (Ethylacetat/Heptan) aufgereinigt. Es werden 17 g (36 mmol, 88%) des gewünschten Produkts erhalten.

Analog können folgende Verbindungen erhalten werden:

| **Bsp.** | **Edukt 1** | **Edukt 2** | **Produkt** | **Ausbeute [%1** |
|---|---|---|---|---|
| 1a | 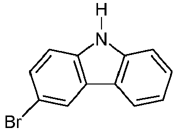 | 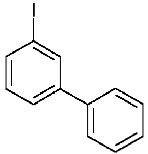 | 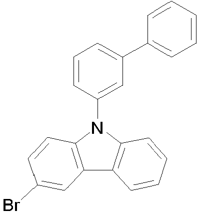 | 94 |
| | CAS 86-74-8 | CAS 20442-79-9 | | |
| 1b | 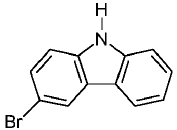 | 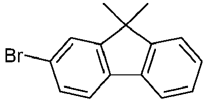 | 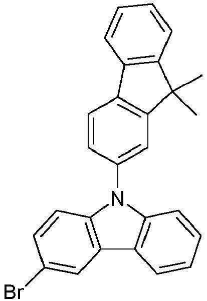 | 91 |
| | CAS 86-74-8 | CAS 28320-31-2 | | |

### Beispiel 2: 9-(9,9-Dimethyl-9H-fluoren-2-yl)-9H-carbazol-3-Boronsäure

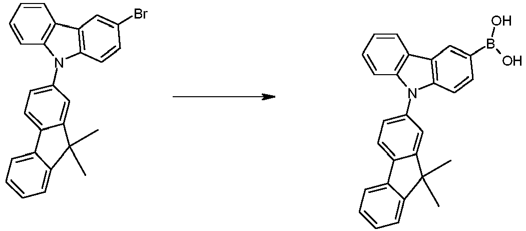

22,3 g (51 mmol) 3-Bromo-9-(9,9-dimethyl-9H-fluoren-2-yl)-9H-carbazol werden in 600 mL trockenem THF gelöst und auf -78°C gekühlt. Bei dieser Temperatur wird mit 26,2 mL (65,7 mmol/2,5 M in Hexan) n-BuLi innerhalb von ca. 5 min. versetzt und anschließend für 2,5h bei -78°C nachgerührt. Bei dieser Temperatur wird mit 7,3 mL (65,7 mmol) Borsäuretrimethylester möglichst zügig versetzt und die Reaktion langsam auf RT kommen gelassen (ca. 18 h). Die Reaktionslösung wird mit Wasser gewaschen und der ausgefallene Feststoff und die organische Phase werden mit Toluol azeotrop getrocknet. Das Rohprodukt wird aus Toluol/Methylenchlorid bei ca. 40 °C ausgerührt und abgesaugt. Man erhält 17,5 g (85%) des Produktes als weißen Feststoff.

Analog können folgende Verbindungen erhalten werden:

| **Bsp.** | **Edukt 1** | **Produkt** | **Ausbeute [%]** |
|---|---|---|---|
| 2a | 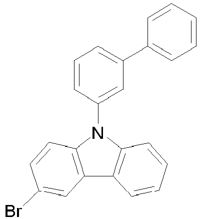 | 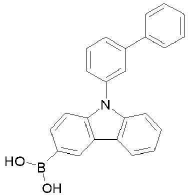 | 79 |

### Beispiel 3: 3-(5-Bromo-biphenyl-3-yl)-9-phenyl-9H-carbazol

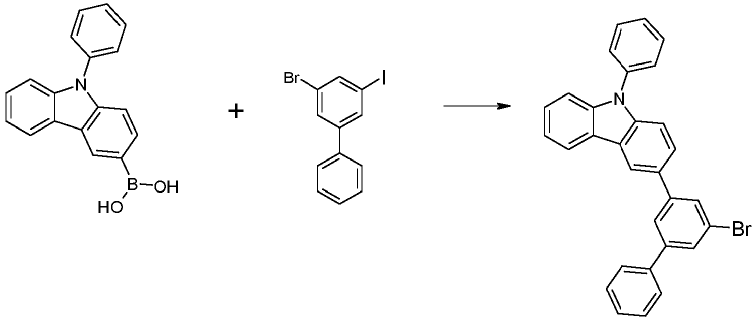

15,5 g (43,3 mmol) 3-Bromo-5-iodo-biphenyl und 13,7 g (48 mmol) (9-Phenyl-9H-carbazol-3-yl)-boronsäure werden in 80 mL Toluol gelöst und entgast. Es wird mit 281 mL einer entgasten 2 M K₂CO₃-Lösung und mit 2,5 g (2,2 mmol) Pd(OAc)₂ versetzt. Die Reaktionsmischung wird anschließend bei 80 °C für 48 h unter Schutzgasatmosphäre gerührt. Die abgekühlte Lösung wird mit Toluol verdünnt, mehrmals mit Wasser gewaschen, getrocknet und eingeengt. Das Produkt wird via Säulenchromatographie an Kieselgel mit Toluol/Heptan (1:2) gereinigt. Die Reinheit beträgt 98 %. Ausbeute: 17,6 g (37 mmol, 78 %) der Theorie.

Analog können folgende Verbindungen erhalten werden:

| **Bsp.** | **Edukt 1** | **Edukt 2** | **Produkt** | **Ausbeute [%]** |
|---|---|---|---|---|
| 3a | 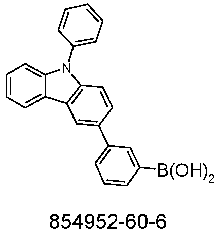 | 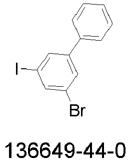 | 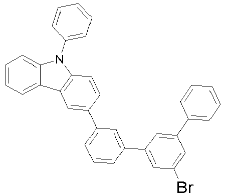 | 70 |
| 3b | 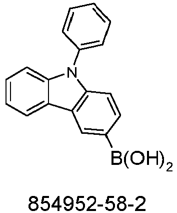 | 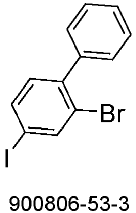 | 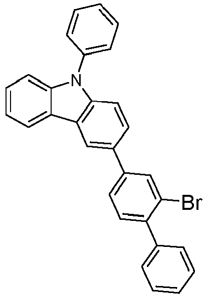 | 69 |
| 3c | 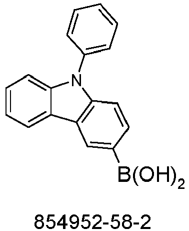 | 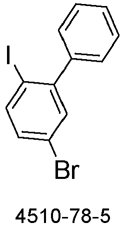 | 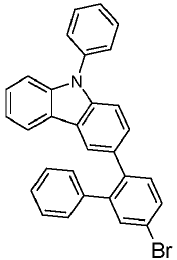 | 68 |
| 3d | 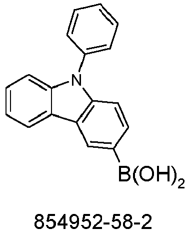 | 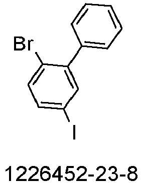 | 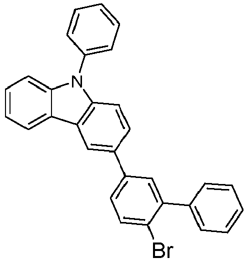 | 83 |

### Beispiel 4: 3-(5-Boronsäure-biphenyl-3-yl)-9-phenyl-9H-carbazol

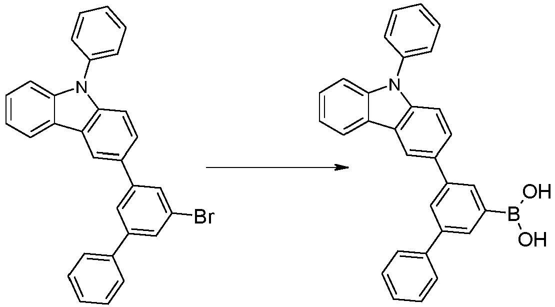

Eine auf -78 °C gekühlte Lösung von 128 g (270 mmol) 3-(5-Bromo-biphenyl-3-yl)-9-phenyl-9-H-carbazol in 1500 ml Diethylether wird tropfenweise mit 110 ml (276 mmol) n-Butyllithium (2,5 M in Hexan) versetzt. Die Reaktionsmischung wird 30 min. bei -78 °C gerührt. Man lässt auf Raumtemperatur kommen, kühlt erneut auf -78 °C und versetzt dann schnell mit einer Mischung von 40 ml (351 mmol) Trimethylborat in 50 ml Diethylether. Nach Erwärmen auf -10 °C wird mit 135 ml 2 N Salzsäure hydrolysiert. Die organische Phase wird abgetrennt, mit Wasser gewaschen, über Natriumsulfat getrocknet und zur Trockene eingeengt. Der Rückstand wird in 300 ml n-Heptan aufgenommen, der farblose Feststoff wird abgesaugt, mit n-Heptan gewaschen und im Vakuum getrocknet. Ausbeute: 112 g (256 mmol), 95 % d. Th.

Analog können folgende Verbindungen erhalten werden:

| **Bsp.** | **Edukt 1** | **Produkt** | **Ausbeute [%]** |
|---|---|---|---|
| 4a | 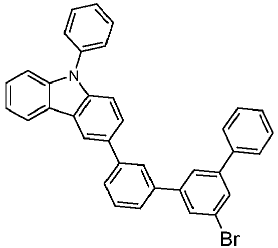 | 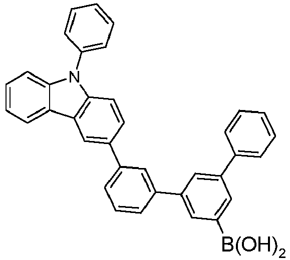 | 59 |
| 4b | 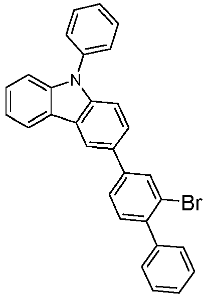 | 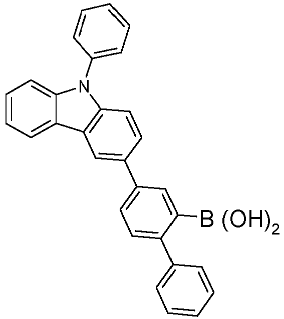 | 60 |
| 4e | 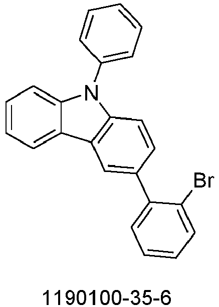 | 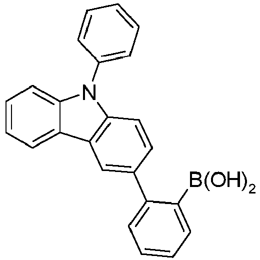 | 59 |
| 4f | 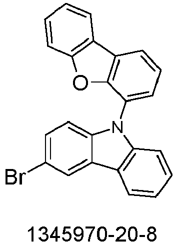 | 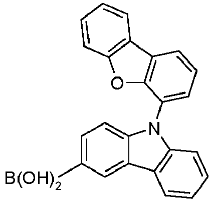 | 83 |

### Beispiel 5: 6-(9-Phenyl-9H-carbazol-3-yl)-phenanthridin (Referenzmaterial)

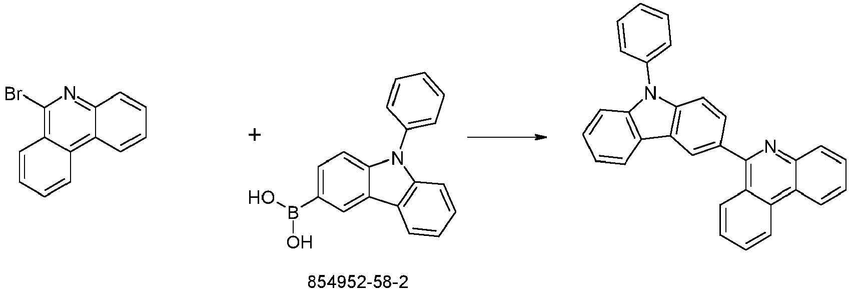

28,9 g (43,3 mmol) 6-Bromphenanthridin und 13,7 g (48 mmol) 9-Phenyl-9H-carbazol-3-boronsäure werden in 80 mL Toluol gelöst und entgast. Es wird mit 281 mL einer entgasten Lösung von 2 M K₂CO₃ und mit 2,5 g (2,2 mmol) Pd(OAc)₂ versetzt. Die Reaktionsmischung wird anschließend bei 80 °C für 48 h unter Schutzgasatmosphäre gerührt. Die abgekühlte Lösung wird mit Toluol aufgestockt, mehrmals mit Wasser gewaschen, getrocknet und eingeengt. Das Produkt wird via Säulenchromatographie an Kieselgel mit Toluol/Heptan (1:2) gereinigt und abschließend im Hochvakuum (p = 5 x 10⁻⁷ mbar) sublimiert. Die Reinheit beträgt 99,9%. Ausbeute: 28 g (31 mmol), 80 % der Theorie.

Analog konnten folgende Verbindungen erhalten werden (5a bis 5s sind Referenzmaterialien):

| **Bsp.** | **Edukt 1** | **Edukt 2** | **Produkt** | **Ausbeute [%]** |
|---|---|---|---|---|
| 5a | 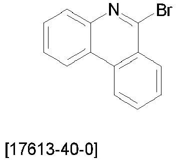 | 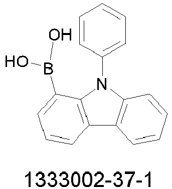 | 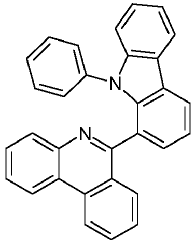 | 59 |
| 5b | 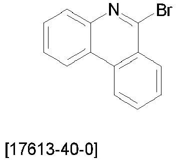 | 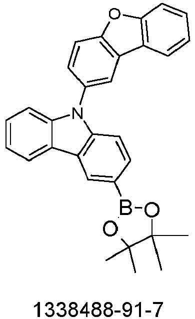 | 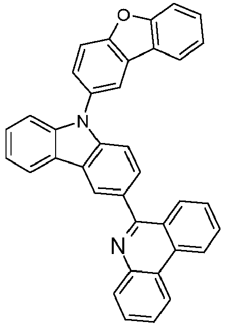 | 60 |
| 5c | 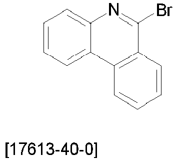 | 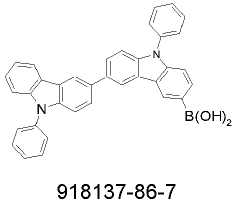 | 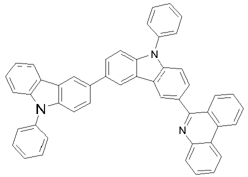 | 67 |
| 5d | 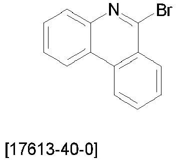 | 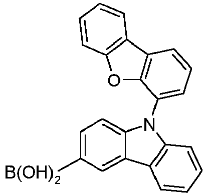 | 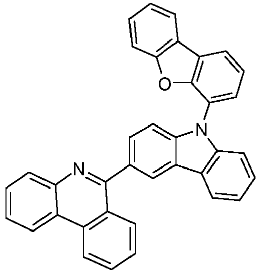 | 69 |
| 5e | 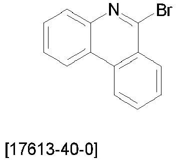 | 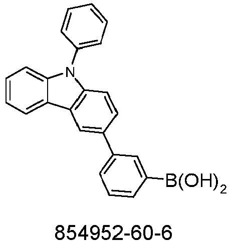 | 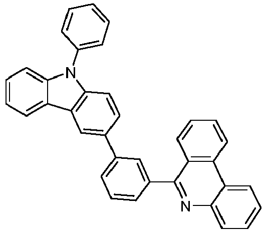 | 71 |
| 5f | 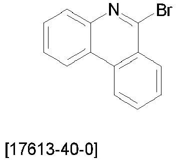 | 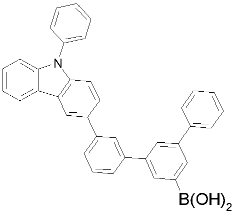 | 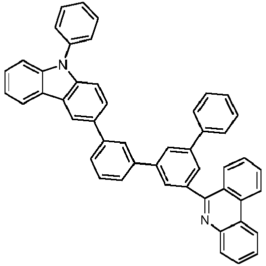 | 73 |
| 5g | 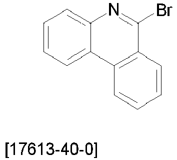 | 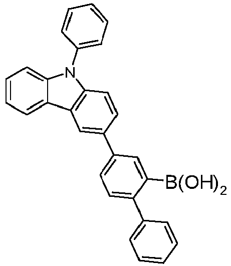 | 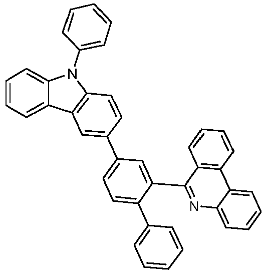 | 66 |
| 5h | 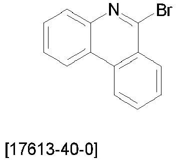 | 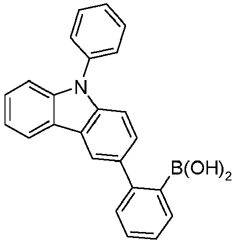 | 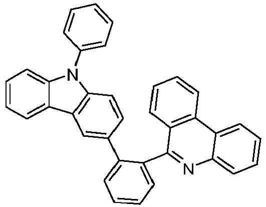 | 75 |
| 5i | 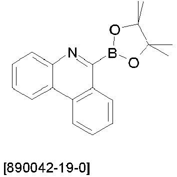 | 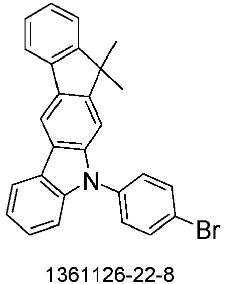 | 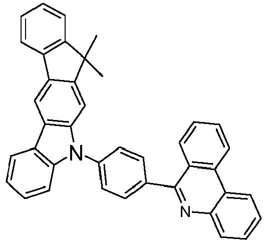 | 78 |
| 5j | 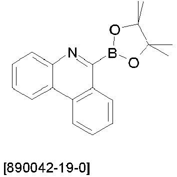 | 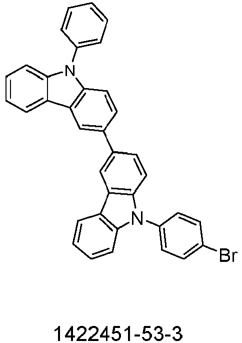 | 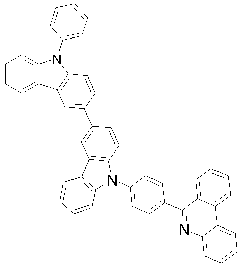 | 81 |
| 5k | 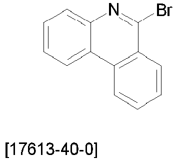 | 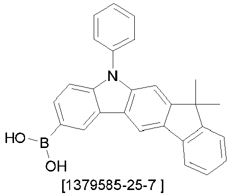 | 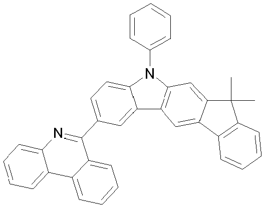 | 82 |
| 5I | 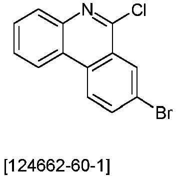 | 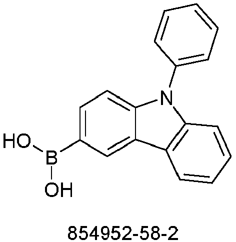 | 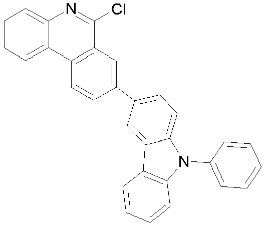 | 84 |
| 5m | 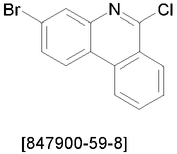 | 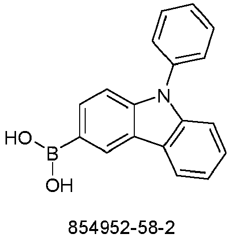 | 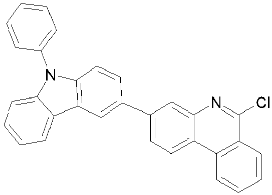 | 80 |
| 5n | 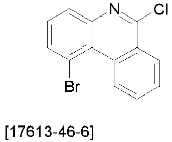 | 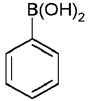 | 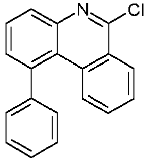 | 84 |
| 5o | 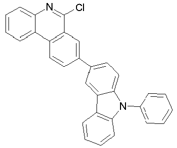 | 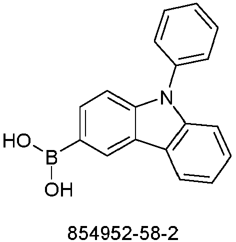 | 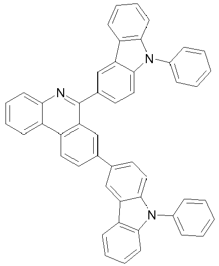 | 79 |
| 5p | 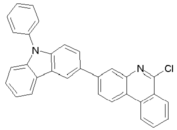 | 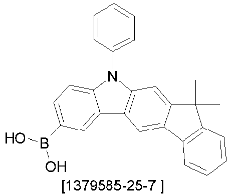 | 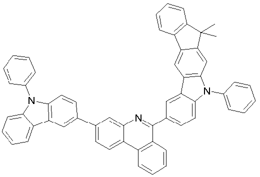 | 78 |
| 5q | 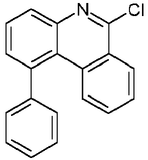 | 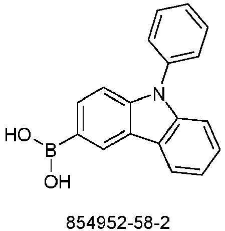 | 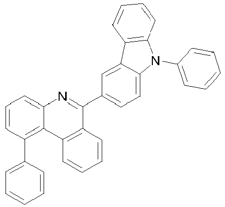 | 77 |
| 5r | 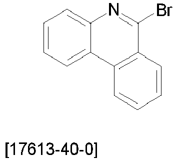 | 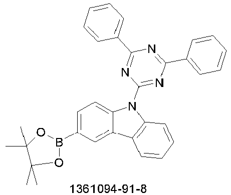 | 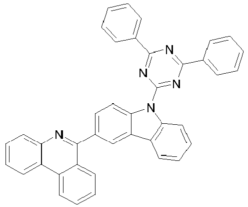 | 89 |
| 5r1 | 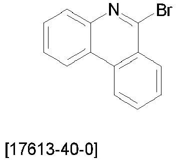 | 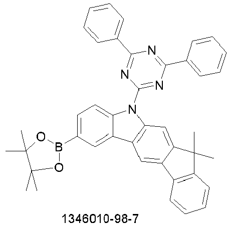 | 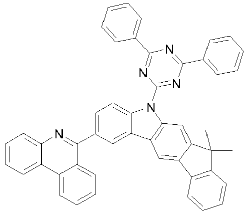 | 82 |

Analog können die folgenden Verbindungen mit 0,5 eq.
Phenathridin erhalten werden.

| **Bsp.** | **Edukt 1** | **Edukt 2** | **Produkt** | **Ausbeute [%]** |
|---|---|---|---|---|
| 5s | 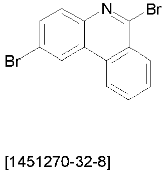 | 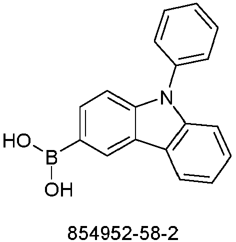 | 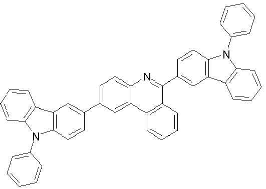 | 69 |

### Beispiel 6 : (9,9-Dimethyl-9H-fluoren-2-yl)-{4-[(Z)-1-eth-(E)-ylidene-penta-2,4-dienyl]-phenyl}-amin

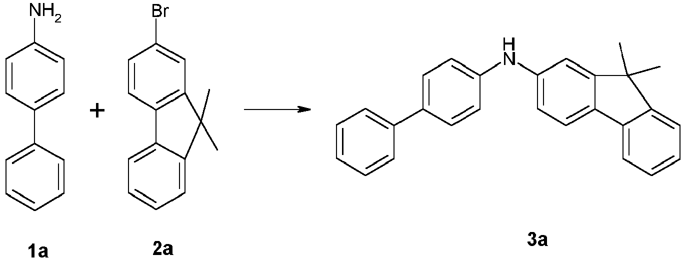

24,0 g (142 mmol, 1,2 eq.) 4-Aminobiphenyl **1a** (CAS 92-67-1) werden zusammen mit 32,0 g (117 mmol, 1.0 eq) 2-Bromo-9,9'-dimethylfluoren **2a** (CAS 28320-31-2) in 950 ml Toluol vorgelegt und 30 Minuten mit Argon gesättigt. Anschließend werden 1,0 g (1,8 mmol, 0,02 eq) 1,1'-Bis(diphenylphosphino)ferrocen (CAS 12150-46-8), 350 mg (1,6 mmol, 0,01 eq) Palladium(II)-acetat (CAS 3375-31-3) und 29 g (300 mmol, 2,6 eq) Natrium-*tert*-butylat (CAS 865-48-5) zugegeben und über Nacht unter Rückfluss erhitzt. Nach beendeter Reaktion wird der Ansatz mit 300 ml Toluol verdünnt und mit Wasser extrahiert. Die organische Phase wird über Natriumsulfat getrocknet und das Lösungsmittel am Rotationsverdampfer entfernt. Das braune Öl wird mit 50 ml Ethylacetat versetzt und in eine Mischung aus Heptan/Essigester 20:1 gegeben. Der entstandene Feststoff wird abgesaugt und mit Heptan gewaschen. Nach Trocknung werden 29 g (80 mmol, 69%) des gewünschten Produkts **3a** mit einer HPLC-Reinheit von 99,1% erhalten.

Analog können folgende Verbindungen erhalten werden:

| **Bsp.** | **Edukt 1** | **Edukt 2** | **Produkt** | **Ausbeute [%]** |
|---|---|---|---|---|
| 6a | 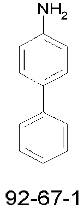 | 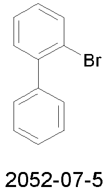 | 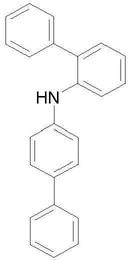 | 71 |
| 6b | 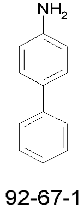 | 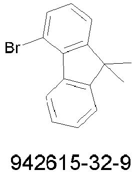 | 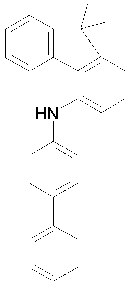 | 61 |
| 6c | 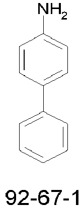 | 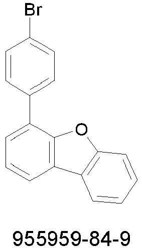 | 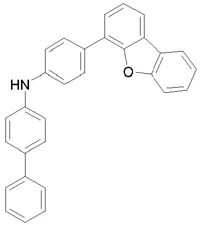 | 78 |
| 6d | 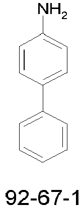 | 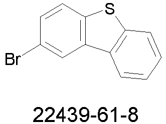 | 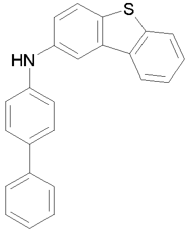 | 82 |
| 6e | 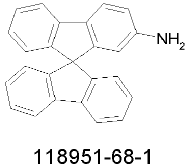 | 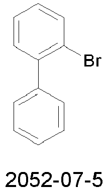 | 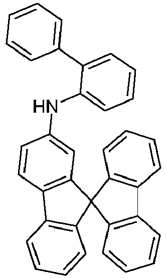 | 62 |
| 6f | 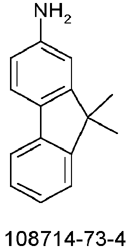 | 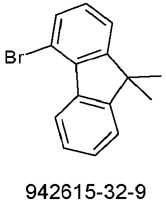 | 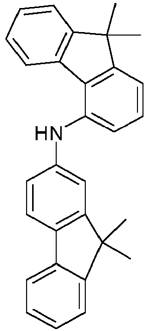 | 47 |
| 6g | 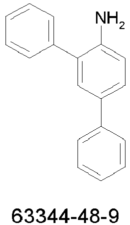 | 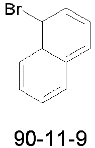 | 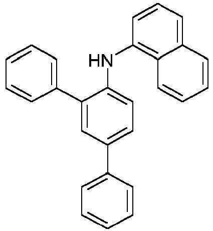 | 92 |
| 6h | 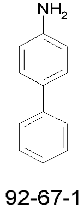 | 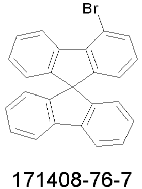 | 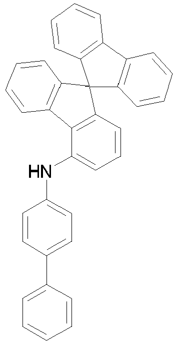 | 75 |
| 6i | 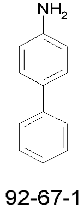 | 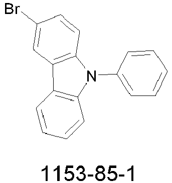 | 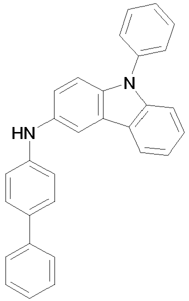 | 84 |
| 6j | 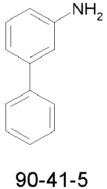 | 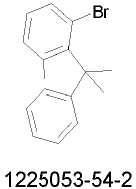 | 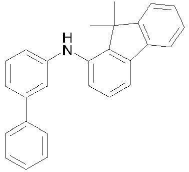 | 62 |
| 6k | 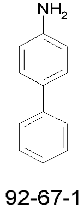 | 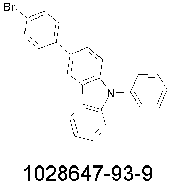 | 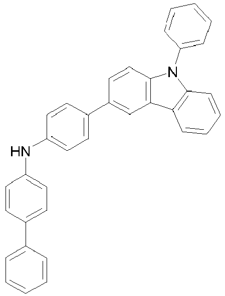 | 78 |
| 6I | 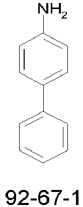 | 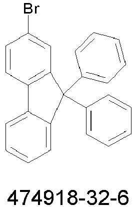 | 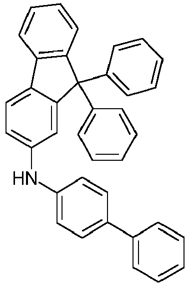 | 74 |
| 6m | 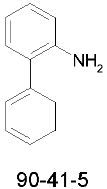 | 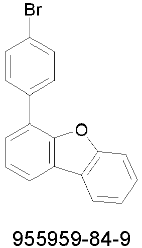 | 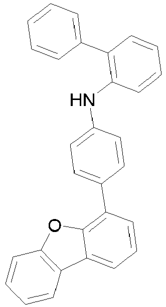 | 62 |
| 6n | 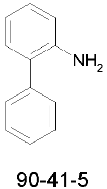 | 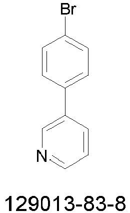 | 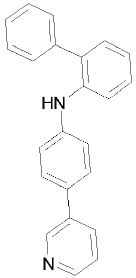 | 67 |
| 6o | 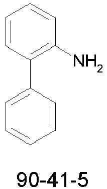 | 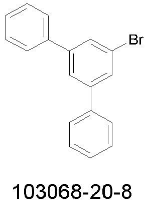 | 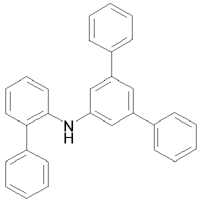 | 93 |
| 6p | 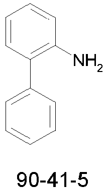 | 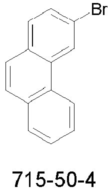 | 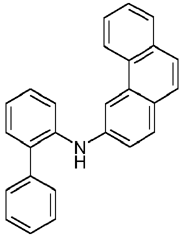 | 88 |
| 6q | 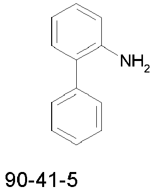 | 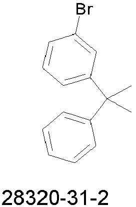 | 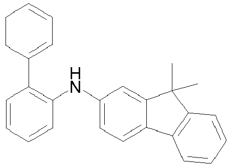 | 74 |

### Beispiel 7: Biphenyl-4-yl-(3'-bromo-biphenyl-3-yl)-(9,9-dimethyl-9H-fluoren-2-yl)-amin

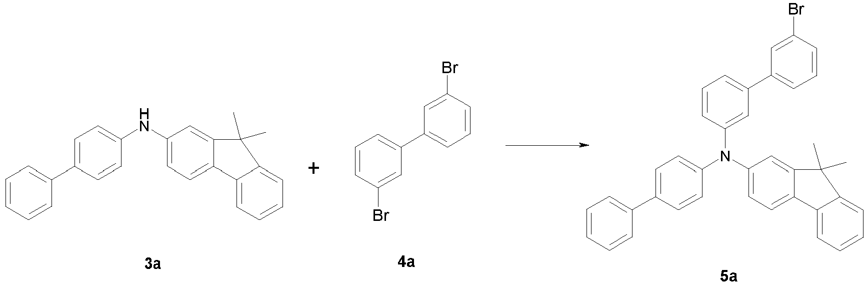

Es werden 29 g (80 mmol, 1,0 eq) des Zwischenprodukts **3a** zusammen mit 25 g (80 mmol, 1,0 eq) 3,3'-Dibromo-1,1'-biphenyl **4a** (CAS 16400-51-4) in 600 ml Toluol gelöst und für 30 Minuten entgast. Anschließend werden 45 g (240 mmol, 3,0 eq) Natrium-*tert*-butylat, 890 mg (0,40 mmol, 0,050 eq) Palladium(II)-acetat und 8 ml (8,0 mmol, 0,10 eq.) einer 1M tri*tert*-Butylphosphin-Lösung zugegeben. Der Ansatz wird über Nacht unter Rückfluss erhitzt und nach beendeter Reaktion zweimal über Aluminiumoxid mit Toluol filtriert. Nach Entfernung des Lösungsmittels am Rotationsverdampfer wird das Öl in etwas THF gelöst und in Heptan eingetragen. Der entstandene Feststoff wird abgesaugt und mittels Heißextraktion in Heptan/Toluol 1:1 aufgereinigt. Es werden 16,6 g (28 mmol, 35%) des gewünschten Produkts **5a** erhalten.

Analog können folgende Verbindungen erhalten werden:

| **Bsp.** | **Edukt 3** | **Edukt 4** | **Produkt** | **Ausbeute [%]** |
|---|---|---|---|---|
| 7a | 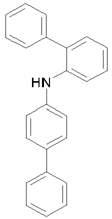 | 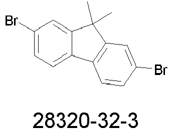 | 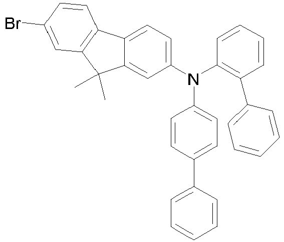 | 49 |
| 7b | 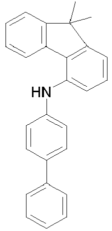 | 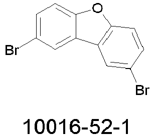 | 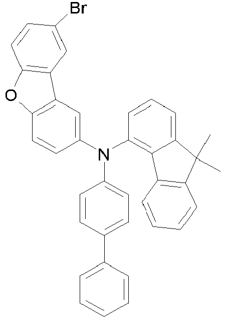 | 37 |
| 7c | 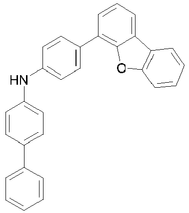 | 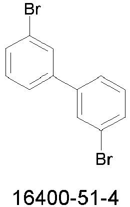 | 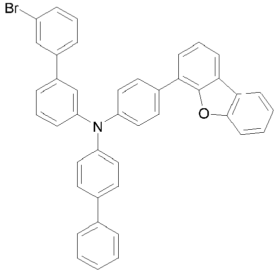 | 72 |
| 7d | 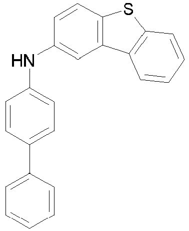 | 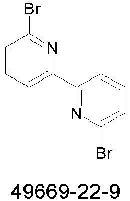 | 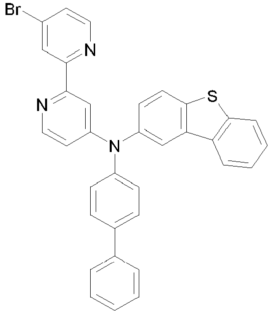 | 28 |
| 7e | 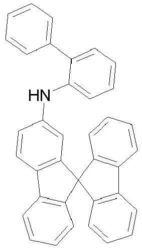 | 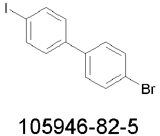 | 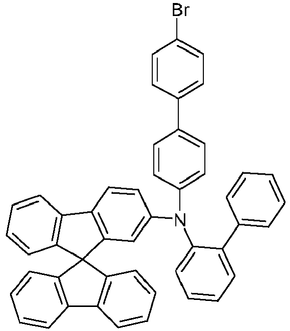 | 82 |
| 7f | 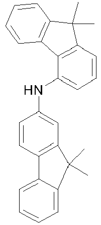 | 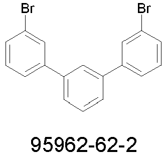 | 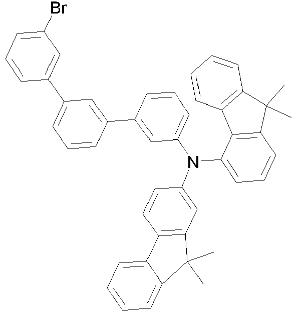 | 32 |
| 7g | 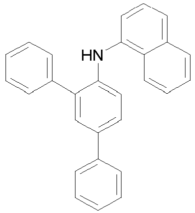 | 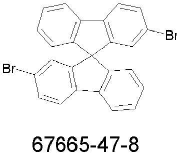 | 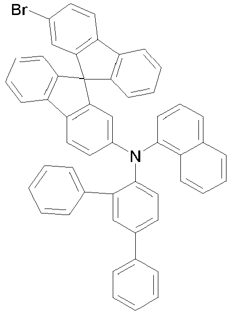 | 46 |
| 7h | 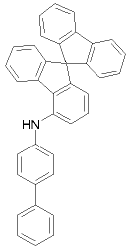 | 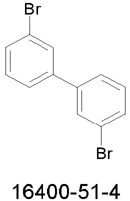 | 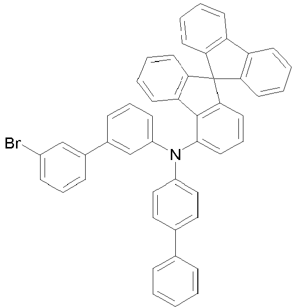 | 41 |
| 7i | 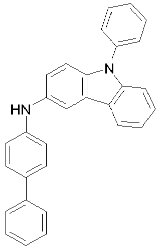 | 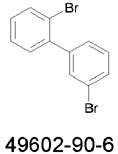 | 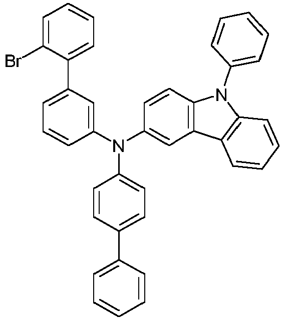 | 31 |
| 7j | 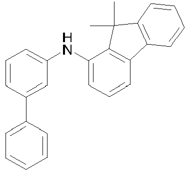 | 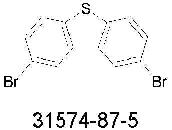 | 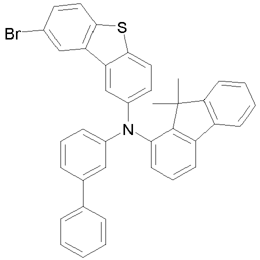 | 27 |
| 7k | 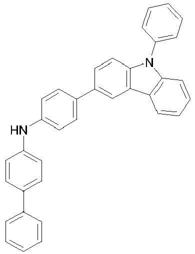 | 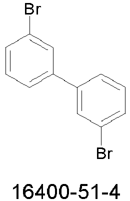 | 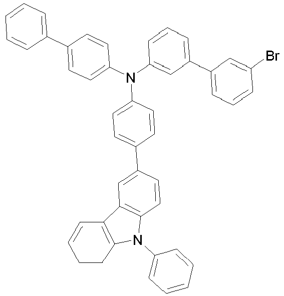 | 38 |
| 7l | 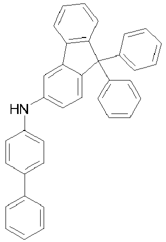 | 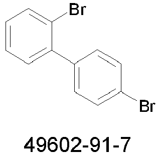 | 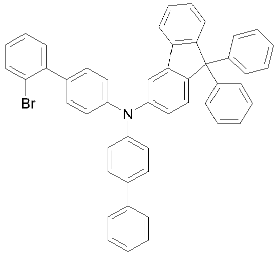 | 56 |
| 7m | 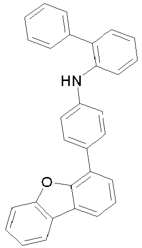 | 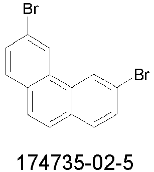 | 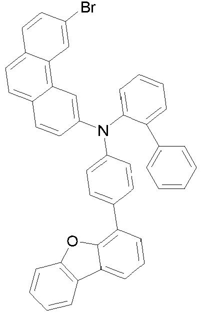 | 33 |
| 7n | 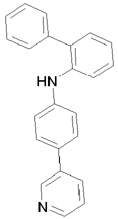 | 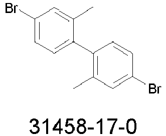 | 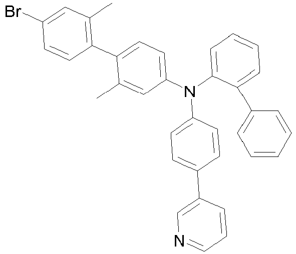 | 47 |
| 7o | 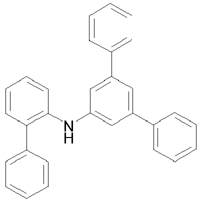 | 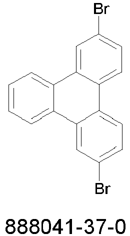 | 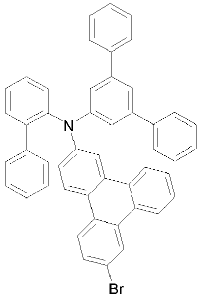 | 24 |
| 7p | 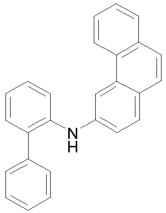 | 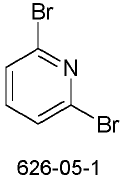 | 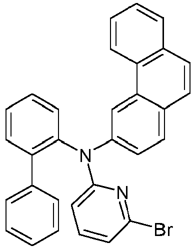 | 81 |
| 7q | 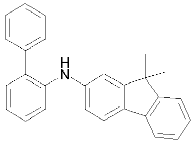 | 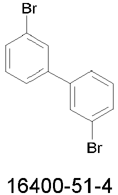 | 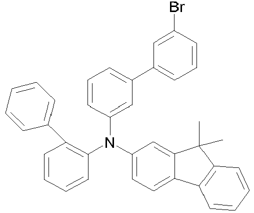 | 57 |
| 7r | 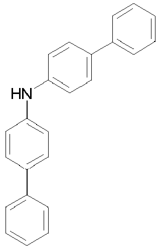 | 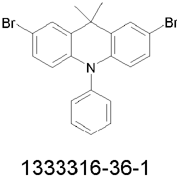 | 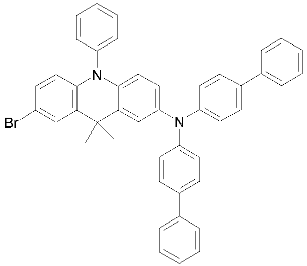 | 29 |
| 7s | 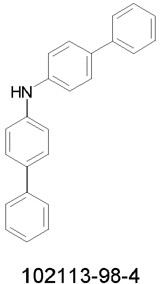 | 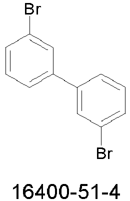 | 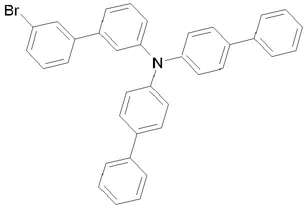 | 36 |

### Beispiel 8: Biphenyl-4-yl-(9,9-dimethyl-9H-fluoren-2-yl)-[3'-(4,4,5,5-tetramethyl-[1,3,2]dioxaborolan-2-yl)-biphenyl-3-yl]-amin

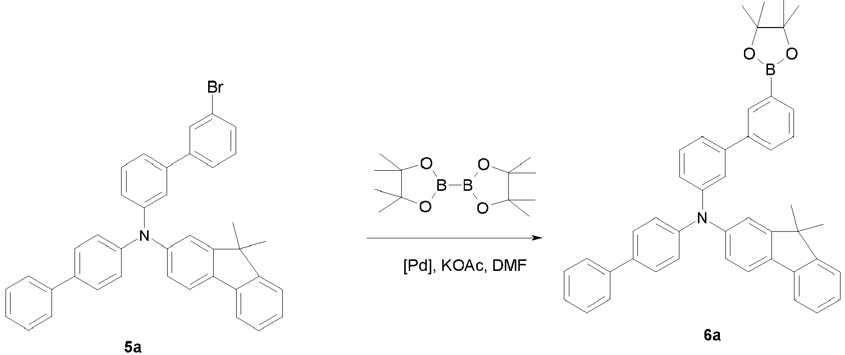

In einem 500 ml-Kolben werden unter Schutzgas 16,6 g (28 mmol, 35%) des Bromids **5a** zusammen mit 8,5 g (34 mmol, 1,2 eq) Bis-(pinacolato)-diboran (CAS 73183-34-3) in 120 ml trockenem DMF gelöst und für 30 Minuten entgast. Anschließend werden 8,2 g (84 mmol, 3,0 eq) Kaliumacetat und 690 mg (0,84 mmol, 3 mol-%) [1,1'-Bis(diphenylphosphino)ferrocen]-dichlorpalladium(II)-Komplex mit Dichlormethan (CAS 95464-05-4) zugegeben und der Ansatz über Nacht auf 90 °C erhitzt. Nach beendeter Reaktion wird mit 300 ml Toluol verdünnt und das Gemisch mit Wasser extrahiert. Das Lösungsmittel wird am Rotationsverdampfer entfernt und der erhaltene Feststoff 14,7 g (23 mmol, 82%) getrocknet. Der Boronester **6a** wird ohne weitere Aufreinigung umgesetzt.

Analog können folgende Verbindungen erhalten werden:

| **Bsp.** | **Edukt 5** | **Produkt 6** | **Ausbeute [%]** |
|---|---|---|---|
| 8a | 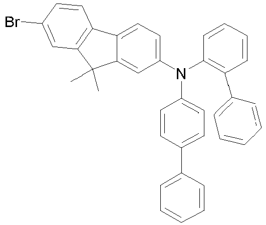 | 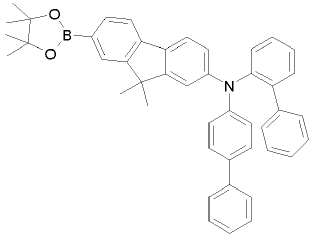 | 88 |
| 8b | 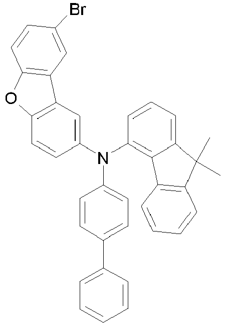 | 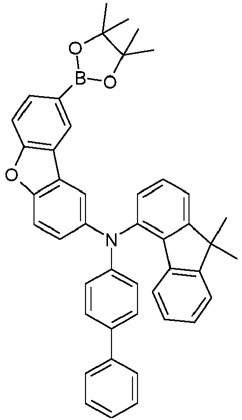 | 81 |
| 8c | 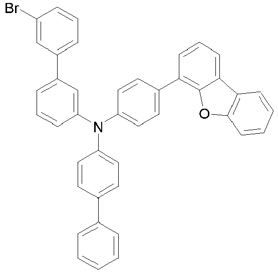 | 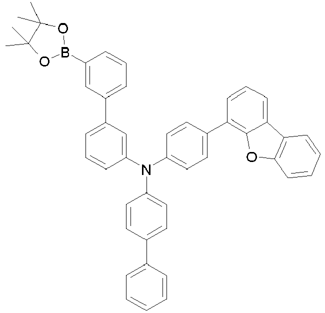 | 75 |
| 8d | 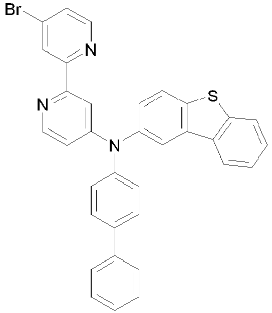 | 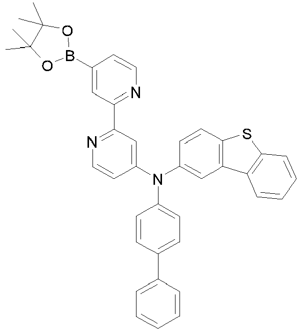 | 67 |
| 8e | 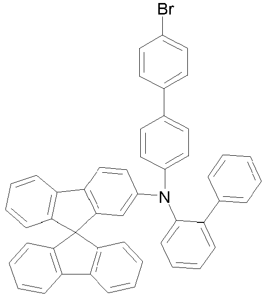 | 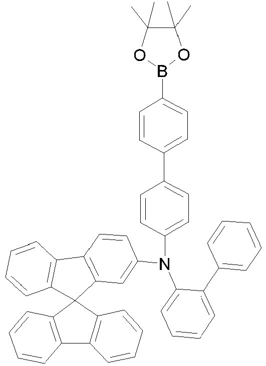 | 79 |
| 8f | 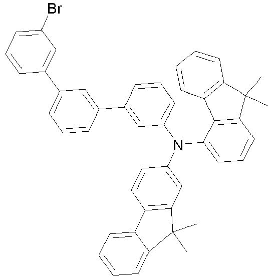 | 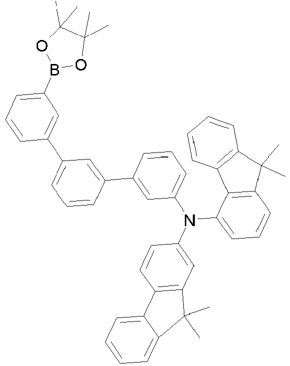 | 93 |
| 8g | 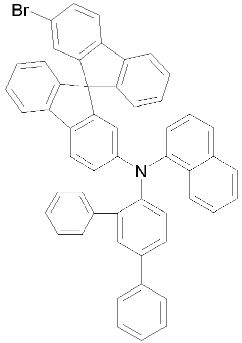 | 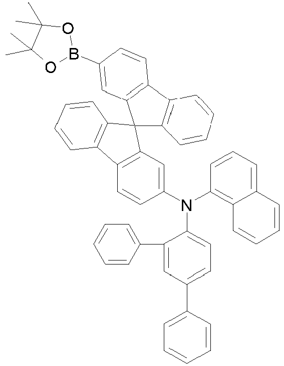 | 44 |
| 8h | 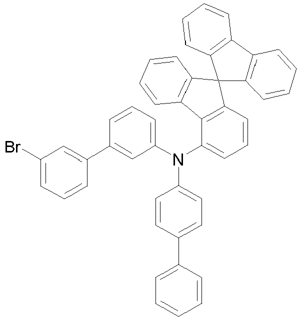 | 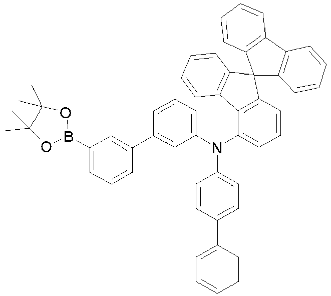 | 87 |
| 8i | 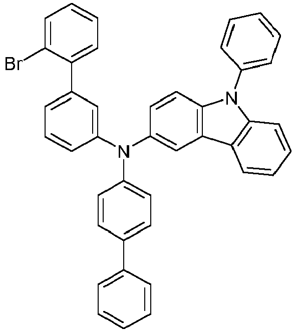 | 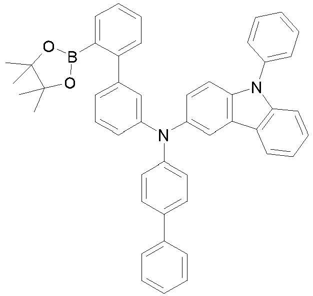 | 28 |
| 8j | 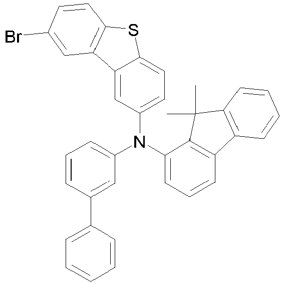 | 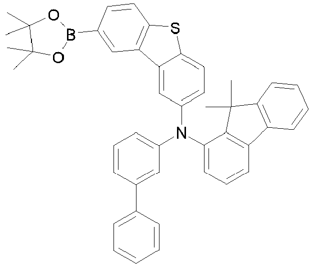 | 35 |
| 8k | 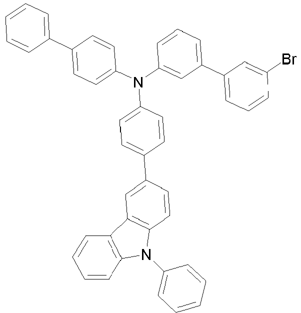 | 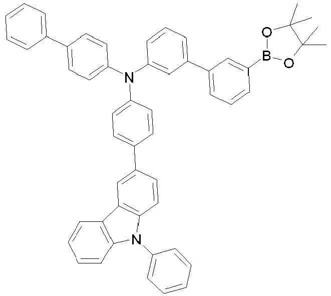 | 77 |
| 8I | 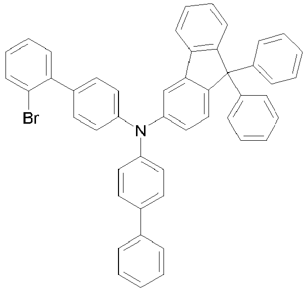 | 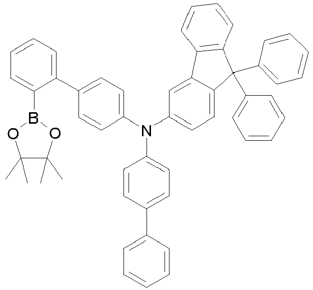 | 38 |
| 8m | 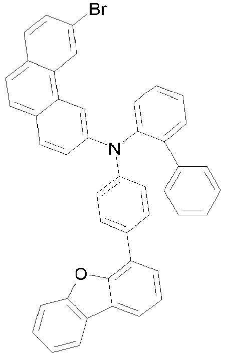 | 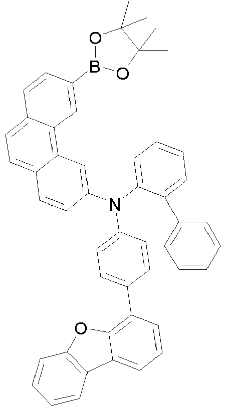 | 55 |
| 8n | 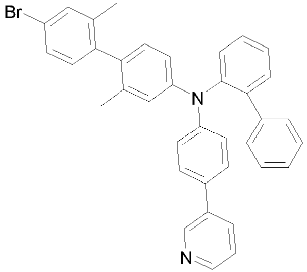 | 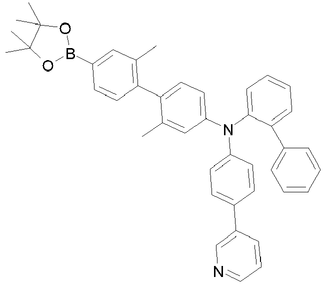 | 41 |
| 8o | 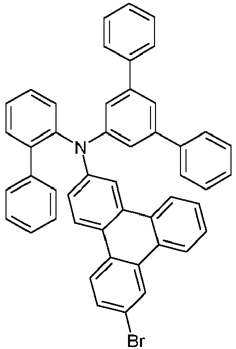 | 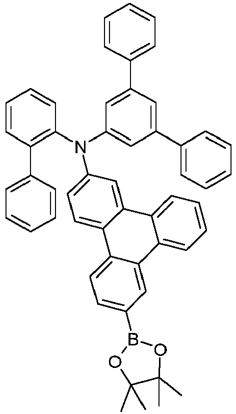 | 67 |
| 8p | 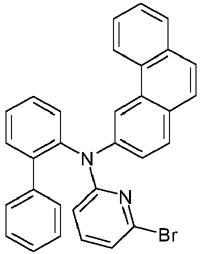 | 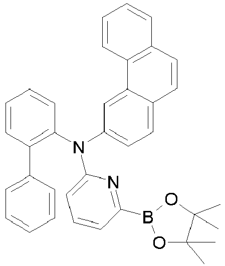 | 82 |
| 8q | 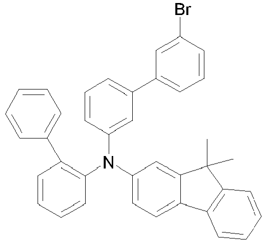 | 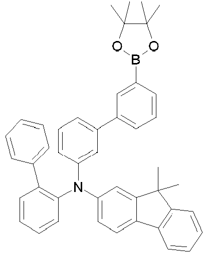 | 91 |
| 8r | 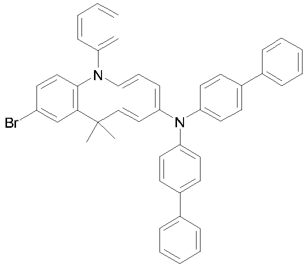 | 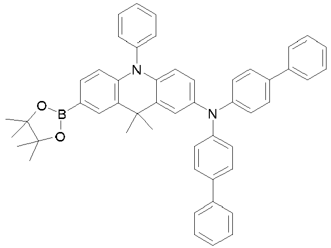 | 87 |
| 8s | 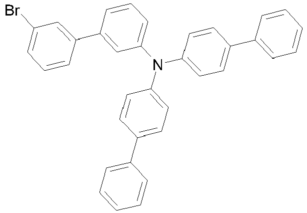 | 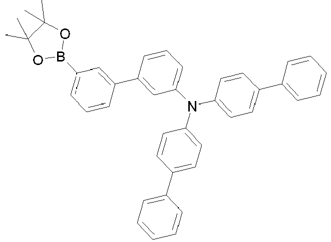 | 96 |

### Beispiel 9:

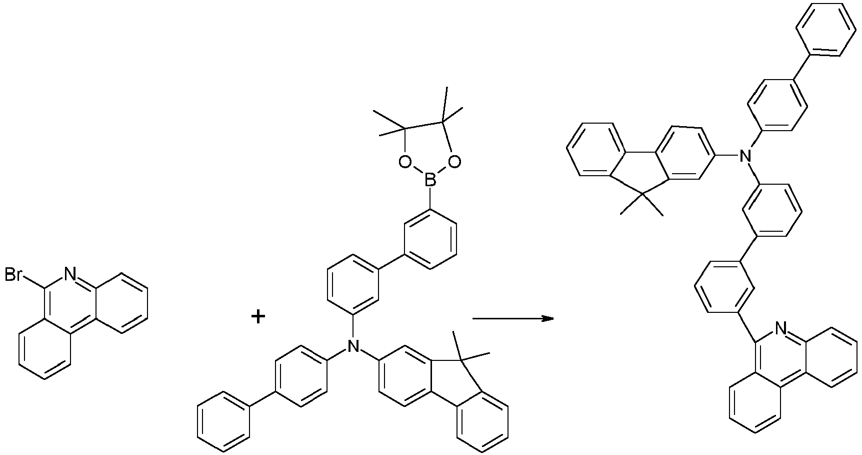

Analog **Beispiel 5** können folgende Moleküle hergestellt werden (* Referenzmaterialien):

| **Bsp.** | **Edukt 1** | **Edukt 2** | **Produkt** | **Ausbeute [%]** |
|---|---|---|---|---|
| 9a | 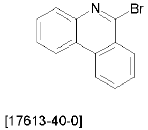 | 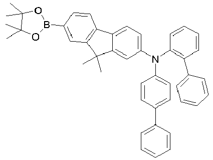 | 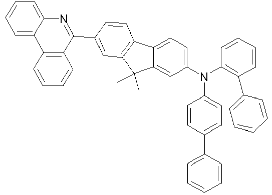 | 70 |
| 9b | 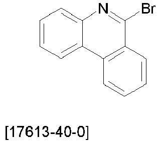 | 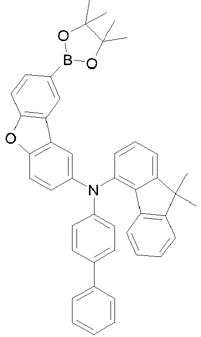 | 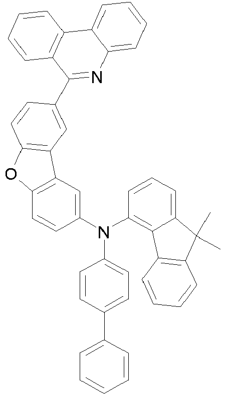 | 71 |
| 9c | 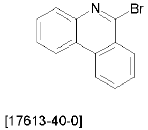 | 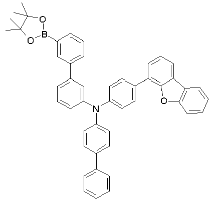 | 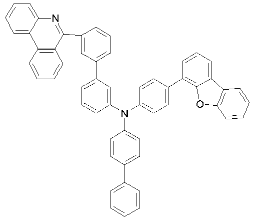 | 74 |
| 9d* | 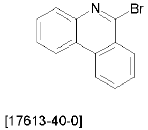 | 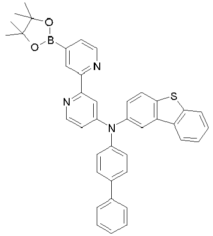 | 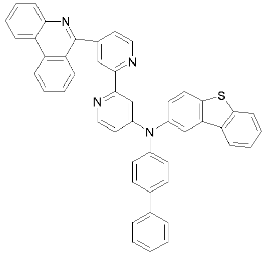 | 80 |
| 9e | 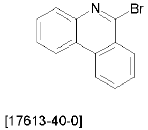 | 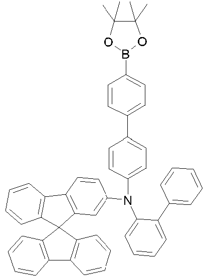 | 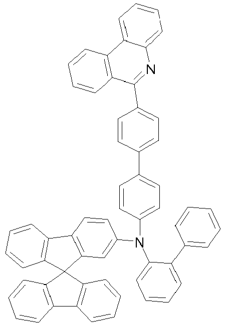 | 74 |
| 9f | 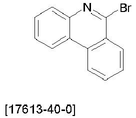 | 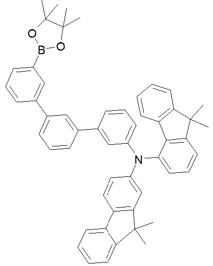 | 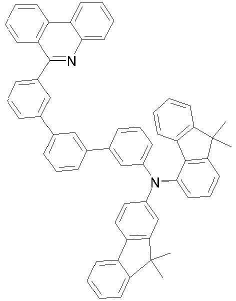 | 57 |
| 9g* | 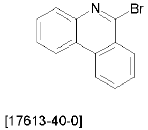 | 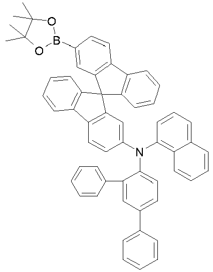 | 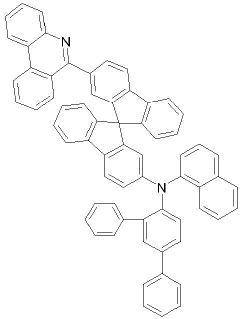 | 72 |
| 9h | 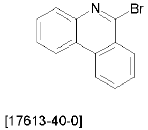 | 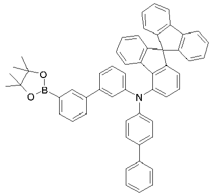 | 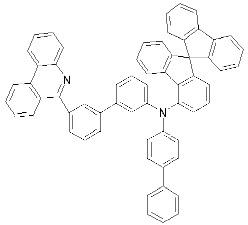 | 75 |
| 9i | 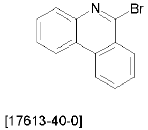 | 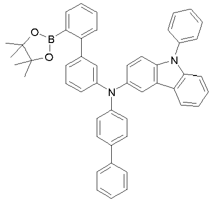 | 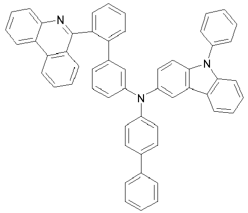 | 83 |
| 9j | 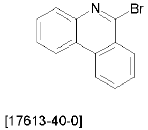 | 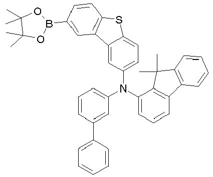 | 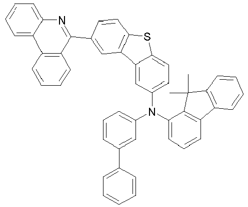 | 60 |
| 9k | 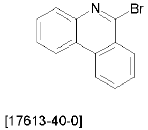 | 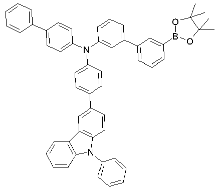 | 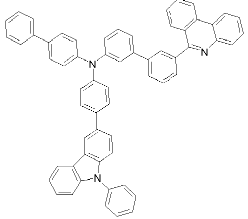 | 77 |
| 9l | 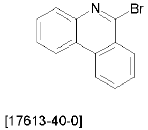 | 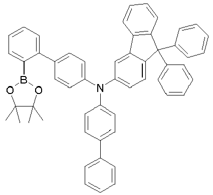 | 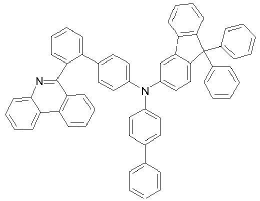 | 72 |
| 9m* | 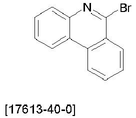 | 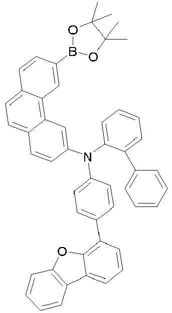 | 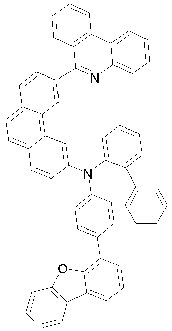 | 73 |
| 9n | 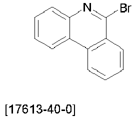 | 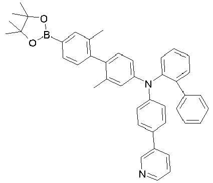 | 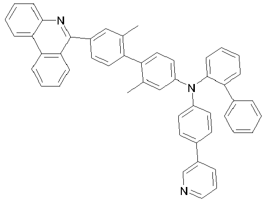 | 66 |
| 9o* | 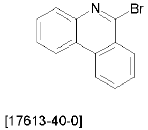 | 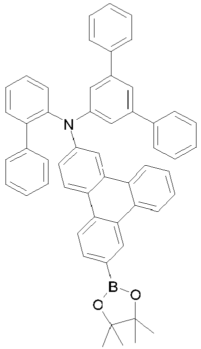 | 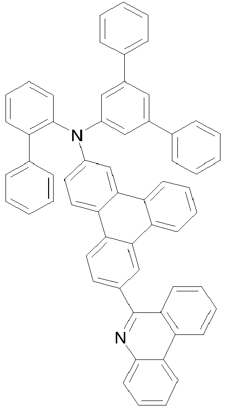 | 87 |
| 9p* | 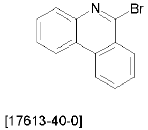 | 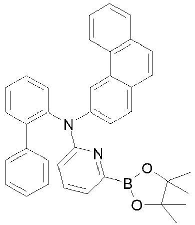 | 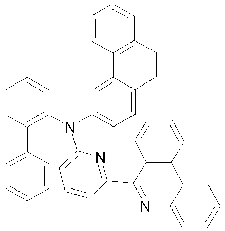 | 84 |
| 9q | 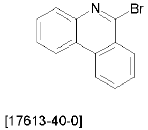 | 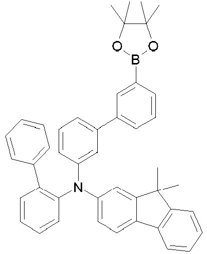 | 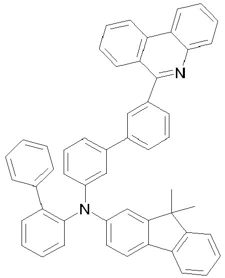 | 73 |
| 9r* | 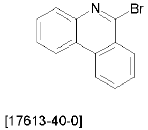 | 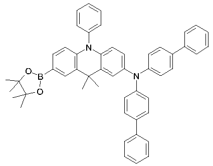 | 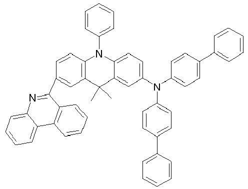 | 78 |
| 9s | 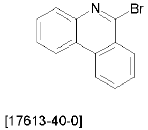 | 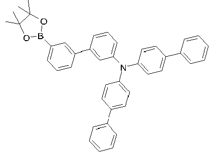 | 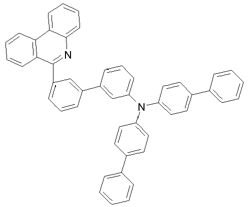 | 91 |
| 9t* | 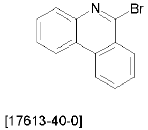 | 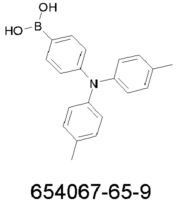 | 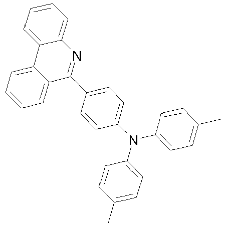 | 85 |
| 9u* | 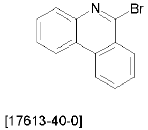 | 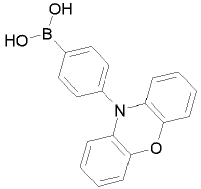 | 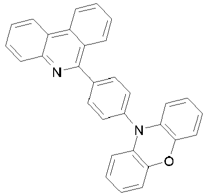 | 79 |

### Beispiel 10: Biphenyl-4-yl-(9,9-dimethyl-9H-fluoren-2-yl)-phenanthridin-6-yl-amin (Referenzmaterial)

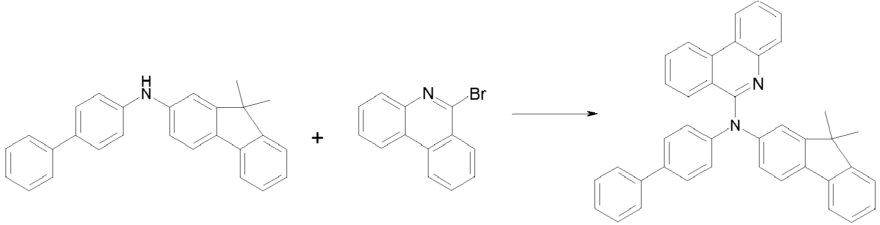

Es werden 29 g (80 mmol, 1,0 eq) Biphenyl-4-yl-(9,9-dimethyl-9H-fluoren-2-yl)-amine mit 20 g (80 mmol, 1,0 eq) 6-Bromphenanthridin in 600 ml Toluol gelöst und für 30 Minuten entgast. Anschließend werden 45 g (240 mmol, 3,0 eq) Natrium-*tert*-butylat, 890 mg (0,40 mmol, 0,050 eq) Palladium(II)-acetat und 8 ml (8,0 mmol, 0,10 eq.) einer 1M tri-*tert-*Butylphosphin-Lösung zugegeben. Der Ansatz wird über Nacht unter Rückfluss erhitzt und nach beendeter Reaktion zweimal über Aluminiumoxid mit Toluol filtriert. Nach Entfernung des Lösungsmittels am Rotationsverdampfer wird das ÖI in etwas THF gelöst und in Heptan eingetragen. Das Produkt wird via Säulenchromatographie an Kieselgel mit Toluol/Heptan (1:2) gereinigt und abschließend im Hochvakuum (p = 5 x 10⁻⁷ mbar) sublimiert. Es werden 29 g (28 mmol, 69%) des gewünschten Produkts mit der Reinheit <99,9% erhalten.

Analog können folgende Verbindungen erhalten werden (10a bis 10p sind Referenzmaterialien):

| **Bsp.** | **Edukt 1** | **Edukt 2** | **Produkt** | **Ausbeute [%]** |
|---|---|---|---|---|
| 10a | 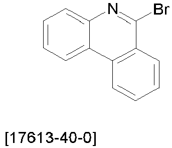 | 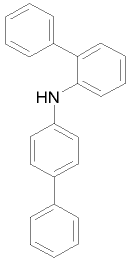 | 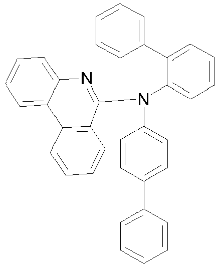 | 49% |
| 10b | 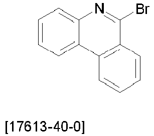 | 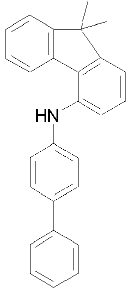 | 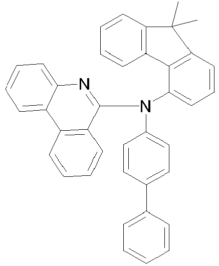 | 53 |
| 10c | 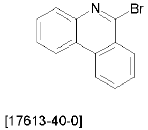 | 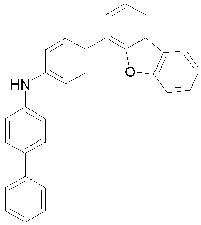 | 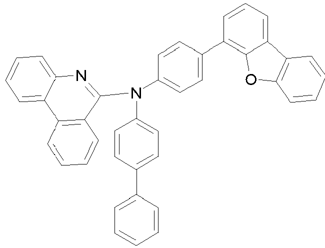 | 68 |
| | | | | |
| 10d | 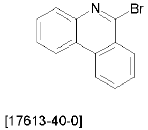 | 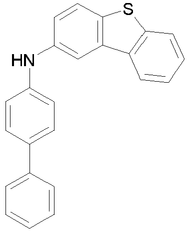 | 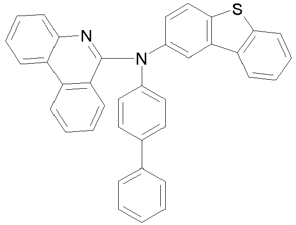 | 69 |
| 10e | 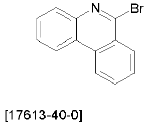 | 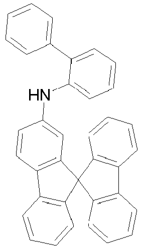 | 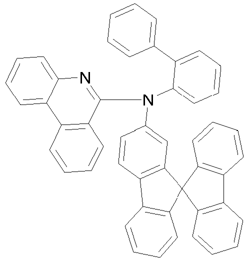 | 58 |
| 10f | 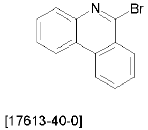 | 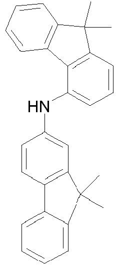 | 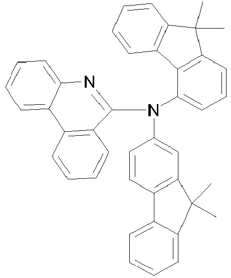 | 63 |
| 10g | 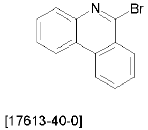 | 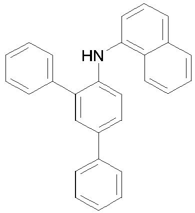 | 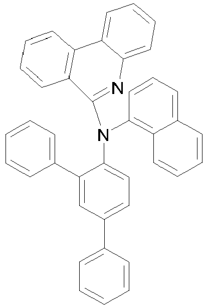 | 65 |
| 10h | 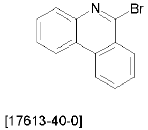 | 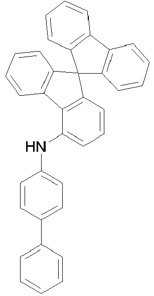 | 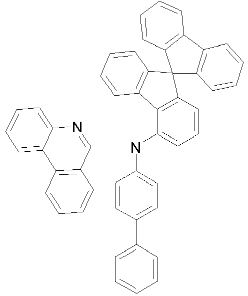 | 72 |
| 10i | 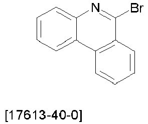 | 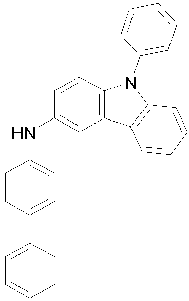 | 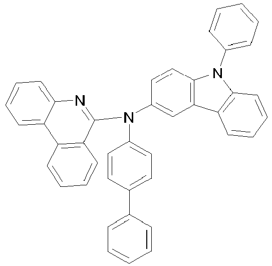 | 67 |
| 10m | 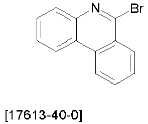 | 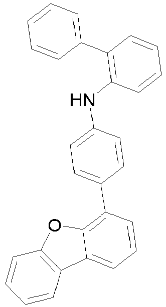 | 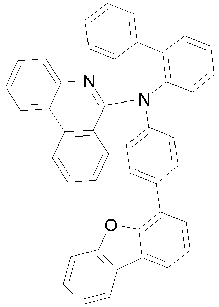 | 77 |
| 10p | 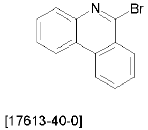 | 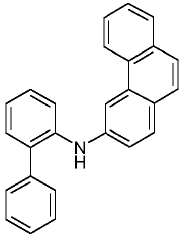 | 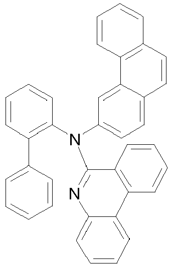 | 70 |

### Beispiel 11: Herstellung der OLEDs

In den folgenden Beispielen E1 bis E10 (siehe Tabellen 1 und 2) werden die Daten verschiedener OLEDs vorgestellt. Gereinigte Glasplättchen (Reinigung in Laborspülmaschine, Reiniger Merck Extran), die mit strukturiertem ITO (Indium Zinn Oxid) der Dicke 50 nm beschichtet sind werden 25 Minuten mit UV-Ozon vorbehandelt (UV-Ozon Generator PR-100, Firma UVP) und innerhalb 20min zur verbesserten Prozessierung mit 20nm PEDOT:PSS beschichtet (Poly(3,4-ethylenedioxythiophene) poly(styrenesulfonate), bezogen als CLEVlOS™ P VP AI 4083 von Heraeus Precious Metals GmbH Deutschland, aus wässriger Lösung aufgeschleudert) und anschließend bei 180°C 10min lang ausgeheizt. Diese beschichteten Glasplättchen bilden die Substrate, auf welche die OLEDs aufgebracht werden.

Die OLEDs haben prinzipiell folgenden Schichtaufbau: Substrat / Lochtransportschicht (HTL) / Zwischenschicht (IL) / Elektronenblockierschicht (EBL) / Emissionsschicht (EML) / Optionale Lochblockierschicht (HBL) / Elektronentransportschicht (ETL) und abschließend eine Kathode. Die Kathode wird durch eine 100 nm dicke Aluminiumschicht gebildet. Der genaue Aufbau der OLEDs ist Tabelle 1 zu entnehmen. Eine Bezeichnung wie "5a" in der Tabelle bezieht sich auf die in den Tabellen zu den Beispielen 4-10 gezeigten Materialien. Die weiteren zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC2:5c:TEG1 (60%:30%:10%) bedeutet hierbei, dass das Material IC2 in einem Volumenanteil von 60%, 5c in einem Anteil von 30% und TEG1 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 2 bezeichnet die Spannung, die für eine Leuchtdichte von 1000 cd/m² benötigt wird. SE1000 und LE1000 bezeichnen die Strom- bzw. Leistungseffizienz, die bei 1000 cd/m² erreicht werden. EQE1000 schließlich bezeichnet die externe Quanteneffizienz bei einer Betriebsleuchtdichte von 1000 cd/m².

Die Daten der verschiedenen OLEDs sind in Tabelle 2 zusammengefasst.

**Tabelle 1: Aufbau der OLEDs**

| Bsp. | HTL | IL | EBL | EML | HBL | ETL |
|---|---|---|---|---|---|---|
| | Dicke | Dicke | Dicke | Dicke | Dicke | Dicke |
| E1 | SpA1 | HATC | SpMA1 | IC1:TEG1 (90%:10%) 30nm | IC1 | 5r1:LiQ (50%:50%) 30nm |
| | 70nm | N 5nm | 90nm | | 10nm | |
| E2 | SpA1 | HATC | SpMA1 | 5r1:TEG1 (90%:15%) 30nm | IC1 | ST1:LiQ (50%:50%) 30nm |
| | 70nm | N 5nm | 90nm | | 10nm | |
| E3 | SpA1 | HATC | SpMA1 | 5r1:TER1 (90%:10%) 40nm | --- | ST1:LiQ (50%:50%) 40nm |
| | 90nm | N 5nm | 130nm | | | |
| E4 | SpA1 | HATC | SpMA1 | IC2:5a:TEG1 (60%:30%:10%) 30nm | IC1 | ST1:LiQ (50%:50%) 30nm |
| | 70nm | N 5nm | 90nm | | 10nm | |
| E5 | SpA1 | HATC | SpMA1 | IC2:5c:TEG1 (60%:30%:10%) 30nm | IC1 | ST1:LiQ (50%:50%) 30nm |
| | 70nm | N 5nm | 90nm | | 10nm | |
| E6 | SpA1 | HATC | SpMA1 | IC2:5i:TEG1 (45%:45%:10%) 30nm | ST1 | ST1:LiQ (50%:50%) 30nm |
| | 70nm | N 5nm | 90nm | | 10nm | |
| E7 | SpA1 | HATC | 9 | IC2:TEG1 (90%:10%) 30nm | IC1 | ST1:LiQ (50%:50%) 30nm |
| | 70nm | N 5nm | 90nm | | 10nm | |
| E8 | SpA1 | HATC | 9j | IC1:TEG1 (90%:10%) 30nm | IC1 | ST1:LiQ (50%:50%) 30nm |
| | 70nm | N 5nm | 90nm | | 10nm | |
| E9 | SpA1 | HATC | 9s | IC1:TEG1 (90%:10%) 30nm | IC1 | ST1:LiQ (50%:50%) 30nm |
| | 70nm | N 5nm | 90nm | | 10nm | |
| E10 | SpA1 | HATC | SpMA1 | IC1 :9c:TEG1 (55%:35%:10%) 30nm | IC1 | ST1:LiQ (50%:50%) 30nm |
| | 70nm | N 5nm | 90nm | | 10nm | |

**Tabelle 2: Daten der OLEDs**

| Bsp. | U1000 (V) | SE1000 (cd/A) | LE1000 (Im/W) | EQE 1000 | CIE x/y bei 1000 cd/m² |
|---|---|---|---|---|---|
| E1 | 4.8 | 55 | 36 | 15.3% | 0.34/0.62 |
| E2 | 3.6 | 57 | 49 | 15.8% | 0.33/0.63 |
| E3 | 4.9 | 12.2 | 7.8 | 13.2% | 0.67/0.33 |
| E4 | 3.5 | 55 | 49 | 15.4% | 0.33/0.62 |
| E5 | 3.7 | 50 | 42 | 13.7% | 0.33/0.63 |
| E6 | 3.2 | 57 | 55 | 15.9% | 0.34/0.62 |
| E7 | 3.3 | 47 | 45 | 13.3% | 0.34/0.62 |
| E8 | 3.4 | 60 | 55 | 16.8% | 0.34/0.62 |
| E9 | 3.5 | 61 | 55 | 17.2% | 0.34/0.62 |
| E10 | 3.4 | 64 | 59 | 18.0% | 0.34/0.62 |

**Tabelle 3: Strukturformeln der Materialien für die OLEDs**

| | |
|---|---|
| 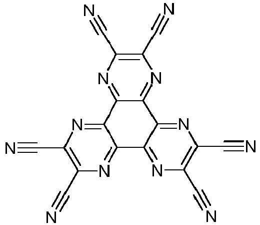 | 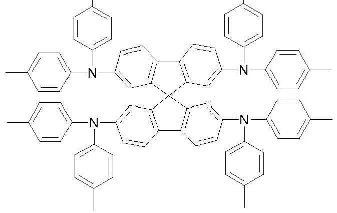 |
| HATCN | SpA1 |
| 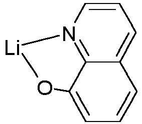 | 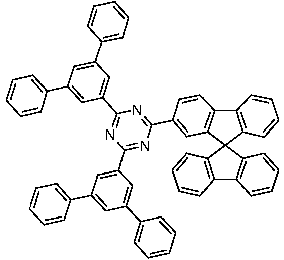 |
| LiQ | ST1 |
| 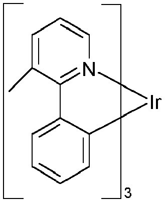 | 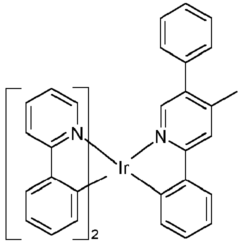 |
| TEG1 | TEG2 |
| 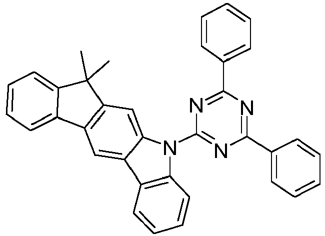 | 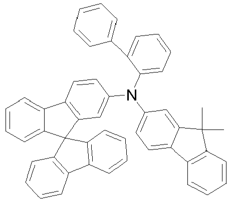 |
| IC1 | SpMA1 |
| 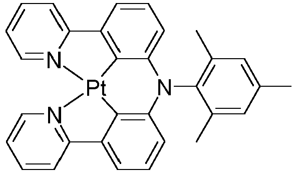 | 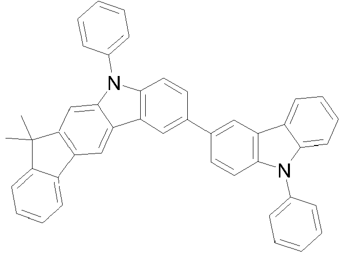 |
| TER1 | BIC1 |
| 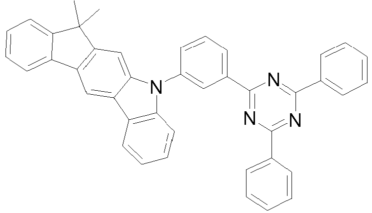 | |
| IC2 | |
| 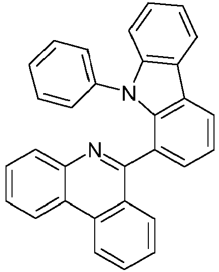 | 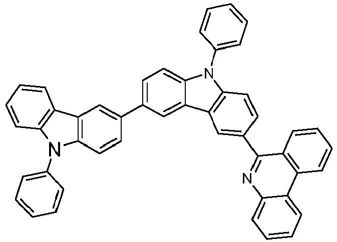 |
| 5a* | 5c* |
| 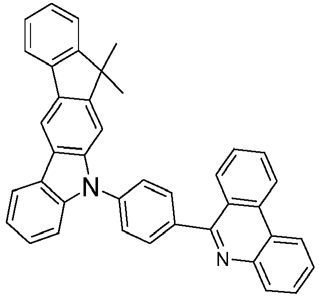 | 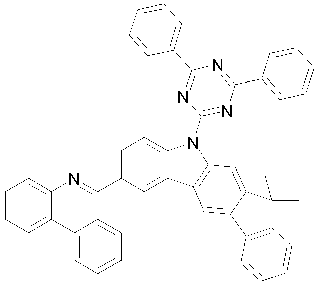 |
| 5i* | 5r1* |
| 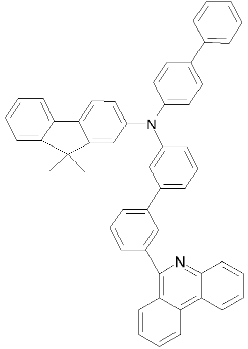 | 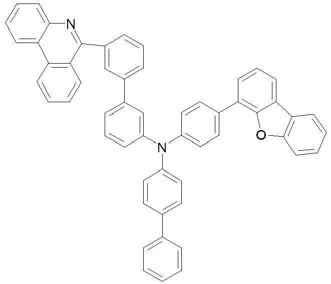 |
| 9 | 9c |
| 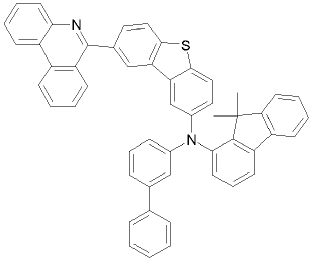 | 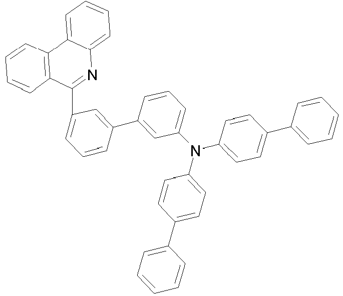 |
| 9j | 9s |

| | |
|---|---|
| *Referenzmaterialien | |

## Patentansprüche

1. Verbindung gemäß Formel (3) 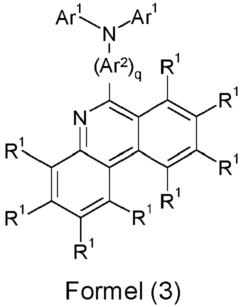 wobei für die verwendeten Symbole und Indizes gilt:
Ar¹ sind bei jedem Auftreten gleich oder verschieden ausgewählt aus den Gruppen mit den folgenden Formeln (Ar-1) bis (Ar-13) 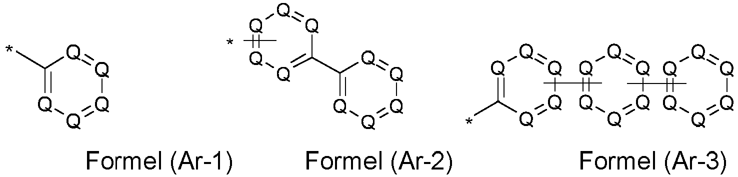 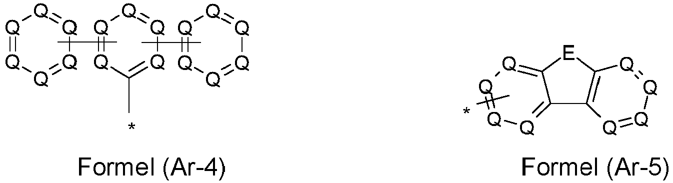 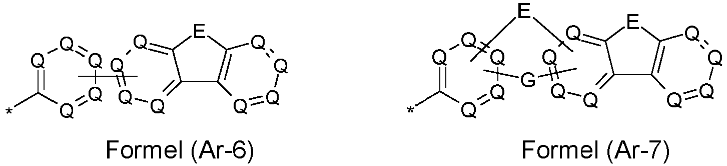 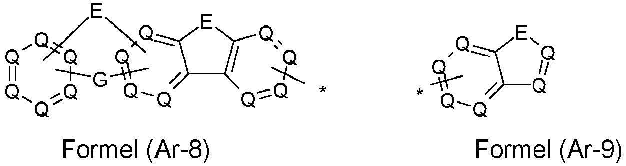 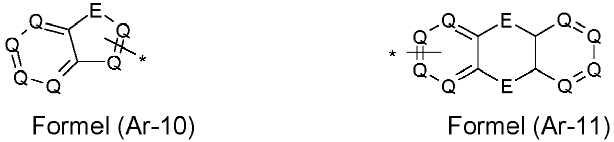 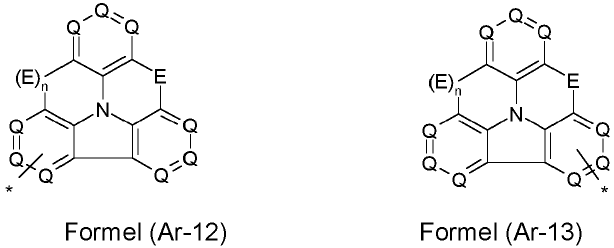 wobei:
Q ist bei jedem Auftreten gleich oder verschieden CR² oder N, wobei maximal 3 Symbole Q pro Cyclus für N stehen;
E ist bei jedem Auftreten gleich oder verschieden (CR²)₂, NR², O, S oder C=O;
G ist bei jedem Auftreten eine Einfachbindung, (CR²)₂, NR², O, S oder C=O;
n ist 0 oder 1, wobei n gleich 0 bedeutet, dass an dieser Position keine Gruppe E gebunden ist und an den entsprechenden Kohlenstoffatomen stattdessen Reste R² gebunden sind; und
* die Bindung zur Ar²-N-Gruppe darstellt;
Ar² ist bei jedem Auftreten gleich oder verschieden ausgewählt aus der Gruppe bestehend aus ortho-, meta- oder para-Biphenyl, Fluoren, Dibenzofuran, Dibenzothiophen oder Carbazol, wobei im Falle von heteroaromatischen Ringsystemen die divalente Bindung zum Grundgerüst nicht über das N-Atom erfolgt, wobei diese Gruppen durch einen oder mehrere Reste R² substituiert sein können;
R¹ ist bei jedem Auftreten gleich oder verschieden H, D, F, Si(R²)₃, CN, eine geradkettige Alkyl- oder Alkoxygruppe mit 1 bis 10 C-Atomen oder eine verzweigte oder cyclische Alkyl- oder Alkoxygruppe mit 3 bis 10 C- Atomen, die jeweils mit einem oder mehreren Resten R² substituiert sein kann, wobei eine oder mehrere nicht-benachbarte CH₂-Gruppen durch O ersetzt sein können und wobei ein oder mehrere H-Atome durch D oder F ersetzt sein können, ein aromatisches oder heteroaromatisches Ringsystem mit 6 bis 60 aromatischen Ringatomen, das jeweils mit einem oder mehreren Resten R² substituiert sein kann;
R² ist bei jedem Auftreten gleich oder verschieden H, D, F, Si(R³)₃, CN, eine geradkettige Alkyl- oder Alkoxygruppe mit 1 bis 10 C-Atomen oder eine verzweigte oder cyclische Alkyl- oder Alkoxygruppe mit 3 bis 10 C- Atomen, die jeweils mit einem oder mehreren Resten R³ substituiert sein kann, wobei eine oder mehrere nicht-benachbarte CH₂-Gruppen durch O ersetzt sein können und wobei ein oder mehrere H-Atome durch D oder F ersetzt sein können, ein aromatisches oder heteroaromatisches Ringsystem mit 6 bis 60 aromatischen Ringatomen, das jeweils mit einem oder mehreren Resten R³ substituiert sein;
R³ ist bei jedem Auftreten gleich oder verschieden H, D, F, CN, Si(R⁴)₃, eine geradkettige Alkyl- oder Alkoxygruppe mit 1 bis 40 C-Atomen oder eine verzweigte oder cyclische Alkyl- oder Alkoxygruppe mit 3 bis 40 C-Atomen, die jeweils mit einem oder mehreren Resten R⁴ substituiert sein kann, oder ein aromatisches oder heteroaromatisches Ringsystem mit 5 bis 60 aromatischen Ringatomen, das jeweils durch einen oder mehrere Reste R⁴ substituiert sein kann;
R⁴ ist bei jedem Auftreten gleich oder verschieden H, D, F oder ein aliphatischer Kohlenwasserstoffrest mit 1 bis 20 C-Atomen oder eine Aryl- oder Heteroarylgruppe mit 5 bis 40 Ringatomen, die durch einen oder mehrere Reste R⁵ substituiert sein kann;
R⁵ ist bei jedem Auftreten gleich oder verschieden H, D, F oder ein aliphatischer Kohlenwasserstoffrest mit 1 bis 20 C-Atomen;
q ist 1;
wobei
Ar² im Falle eines heteroaromatischen Ringsystems über eine C-C-Bindung mit dem Phenanthridin-Grundgerüst verbunden ist; und
R¹ im Falle eines heteroaromatischen Ringsystems über eine C-C-Bindung mit dem Phenanthridin-Grundgerüst verbunden ist.

2. Verfahren zur Herstellung einer Verbindung nach Anspruch 1, **dadurch gekennzeichnet, dass** die Verbindung der Formel (3) durch eine oder mehrere Kupplungsreaktionen und/oder Zyklisierungen aufgebaut wird.

3. Mischung enthaltend mindestens eine Verbindung nach Anspruch 1 und mindestens einen fluoreszierenden oder phosphoreszierenden Dotanden.

4. Formulierung, insbesondere eine Lösung, eine Suspension oder eine Miniemulsion, enthaltend mindestens eine Verbindung nach Anspruch 1 oder eine Mischung nach Anspruch 3 und ein oder mehrere Lösemittel.

5. Verwendung einer Verbindung nach Anspruch 1 oder einer Mischung nach Anspruch 3 in einer elektronischen Vorrichtung.

6. Elektronische Vorrichtung, insbesondere ausgewählt aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen farbstoffsensibilisierten Solarzellen, organischen optischen Detektoren, organischen Photorezeptoren, organischen Feld-Quench-Devices, lichtemittierenden elektrochemischen Zellen, organischen Laserdioden und "organic plasmon emitting devices", enthaltend mindestens eine Verbindung nach Anspruch 1 oder eine Mischung nach Anspruch 3.

7. Elektronische Vorrichtung nach Anspruch 6, **dadurch gekennzeichnet, dass** es sich um eine organische Elektrolumineszenzvorrichtung handelt und die Verbindung nach Anspruch 1 als Matrixmaterial für eine fluoreszierende oder phosphoreszierende Verbindung in einer emittierenden Schicht, und/oder in einer Lochtransportschicht und/oder in einer Elektronenblockierschicht eingesetzt wird.

## Claims

1. Compound of formula (3) 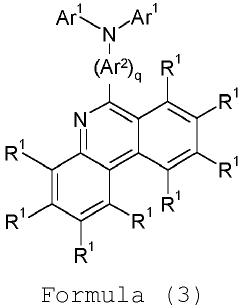 where the symbols and indices used are as follows:
Ar¹ are the same or different at each instance and are selected from the groups having the following formulae (Ar-1) to (Ar-13) 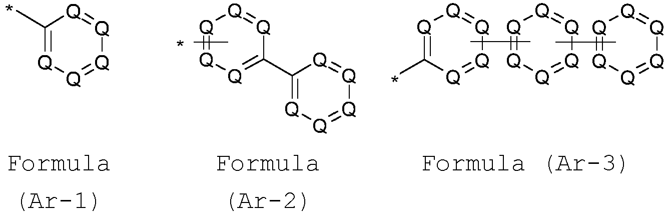 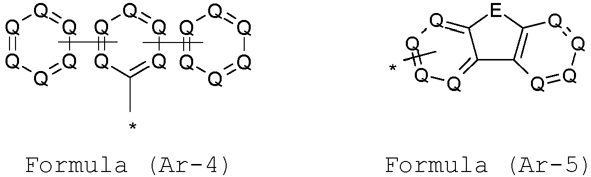 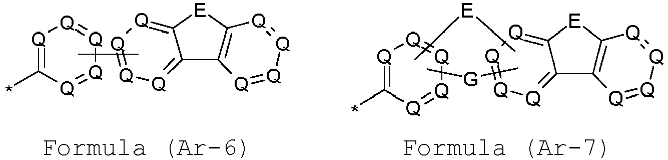 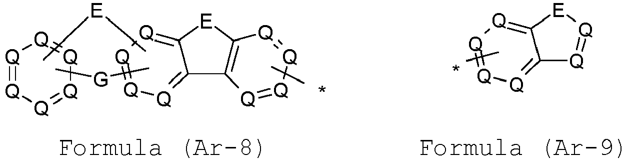 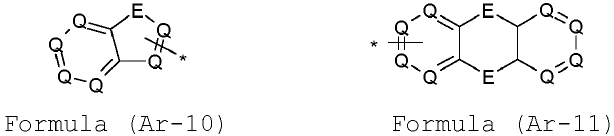 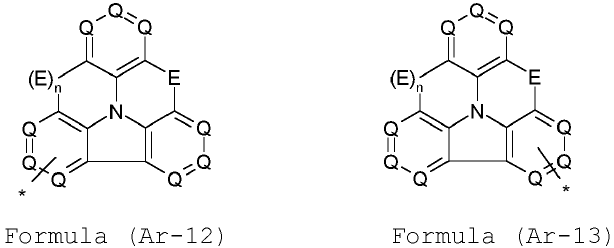
where:
Q is the same or different at each instance and is CR² or N, where not more than 3 Q symbols per cycle are N;
E is the same or different at each instance and is (CR²)₂, NR², O, S or C=O;
G at each instance is a single bond, (CR²)₂, NR², O, S or C=O;
n is 0 or 1, where n = 0 means that no E group is bonded at this position and R² radicals are bonded to the corresponding carbon atoms instead; and
* represents the bond to the Ar²-N group;
Ar² is the same or different at each instance and is selected from the group consisting of ortho-, meta- or para-biphenyl, fluorene, dibenzofuran, dibenzothiophene or carbazole, where the divalent bond to the base skeleton in the case of heteroaromatic ring systems is not via the nitrogen atom, where these groups may be substituted by one or more R² radicals;
R¹ is the same or different at each instance and is H, D, F, Si(R²)₃, CN, a straight-chain alkyl or alkoxy group having 1 to 10 carbon atoms or a branched or cyclic alkyl or alkoxy group having 3 to 10 carbon atoms, each of which may be substituted by one or more R² radicals, where one or more nonadjacent CH₂ groups may be replaced by O and where one or more hydrogen atoms may be replaced by D or F, or an aromatic or heteroaromatic ring system which has 6 to 60 aromatic ring atoms, each of which may be substituted by one or more R² radicals;
R² is the same or different at each instance and is H, D, F, Si(R³)₃, CN, a straight-chain alkyl or alkoxy group having 1 to 10 carbon atoms or a branched or cyclic alkyl or alkoxy group having 3 to 10 carbon atoms, each of which may be substituted by one or more R³ radicals, where one or more nonadjacent CH₂ groups may be replaced by O and where one or more hydrogen atoms may be replaced by D or F, or an aromatic or heteroaromatic ring system which has 6 to 60 aromatic ring atoms, each of which may be substituted by one or more R³ radicals;
R³ is the same or different at each instance and is H, D, F, CN, Si(R⁴)₃, a straight-chain alkyl or alkoxy group having 1 to 40 carbon atoms or a branched or cyclic alkyl or alkoxy group having 3 to 40 carbon atoms, each of which may be substituted by one or more R⁴ radicals, or an aromatic or heteroaromatic ring system which has 5 to 60 aromatic ring atoms, each of which may be substituted by one or more R⁴ radicals;
R⁴ is the same or different at each instance and is H, D, F or an aliphatic hydrocarbyl radical having 1 to 20 carbon atoms or an aryl or heteroaryl group which has 5 to 40 ring atoms and may be substituted by one or more R⁵ radicals;
R⁵ is the same or different at each instance and is H, D, F or an aliphatic hydrocarbyl radical having 1 to 20 carbon atoms;
q is 1;
where
Ar² in the case of a heteroaromatic ring system is joined to the phenanthridine base skeleton via a C-C bond; and,
R¹ in the case of a heteroaromatic ring system is joined to the phenanthridine base skeleton via a C-C bond.

2. Process for preparing a compound according to Claim 1, **characterized in that** the compound of the formula (3) is formed by one or more coupling reactions and/or cyclizations.

3. Mixture comprising at least one compound according to Claim 1 and at least one fluorescent or phosphorescent dopant.

4. Formulation, especially a solution, a suspension or a miniemulsion, comprising at least one compound according to Claim 1 or a mixture according to Claim 3 and one or more solvents.

5. Use of a compound according to Claim 1 or of a mixture according to Claim 3 in an electronic device.

6. Electronic device, especially selected from the group consisting of organic electroluminescent devices, organic integrated circuits, organic field-effect transistors, organic thin-film transistors, organic light-emitting transistors, organic solar cells, organic dye-sensitized solar cells, organic optical detectors, organic photoreceptors, organic field-quench devices, light-emitting electrochemical cells, organic laser diodes and organic plasmon emitting devices, comprising at least one compound according to Claim 1 or a mixture according to Claim 3.

7. Electronic device according to Claim 6, **characterized in that** it is an organic electroluminescent device and the compound according to Claim 1 is used as matrix material for a fluorescent or phosphorescent compound in an emitting layer and/or in a hole transport layer and/or in an electron blocker layer.

## Revendications

1. Composé selon la formule (3) : 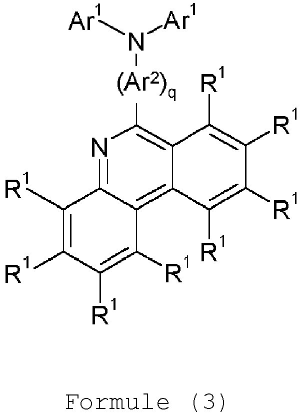 dans laquelle, pour les symboles et indices utilisés :
les Ar¹ sont à chaque occurrence de manière identique ou différente choisis parmi les groupes ayant les formules (Ar-1) à (Ar-13) suivantes : 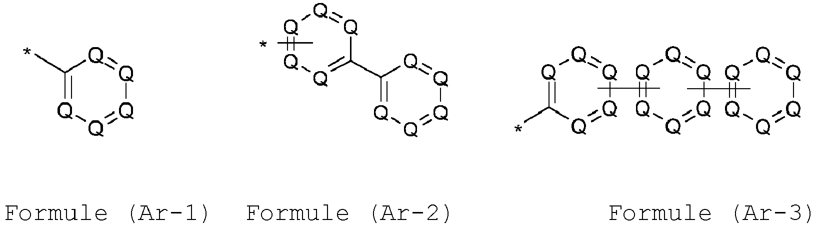 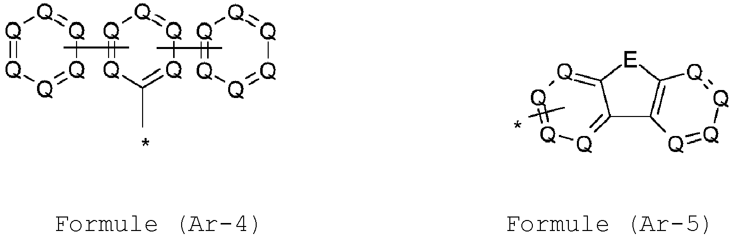 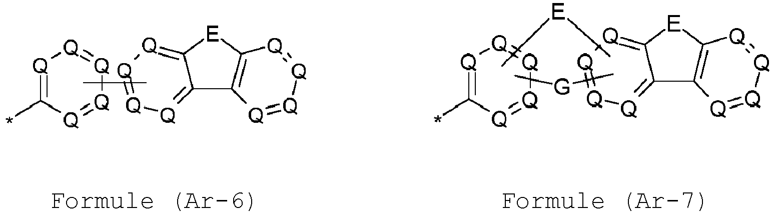 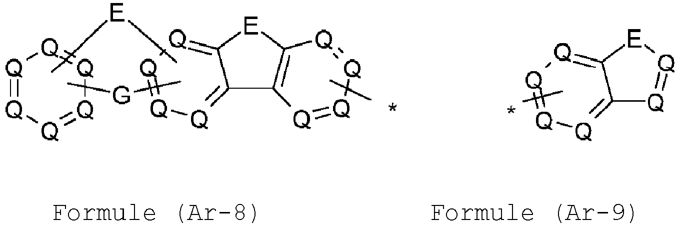 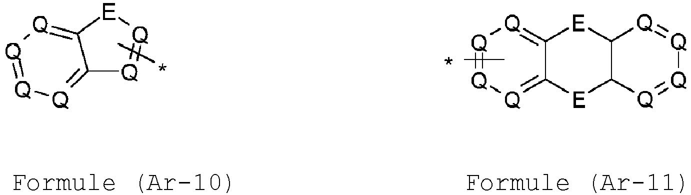 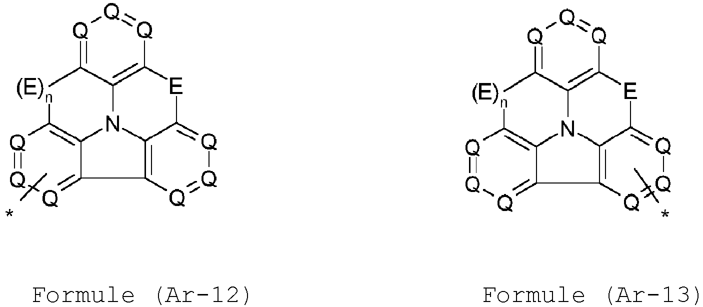 dans lesquelles :
Q est à chaque occurrence de manière identique ou différente CR² ou N, au plus 3 symboles Q par cycle représentant N ;
E est à chaque occurrence de manière identique ou différente (CR²)₂, NR², O, S ou C=O ;
G est à chaque occurrence une simple liaison, (CR²)₂, NR², O, S ou C=O ;
n vaut 0 ou 1, n valant 0 signifiant qu'aucun groupe E n'est relié à cette position et que des radicaux R² sont reliés à la place aux atomes de carbone correspondants ; et
* représente la liaison au groupe Ar²-N ;
Ar² est à chaque occurrence de manière identique ou différente choisi dans le groupe constitué par ortho-, méta- ou para-biphényle, fluorène, dibenzofurane, dibenzothiophène ou carbazole, dans le cas de systèmes cycliques hétéroaromatiques, la liaison divalente au squelette de base n'ayant pas lieu par l'intermédiaire de l'atome N, ces groupes pouvant être substitués par un ou plusieurs radicaux R² ;
R¹ est à chaque occurrence de manière identique ou différente H, D, F, Si(R²)₃, CN, un groupe alkyle ou alcoxy linéaire de 1 à 10 atomes C ou un groupe alkyle ou alcoxy ramifié ou cyclique de 3 à 10 atomes C, qui peut à chaque fois être substitué avec un ou plusieurs radicaux R², un ou plusieurs groupes CH₂ non voisins pouvant être remplacés par O et un ou plusieurs atomes H pouvant être remplacés par D ou F, un système cyclique aromatique ou hétéroaromatique de 6 à 60 atomes de cycle aromatique, qui peut à chaque fois être substitué avec un ou plusieurs radicaux R² ;
R² est à chaque occurrence de manière identique ou différente H, D, F, Si(R³)₃, CN, un groupe alkyle ou alcoxy linéaire de 1 à 10 atomes C ou un groupe alkyle ou alcoxy ramifié ou cyclique de 3 à 10 atomes C, qui peut à chaque fois être substitué avec un ou plusieurs radicaux R³, un ou plusieurs groupes CH₂ non voisins pouvant être remplacés par O et un ou plusieurs atomes H pouvant être remplacés par D ou F, un système cyclique aromatique ou hétéroaromatique de 6 à 60 atomes de cycle aromatique, qui peut à chaque fois être substitué avec un ou plusieurs radicaux R³ ;
R³ est à chaque occurrence de manière identique ou différente H, D, F, CN, Si(R⁴)₃, un groupe alkyle ou alcoxy linéaire de 1 à 40 atomes C ou un groupe alkyle ou alcoxy ramifié ou cyclique de 3 à 40 atomes C, qui peut à chaque fois être substitué avec un ou plusieurs radicaux R⁴, ou un système cyclique aromatique ou hétéroaromatique de 5 à 60 atomes de cycle aromatique, qui peut à chaque fois être substitué par un ou plusieurs radicaux R⁴ ;
R⁴ est à chaque occurrence de manière identique ou différente H, D, F ou un radical hydrocarboné aliphatiique de 1 à 20 atomes C ou un groupe aryle ou hétéroaryle de 5 à 40 atomes de cycle, qui peut être substitué par un ou plusieurs radicaux R⁵ ;
R⁵ est à chaque occurrence de manière identique ou différente H, D, F ou un radical hydrocarboné aliphatiique de 1 à 20 atomes C ;
q vaut 1 ;
Ar² dans le cas d'un système cyclique hétéroaromatique étant relié par l'intermédiaire d'une liaison C-C avec le squelette de base phénanthridine ; et
R¹ dans le cas d'un système cyclique hétéroaromatique étant relié par l'intermédiaire d'une liaison C-C avec le squelette de base phénanthridine.

2. Procédé de fabrication d'un composé selon la revendication 1, **caractérisé en ce que** le composé de la formule (3) est formé par une ou plusieurs réactions de couplage et/ou cyclisations.

3. Mélange contenant au moins un composé selon la revendication 1 et au moins un dopant fluorescent ou phosphorescent.

4. Formulation, notamment solution, suspension ou mini-émulsion, contenant au moins un composé selon la revendication 1 ou un mélange selon la revendication 3 et un ou plusieurs solvants.

5. Utilisation d'un composé selon la revendication 1 ou d'un mélange selon la revendication 3 dans un dispositif électronique.

6. Dispositif électronique, notamment choisi dans le groupe constitué par les dispositifs électroluminescents organiques, les commutateurs intégrés organiques, les transistors à effet de champ organiques, les transistors à film mince organiques, les transistors électroluminescents organiques, les cellules solaires organiques, les cellules solaires organiques sensibilisées par des colorants, les détecteurs optiques organiques, les photorécepteurs organiques, les dispositifs d'extinction de champ organiques, les cellules électrochimiques électroluminescentes, les diodes lasers organiques et les « dispositifs d'émission de plasmon organiques », contenant au moins un composé selon la revendication 1 ou un mélange selon la revendication 3.

7. Dispositif électronique selon la revendication 6, **caractérisé en ce qu'**il s'agit d'un dispositif électroluminescent organique, et le composé selon la revendication 1 est utilisé en tant que matériau de matrice pour un composé fluorescent ou phosphorescent dans une couche d'émission, et/ou dans une couche de transport de trous et/ou dans une couche de blocage d'électrons.
